# EUROPEAN PATENT SPECIFICATION

(11) **EP 4 392 123 B1**
(45) Date of publication and mention of the grant of the patent: **09.07.2025**
(21) Application number: 22769381.9
(22) Date of filing: 24.08.2022
(51) Int. Cl.: A61N 1/05, A61N 1/36, A61N 1/375, A61N 1/378

(54) **IMPLANTABLE STIMULATOR WITH AN ELECTRODE ARRAY, CONFORMABLE SUBSTRATE, AND MECHANICAL STRAIN RELIEF**
IMPLANTIERBARER STIMULATOR MIT EINER ELEKTRODENANORDNUNG, EINEM ANPASSBAREN SUBSTRAT UND MECHANISCHER ZUGENTLASTUNG
STIMULATEUR IMPLANTABLE AVEC RÉSEAU D'ÉLECTRODES, SUBSTRAT CONCORDANT ET ATTÉNUATION DES CONTRAINTES MÉCANIQUES

(30) Priority: 25.08.2021 US 202117412191
(43) Date of publication of application: 03.07.2024
(73) Proprietor: Salvia Bioelectronics B.V., 5656 AE Eindhoven (NL)
(72) Inventor: MARTENS, Hubert Cecile Francois, 5656 AE Eindhoven (NL); SCHOBBEN, Daniël Willem Elisabeth, 5656 AE Eindhoven (NL); VAN DER ZALM, Maartje, 5656 AE Eindhoven (NL); BOERE, Stijn, 5656 AE Eindhoven (NL)
(74) Representative: Lees, Kate Jane
(86) International application number: PCT/IB2022/057941
(87) International publication number: WO 2023/026216

(56) References cited:
- WO-A1-2021/111371
- US-A1- 2017 128 015
- US-A1- 2021 170 175
- US-A1- 2021 170 176
- US-A1- 2021 387 001

## Description

### TECHNICAL FIELD

The present disclosure relates to an implantable stimulator, for providing electrical stimulation to human or animal tissue, having an electrode array located along a conformable portion of a substrate, In particular, it relates to an implantable stimulator having an encapsulation layer at least partially covering a portion of the substrate. It also relates to a method of manufacturing an implantable stimulator.

### BACKGROUND

Implantable electrical stimulation systems may be used to deliver electrical stimulation therapy to patients to treat a variety of symptoms or conditions such as headaches, lower back pain and incontinence.

In many electrical stimulation applications, it is desirable for a stimulator, typically comprising a therapeutic lead (a lead comprises electrodes and electrical connections), to provide electrical stimulation to one or more precise locations within a body - in many cases, precisely aligning the stimulation electrodes during implantation may be difficult due to the curvature of tissues and anatomical structures. A mismatch in curvature of the electrode section of a lead may create unexpected and/or unpredictable electrical resistance between one or more electrodes and the underlying tissue. In addition, repeated movement of the relevant areas of the body may even worsen the mismatch. A particular problem with subcutaneous implants is that even small differences in flexibility between the implant and surrounding tissue may affect patient comfort, and can cause irritation of the overlying skin. This is a particular problem with sub-cutaneous implants.

In particular, the use of neurostimulation leads in the craniofacial region is associated with skin erosion and lead migration. The cylindrical shape and associated thickness of state-of-the-art leads results in the lead eroding through the skin or results in the lead being displaced so that the electrodes no longer cover the targeted nerves.

More recently, use has been made of plastics and polymers, which have an inherent flexibility or may be made in a curved shape - for example, as described in US application US 2016/0166828. Although such leads may be manufactured in a curved-shape or deformed by human manual manipulation during implantation, this is inconvenient. The high degree of anatomic variability found in humans and animals means that a manufacturer must provide either a large range or pre-curved leads or allow the leads to be made to measure. In the case that they are deformable during implantation, this further complicates the implantation process.

Implantable active devices require a protection method to protect the implant electronics from bodily fluids present in human or animal bodies. Bodily fluids typically contain ions that may cause electrochemical reactions, like corrosion, in the presence of an electric current. Encapsulation is thus a critical component for the design of a medical device - it acts as a barrier between these ionic fluids and critical electronic/electric interfaces to reduce and/or prevent degradation of the implant electronics.

Polyimides are popular for use as a substrate material for the microfabrication of electronics, and attempts have been made to encapsulate polyimides with silicone rubber encapsulants, such as polydimethylsiloxane rubber (PDMS). As described in "Irreversible bonding of polyimide and polydimethylsiloxane (PDMS) based on a thiol-epoxy click reaction", Hoang, Chung and Elias, Journal of Micromechanics and Microengineering, 10.1088/0960-1317/26/10/105019, bonding these two flexible materials remains a crucial challenge - the resistance to fluid ingress may be reduced by the encapsulant delaminating to some degree from the substrate. The degree of bonding was increased by functionalizing the surfaces of the PDMS and polyimide substrates with mercaptosilanes and epoxysilanes, respectively, for the formation of a thiolepoxy bond in the click reaction. It was also increased by functionalizing one or both surfaces with mercaptosilane and introducing an epoxy adhesive layer between the two surfaces.

Although PDMS can be substantially biocompatible, causing minimal tissue reaction while having a relative long period of biostability, it still has a relatively high permeability to moisture which can lead to degradation of the implant electronics. Many other encapsulants with a lower degree of moisture permeability may have a lower degree of biocompatibility. Recently, LCP's (Liquid Crystal Polymers) have been considered for use as a substrate for electronics, and there is also a need for improved bonding techniques between LCP and encapsulants.

US2021/170176 discloses an implantable stimulator.

### SUMMARY

The invention is defined by the independent claims. It is to be understood that both the following summary and the detailed description are exemplary and explanatory and are intended to provide further explanation of the invention as claimed. Neither the summary nor the description that follows is intended to define or limit the scope of the invention to the particular features mentioned in the summary or in the description. Rather, the scope of the invention is defined by the appended claims.

In certain embodiments, the disclosed embodiments may include one or more of the features described herein.

An implantable stimulator is provided, comprising: a substrate comprising a first surface and a second surface, wherein a thickness of the substrate is defined by the first and second surfaces; the substrate comprising a first portion along which a pulse generator is located, the pulse generator comprising one or more electrical or electronic components, configured to generate at least one stimulation pulse; an electrode array comprising at least two electrodes located along a conformable second portion of the substrate; a plurality of electrical interconnections electrically coupling the pulse generator to the at least two electrodes of the electrode array, wherein the plurality of electrical interconnections are positioned between the first and second surfaces of the substrate, wherein the implantable stimulator comprises one or more electrical interfaces between the first portion and the conformable second portion, the one or more electrical interfaces being between the plurality of electrical interconnections and the pulse generator, wherein the implantable stimulator comprises at least one conductive elastomer, configured and arranged to electrically connect one or more electrical interfaces between the plurality of electrical interconnections and the pulse generator, wherein the thickness of the substrate along the conformable second portion is equal to or less than 0.5 millimeters; the implantable stimulator further comprising at least one encapsulation layer at least partially covering the first portion and at least partially covering the conformable second portion of the substrate, the at least one encapsulation layer being configured to resist separation of the conformable second portion from the first portion at the meeting of the first portion and the conformable second portion.

The products and methods described herein provide a high degree of conformability as well as high degree of configurability. A higher degree of conformability may increase the comfort for the user. Optionally, the thickness of the conformable portion is equal to or less than 0.3 millimeters, or equal to or less than 0.2 millimeters, or equal to or less than 0.1 millimeters.,

By providing at least one encapsulation layer, a conductive elastomer may be used which may simplify manufacturing and/or repair. Optionally, the degree of separation resistance of the conformable second portion from the first portion may be provided by the at least one encapsulation layer due to at least one property of the at least one encapsulation layer, wherein the at least one property is a shape, an extent, a thickness, a physical property, an adherence, or any combination thereof.

Optionally, the at least one conductive elastomer may be an Anisotropic Conductive Adhesive, an Anisotropic Conductive Film, an Anisotropic Conductive Foam, an Anisotropic Conductive Paste, or any combination thereof. An ACA elastomer such as an ACF polymer, may provide a high degree of separation resistance to longitudinal forces.

Optionally, the at least one encapsulation layer may cover the first portion of the substrate (300, 1400).

Optionally, the first portion of the substrate and pulse generator may be at least partially embedded in one or more flexible bio-compatible encapsulation layers. Improved encapsulation may improve the reliability and/or lifetime of the implantable substrate.

Additionally or alternatively, the substrate further comprises at least one mechanical brace at the meeting of the first portion and the conformable second portion, the at least one mechanical brace being configured to resist separation of the conformable second portion from the first portion.

By providing at least one mechanical brace, the risk of moisture ingress may be reduced, and/or the risk of damage to one or more interconnections passing from the conformable second portion to the first portion may also be reduced.

Optionally, the at least one mechanical brace may comprise one or more openings and/or grooves, configured to receive significant amounts of encapsulant from the at least one encapsulation layer. Optionally, the at least one mechanical brace is configured to be releasable.

Additionally or alternatively, the implantable stimulator further comprises an adhesion layer adjacent to at least part of the first portion and at least part of the conformable second portion of the substrate. Optionally, the substrate comprises more than one adjacent substrate layer and the adhesion layer is between substrate layers.

One or more adhesion layers may improve the performance of the encapsulation. This may also improve the reliability and/or lifetime of the implantable substrate. By providing a multilayer substrate, thinner conformable portions may be provided, adding to the flexibility and therefore improving conformability.

Additionally or alternatively, the thickness of the stimulator along the first portion is equal to or less than 5 millimeters, or equal to or less than 4 millimeters, or equal to or less than 3 millimeters. This may further improve conformability of the first portion of the substrate.

Additionally or alternatively, the plurality of electrical interconnections are positioned between the first and second surfaces of the substrate using metallization. Additionally or alternatively, the plurality of electrical interconnections are comprised in one or more conductive interconnection layers, the one or more conductive interconnection layers being comprised between two adjacent polymeric substrate layers.

By providing a more easily patternable substrate, more complicated electrode array configurations may be supported, allowing a higher degree of flexibility to address transverse and/or longitudinal misalignment.

A method of manufacturing an implantable stimulator is provided, comprising: providing a substrate, the substrate comprising a first surface and a second surface, wherein a thickness of the substrate is defined by the first and second surfaces; providing a pulse generator, the pulse generator being configured to generate at least one stimulation pulse; locating an electrode array comprising at least two electrodes along a conformable portion of the substrate; depositing or electro-plating onto the substrate a plurality of electrical interconnections electrically coupling the pulse generator to the at least two electrodes of the electrode array; providing one or more electrical interfaces between the first portion and the conformable second portion, wherein the one or more electrical interfaces are between the plurality of electrical interconnections and the pulse generator; providing one or more electrical interfaces between the first portion and the conformable second portion, the one or more electrical interfaces being between the plurality of electrical interconnections and the pulse generator; providing at least one conductive elastomer to electrically connect one or more electrical interfaces between the plurality of electrical interconnections and the pulse generator, wherein the thickness of the substrate along the conformable portion is equal to or less than 0.5 millimeters, and providing at least one encapsulation layer to at least partially cover the first portion and to at least partially cover the conformable second portion of the substrate, the at least one encapsulation layer being configured to resist separation of the conformable second portion from the first portion at the meeting of the first portion and the conformable second portion.

Such products and associated methods described herein provide improved bonding to improve resistance to fluid ingress in implantable devices comprising flexible substrates. The encapsulant/adhesion layer may be optimized to protect a surface of many types of substrates. If the substrate is configured and arranged to be substantially flexible, the substrate may have a high degree of conformability. The high degree of adhesion of the encapsulant/adhesion layer allows the flexible encapsulant layer to provide a high degree of ingress protection for one or more surfaces of a flexible substrate.

One or more regions of a substrate surface may be protected by an encapsulant/adhesion layer. Each encapsulant/adhesion layer may be optimized separately or together to a predetermined degree.

Optionally, the conformable portion of the substrate comprises a liquid crystal polymer (LCP).

Additionally or alternatively, wherein the substrate comprises a further portion along which the pulse generator is located, the encapsulation layer at least partially covering the further portion of the substrate.

Optionally, the encapsulation layer comprises a polymer and/or Polydimethylsiloxane (PDMS).

By providing a bilayer having an encapsulant comprising a PDMS and a conformal adhesion layer, the adhesion layer appears to show significantly higher stability in ionic media, thereby providing relatively longer protection in case of any delamination or water permeation through the encapsulant. A PDMS may further contribute to longer-lasting adhesion and defect reduction due to flowing in-between any defects and crevices in the adhesion layer - in particular, a PDMS with a relatively low viscosity may provide an even higher degree of defect reduction.

A method of manufacturing an implantable stimulator is provided, comprising:
Optionally, the adhesion layer is applied using atomic layer deposition (ALD). Additionally or alternatively, the pulse generator is provided along a further portion of the substrate, wherein the adhesion layer and encapsulation layer are applied to at least partially cover the further portion of the substrate.

In additional embodiments, an implantable stimulator comprises: a substrate comprising a first surface and a second surface, wherein a thickness of the substrate is defined by the first and second surfaces; a pulse generator being configured to generate at least one stimulation pulse; at least two electrodes located along a conformable portion of the substrate; a plurality of electrical interconnections electrically coupling the pulse generator to the at least two electrodes; an encapsulation layer at least partially covering the substrate; and an adhesion layer between the encapsulation layer and the substrate in at least one location; wherein the thickness of the substrate along the conformable portion is equal to or less than 0.5 millimeters. In some embodiments, the thickness of the substrate along the conformable portion may be equal to or less than 0.3 millimeters.

In any of the implantable stimulators described above herein, the encapsulation layer may cover at least part of the conformable portion of the substrate, and the adhesion layer may be between the encapsulation layer and the at least part of the conformable portion of the substrate. The conformable portion of the substrate may also comprise one or more layers of the LCP. The substrate may additionally comprise a further portion along which the pulse generator is located, the encapsulation layer at least partially covering the further portion of the substrate. In some embodiments, the further portion of the substrate is also conformable, and the further portion may be LCP. Additionally, the thickness of the stimulator along the further portion may be equal to or less than 5 millimeters. The thickness of the stimulator along the further portion may also be equal to or less than 4 millimeters.

In at least one embodiment of the implantable stimulator, the conformable part of the substrate has a Young's modulus in the range 2500 to 3600 MPa. Additionally, the encapsulation layer may have a tensile strength in the range 6 to 8 MPa.

Any of the implantable stimulators described above herein may further comprise other adhesion layers, wherein the substrate comprises more than one substrate layer and the other adhesion layers are between substrate layers.

In at least one embodiment, the encapsulation layer covers the first surface of the substrate and not the second surface, further comprising a second encapsulation layer covering the second surface of the substrate.

In a further embodiment, the adhesion layer is biocompatible. The adhesion layer may also conform to the first surface and/or the second surface of the substrate. Both the adhesion layer and the encapsulation layer may also be configured to resist ingress of fluids onto the substrate. The encapsulation layer may comprise Polydimethylsiloxane (PDMS).

In additional embodiments, the conformable portion of the substrate may comprise a substance selected from the group consisting of: a Liquid-Crystal Polymer (LCP), a polyimide, Parylene-C, SU-8, a polyurethane, or any combination thereof.

The implantable stimulator in at least one embodiment has a substrate that comprises a first conformable layer and at least one second conformable layer, wherein the plurality of electrical interconnections are positioned along the first layer using a deposition technique, and wherein the at least one second layer is secured to the first layer so as to cover the plurality of electrical interconnections.

In at least a further embodiment, an implantable stimulator comprises: a substrate, the substrate comprising a top surface and a bottom surface; a pulse generator located along a first portion of the substrate, the pulse generator being configured to generate at least one stimulation pulse; at least two electrodes located along a second, conformable portion of the substrate; a plurality of electrical interconnections electrically coupling the pulse generator to the at least two electrodes; wherein the plurality of electrical interconnections are positioned between the top and bottom surfaces of the substrate; an encapsulation layer covering at least part of the first portion of the substrate; and an adhesion layer between the encapsulation layer and the substrate in at least one location; wherein a maximum thickness of the substrate in the second portion is equal to or less than 0.5 millimeters.

Additionally disclosed herein is a method of manufacturing an implantable stimulator, the method comprising: providing a substrate, the substrate comprising a first surface and a second surface, wherein a thickness of the substrate is defined by the first and second surfaces; providing a pulse generator, the pulse generator being configured to generate at least one stimulation pulse; locating at least two electrodes along a conformable portion of the substrate; depositing or electro-plating onto the substrate a plurality of electrical interconnections electrically coupling the pulse generator to the at least two electrodes; applying an adhesion layer at least partially covering the substrate; and applying an encapsulation layer over the adhesion layer; wherein the thickness of the substrate along the conformable portion is equal to or less than 0.5 millimeters.

In some embodiments of the method, the adhesion layer is applied using atomic layer deposition (ALD). In further embodiments, the pulse generator is provided along a further portion of the substrate, wherein the adhesion layer and encapsulation layer are applied to at least partially cover the further portion of the substrate.

### BRIEF DESCRIPTION OF THE DRAWINGS

Certain illustrative embodiments illustrating organization and method of operation, together with objects and advantages may be best understood by reference to the detailed description that follows, taken in conjunction with the accompanying drawings, which are not necessarily drawn to scale.

The accompanying drawings, which are incorporated herein and form a part of the specification, illustrate exemplary embodiments and, together with the description, further serve to enable a person skilled in the pertinent art to make and use these embodiments and others that will be apparent to those skilled in the art:
FIG. 1A is a transverse view of a first implementation of an implantable stimulator consistent with certain embodiments of the present invention.
FIG. 1B is a top view of a first implementation of an implantable stimulator consistent with certain embodiments of the present invention.
FIG. 1C is a bottom view of a first implementation of an implantable stimulator consistent with certain embodiments of the present invention.
FIG. 2A is a transverse view of a second implementation of an implantable stimulator consistent with certain embodiments of the present invention.
FIG. 2B is a top view of a second implementation of an implantable stimulator consistent with certain embodiments of the present invention.
FIG. 2C is a bottom view of a second implementation of an implantable stimulator consistent with certain embodiments of the present invention.
FIG. 3A is a transverse view of a third implementation of an implantable stimulator consistent with certain embodiments of the present invention.
FIG. 3B is a top view of a third implementation of an implantable stimulator consistent with certain embodiments of the present invention.
FIG. 3C is a bottom view of a third implementation of an implantable stimulator consistent with certain embodiments of the present invention.
FIG. 4A is a first view of alternative electrode configurations of an implantable stimulator consistent with certain embodiments of the present invention.
FIG. 4B is a second view of alternative electrode configurations of an implantable stimulator consistent with certain embodiments of the present invention.
FIG. 4C is a third view of alternative electrode configurations of an implantable 30 stimulator consistent with certain embodiments of the present invention.
FIG. 5 presents locations of nerves in the anterior portion of a human head that may be treated through operation of an implantable stimulator consistent with certain embodiments of the present invention.
FIG. 6 presents locations of nerves in the posterior portion of a human body that may be treated through operation of an implantable stimulator consistent with certain embodiments of the present invention.
FIG. 7 presents locations of nerves in a human body that may be treated through operation of an implantable stimulator consistent with certain embodiments of the present invention.
FIG. 8 is a transverse view of a further implementation of an implantable stimulator consistent with certain embodiments of the present invention.
FIG. 9 is a transverse view of a further implementation of an implantable stimulator consistent with certain embodiments of the present invention.
FIG. 10A to 10F are bottom views of implementations of a mechanical brace consistent with certain embodiments of the present invention.
FIG. 11A, FIG. 11B and FIG. 11C depict cross-sections through improved implantable electrical devices.
FIG. 12A and FIG. 12B depict cross-sections through improved implantable medical devices comprising an improved implantable electrical device, and one or more electrodes.
FIG. 13A is a bottom view of a further implementation of an implantable stimulator consistent with certain embodiments of the present invention.
FIG. 13B is a transverse view of a further implementation of an implantable stimulator consistent with certain embodiments of the present invention.
FIG. 14A is a transverse view of a further implementation of an implantable stimulator consistent with certain embodiments of the present invention.
FIG. 14B is a bottom view of a further implementation of an implantable stimulator consistent with certain embodiments of the present invention.
FIG. 15 presents measurement results comparing the average pull force , dry and after soaking, of LCP coated with PDMS using different processes.

### DETAILED DESCRIPTION

While this invention is susceptible of embodiment in many different forms, there is shown in the drawings and will herein be described in detail specific embodiments, with the understanding that the present disclosure of such embodiments is to be considered as an example of the principles and not intended to limit the invention to the specific embodiments shown and described. The embodiments described, and their detailed construction and elements, are merely provided to assist in a comprehensive understanding of the invention. The scope of the invention is best defined by the appended claims. In the description below, like reference numerals are used to describe the same, similar or corresponding parts in the several views of the drawings or even in different drawings.

Thus, it is apparent that the present invention can be carried out in a variety of ways, and does not require any of the specific features described herein. Also, well-known functions or constructions are not described in detail since they would obscure the invention with unnecessary detail. Any signal arrows in the drawings/figures should be considered only as exemplary, and not limiting, unless otherwise specifically noted,

It will be understood that, although the terms first, second, etc. may be used herein to describe various elements, these elements should not be limited by these terms. These terms are only used to distinguish one element from another. For example, a first element could be termed a second element, and, similarly, a second element could be termed a first element, without departing from the scope of example embodiments. As used herein, the term "and/or" includes any and all combinations of one or more of the associated listed items. As used herein, "at least one of A, B, and C" indicates A or B or C or any combination thereof. As used herein, the singular form of a word includes the plural, and vice versa, unless the context clearly dictates otherwise.

The terms "a" or "an", as used herein, are defined as one or more than one. The term "plurality", as used herein, is defined as two or more than two. The term "another", as used herein, is defined as at least a second or more. The terms "including" and/or "having", as used herein, are defined as comprising (i.e., open language). The term "coupled", as used herein, is defined as connected, although not necessarily directly, and not necessarily mechanically.

It should also be noted that in some alternative implementations, the functions/acts noted may occur out of the order noted in the figures. For example, two figures shown in succession may in fact be executed substantially concurrently or may sometimes be executed in the reverse order, depending upon the functionality/acts involved.

As used herein, ranges are used herein in shorthand, so as to avoid having to list and describe each and every value within the range. Any appropriate value within the range can be selected, where appropriate, as the upper value, lower value, or the terminus of the range.

The words "comprise", "comprises", and "comprising" are to be interpreted inclusively rather than exclusively. Likewise the terms "include", "including" and "or" should all be construed to be inclusive, unless such a construction is clearly prohibited from the context. The terms "comprising" or "including" are intended to include embodiments encompassed by the terms "consisting essentially of" and "consisting of". Similarly, the term "consisting essentially of" is intended to include embodiments encompassed by the term "consisting of". Although having distinct meanings, the terms "comprising", "having", "containing" and "consisting of" may be replaced with one another throughout the description of the invention.

"About" means a referenced numeric indication plus or minus 10% of that referenced numeric indication. For example, the term "about 4" would include a range of 3.6 to 4.4. All numbers expressing quantities of ingredients, reaction conditions, and so forth used in the specification are to be understood as being modified in all instances by the term "about." Accordingly, unless indicated to the contrary, the numerical parameters set forth herein are approximations that can vary depending upon the desired properties sought to be obtained. At the very least, and not as an attempt to limit the application of the doctrine of equivalents to the scope of any claims, each numerical parameter should be construed in light of the number of significant digits and ordinary rounding approaches.

Wherever the phrase "for example," "such as," "including" and the like are used herein, the phrase "and without limitation" is understood to follow unless explicitly stated otherwise.

"Typically" or "optionally" means that the subsequently described event or circumstance may or may not occur, and that the description includes instances where said event or circumstance occurs and instances where it does not.

Reference throughout this document to "one embodiment", "certain embodiments", "an embodiment" or similar terms means that a particular feature, structure, or characteristic described in connection with the embodiment is included in at least one embodiment of the present invention. Thus, the appearances of such phrases or in various places throughout this specification are not necessarily all referring to the same embodiment. Furthermore, the particular features, structures, or characteristics may be combined by a person skilled in the art in any suitable manner in one or more embodiments without limitation.

In the following detailed description, numerous non-limiting specific details are given to assist in understanding this disclosure.

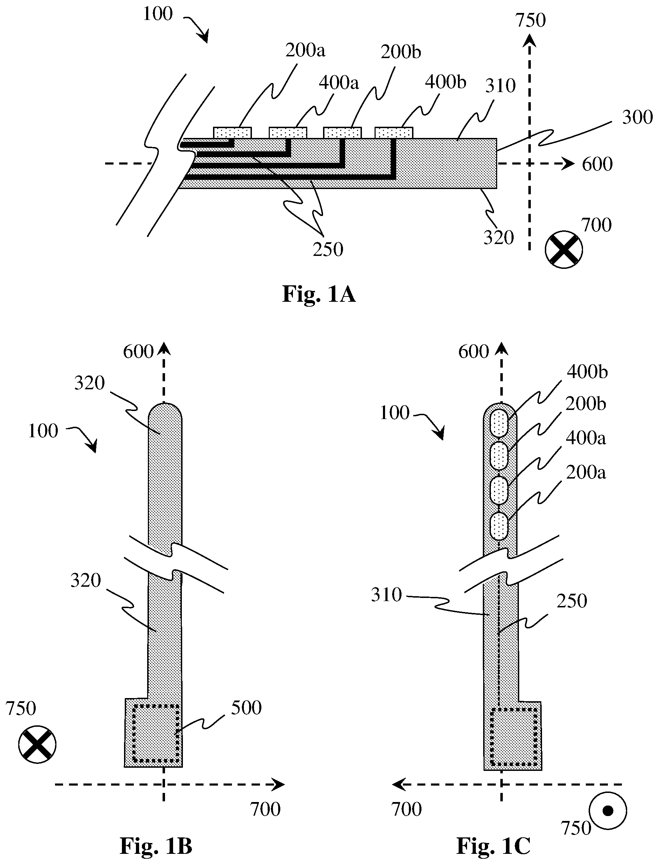

FIG. 1A, 1B & 1C depict longitudinal cross-sections through a first embodiment 100 of an implantable stimulator comprising:
- a pulse generator 500 (only depicted in FIG. 1B and 1C) for generating at least one electrical treatment stimulation pulse; and
- a conformable portion of a foil-like substrate 300 having a longitudinal axis 600 extending from the pulse generator 500 to a distal end of the substrate 300. The substrate 300 comprises one or more adjacent polymeric substrate layers and has a first 310 and a second 320 planar (outer) surface.

The implantable stimulator 100 also comprises:
- an electrode array 200, 400, proximate the distal end, having at least one electrode of a first 200a, 200b type and at least one electrode of a second type 400a, 400b. The electrodes 200, 400 are comprised in the first 310 or second 320 surface, and each is configurable for transferring treatment energy, in use, to (as a stimulation electrode) and/or from (as a return electrode) human or animal tissue. In this context, an array may be considered a systematic arrangement of two or more electrodes 200a, 200b, 400a, 400b. 1D, 2D or 3D arrays may be provided. Optionally, they may be arranged in rows and/or columns.

The implantable stimulator 100 further comprises:
- one or more electrical interconnections 250, between the pulse generator 500 and the first 200a, 200b and the second 400a, 400b electrodes, for transferring electrical energy as one or more electrical treatment stimulation pulses to the coupled first electrodes 200a, 200b and/or the second electrodes 400a, 400b. The one or more electrical interconnections 250 are comprised (or positioned) between the first surface 310 and the second 320 surfaces. A plurality of electrical interconnections 250 is considered to be two, or more than two, electrical interconnections 250.

In this disclosure, the conformability of the electrode array 200, 400 is determined to a high degree by the one or more of the following:
- the conformability of a portion of the substrate 300 proximate the electrodes 200, 300;
- the arrangement and positions of the electrodes 200, 400;
- the materials and dimensions (or extent) of the materials comprised in the electrodes 200, 400;
- the arrangement and positions of the one or more interconnections 250 proximate the electrodes 200, 400; and
- the materials and dimensions (or extent) of the materials comprised in the interconnections 200, 400.

By suitable configuration, arrangement and optimization, an implantable electrode array 200, 400 may be provided which is foil-like (or film-like) and highly conformable.

As depicted, the conformable portion of the foil-like substrate 300 is preferably elongated along the longitudinal axis 600, having a tape-like shape, allowing the pulse generator 500 to be disposed (or located) further away from the position of the electrodes 200, 400.

If the substrate 300 is substantially planar (in a non-limiting example, by allowing the substrate 300 to conform to a planar surface), the first 310 and second 320 surfaces are disposed along substantially parallel transverse planes 600, 700. As depicted in FIG. 1A, the first surface 310 lies in a plane comprising the longitudinal axis 600 and a first transverse axis 700 - the first transverse axis 700 is substantially perpendicular to the longitudinal axis 600. As depicted in FIG. 1A, the plane of the first surface 310 is substantially perpendicular to the plane of the cross-section drawing (substantially perpendicular to the surface of the paper).

The conformable portion of the foil-like substrate 300 has a maximum thickness of 0.5 millimeter or less, proximate the first 200a, 200b and second 400a, 400b electrodes, the thickness being defined by the first 310 and second surfaces 320 - it may be determined by a perpendicular distance between corresponding points on the first 310 and second planar surfaces 320. This is preferably determined when the substrate 300 conforms to a planar surface.

The foil-like substrate 300 has a thickness or extent along a second transverse axis 750 - this second transverse axis 750 is substantially perpendicular to both the longitudinal axis 600 and the first transverse axis 700 - it lies in the plane of the drawing (along the surface of the paper) as depicted. The first surface 310 is depicted as an upper surface and the second surface 320 is depicted as a lower surface.

The thickness may therefore be determined by a perpendicular distance along the second transverse axis 750 between corresponding points on the first 310 and second planar surfaces 320. The maximum thickness of the conformable portion of the foil-like substrate 300 proximate the first 200a, 200b and second 400a, 400b electrodes is 0.5mm or less, preferably 0.3 millimeters or less, even more preferably 0.2 millimeters or less, yet more preferably 0.1 millimeters or less.

In general, the lower the maximum thickness (in other words, the thinner the substrate), the higher the degree of conformance. However, a higher maximum thickness may be preferred to improve mechanical strength.

To clarify the differences between the different views depicted, the axes are given nominal directions:
- the longitudinal axis 600 extends from the proximal end (not depicted in FIG. 1A, but depicted in FIG. 1B and 1C) on the left, to the distal end, depicted on the right of the page;
- the first transverse axis 700 extends into the page as depicted; and
- the second transverse axis 750 extends from bottom to top as depicted.

The conformable portion of the foil-like substrate 300 may be configured and arranged as a multilayer - it comprises two or more adjacent polymeric substrate layers secured to each other, and having the first 310 and second 320 planar surface. The one or more electrical interconnections 250 are also comprised (or positioned) between the first 310 and second 320 planar surfaces. However, it is not necessary that the two or more polymeric layers and /or interconnections have similar extents along the first transverse axis 700. In other words, within the context of this disclosure, there may be regions where an interconnection 250 is sandwiched between regions of polymeric substrate (appears as a multilayer in a longitudinal cross-section), adjacent to regions where the polymeric substrate is substantially contiguous. Similarly, there may be regions where an interconnection 250 is sandwiched between two polymeric substrate layers (appears as a multilayer in a longitudinal cross-section), adjacent to regions where the substrate comprises two adjacent substrate layers. Similarly, a substrate comprising two or more polymeric substrate layer may be modified (physically and/or chemically), such that it appears to be one layer of polymeric substrate.

These polymeric substrate layers are selected for suitability to be conformable, and to comprise the one or more electrical interconnections 250. Preferably, the polymeric substrate materials are also biocompatible and durable, such as a material selected from the group comprising silicone rubber, siloxane polymers, polydimethylsiloxanes, polyurethane, polyether urethane, polyetherurethane urea, polyesterurethane, polyamide, polycarbonate, polyester, polypropylene, polyethylene, polystyrene, polyvinyl chloride, polytetrafluoroethylene, polysulfone, cellulose acetate, polymethylmethacrylate, polyethylene, and polyvinylacetate. Suitable polymer materials, including LCP (Liquid Crystal Polymer) films, are described in "Polymers for Neural Implants", Hassler, Boretius, Stieglitz, Journal of Polymer Science: Part B Polymer Physics, 2011, 49, 18-33 (DOI 10.1002/polb.22169), In particular, Table 1 is included here as reference, depicting the properties of Polyimide (UBE U-Varnish-S), Parylene C (PCS Parylene C), PDMS (NuSil MED-1000), SU-8 (MicroChem SU-8 2000 & 3000 Series), and LCP (Vectra MT1300).

Conformable foil-like substrates 300 are configured to follow the contours of the underlying anatomical features very closely by being flexible. Very thin foil-like substrates 300 have the additional advantage that they have increased flexibility.

Most preferably, the polymeric substrate layers comprise an LCP, Parylene and/or a Polyimide. LCP's are chemically and biologically stable thermoplastic polymers which allow for hermetic sensor modules having a small size and low moisture penetration.

Advantageously, an LCP may be thermoformed allowing complex shapes to be provided. Very thin (and subsequently very conformable) and very flat (highly planar) layers of an LCP may be provided. For fine tuning of shapes, a suitable laser may also be used for cutting.

In a non-limiting example, a conformable foil-like substrate 300 of LCP may have a thickness (extent along the second transverse axis 750) in the range 50 microns (um) to 720 microns (um), preferably 100 microns (um) to 300 microns (um). In an exemplary embodiment, values of 150 um (micron), 100um, 50um, or 25um may be provided.

When conforming to a substantially planar surface, the foil-like surface 300 is substantially comprised in a plane with a transverse extent substantially perpendicular to the longitudinal axis 600, wherein the planar width may be determined by a perpendicular distance between corresponding points on outer surfaces edges of the planar foil-like substrate 300 along the transverse extent. As depicted, this is along the first transverse axis 700. In an embodiment, electrode 200, 400 widths of 2 mm to 20 mm may be provided using LCP.

At room temperature, thin LCP films have mechanical properties similar to steel. This is important as implantable substrates 300 should be strong enough to be implanted, strong enough to be removed (explanted) and strong enough to follow any movement of the neighboring anatomical features and/or structures without deteriorating.

LCP belongs to the polymer materials with the lowest permeability for gases and water. LCP's can be bonded to themselves, allowing multilayer constructions with a homogenous structure.

In contrast to LCP's, polyimides are thermoset polymers, which require adhesives for the construction of multilayer portions with electrode arrays. Polyimides are thermoset polymer material with high temperature and flexural endurance.

In an embodiment, an LCP may be used to provide a conformable substrate 300 as a multilayer - in other words, two or more adjacent polymeric substrate layers. In a non-limiting example, these may be layers of 25 um (micron) thickness.

In an embodiment, one or more electrical interconnections 250 may be provided (or positioned) between the first 310 and second 320 surfaces by metallization. These may be conductors embedded in the substrate 300 such as by having a single polymer layer and applying conductive material using suitable deposition techniques known from the semiconductor industry.

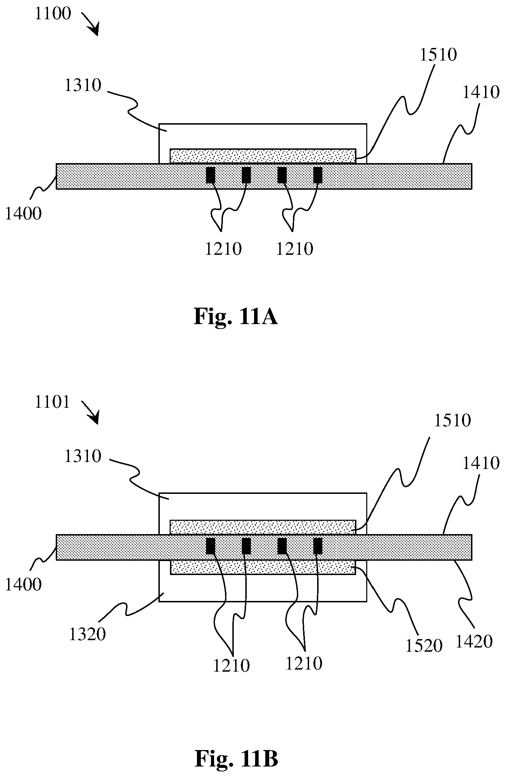

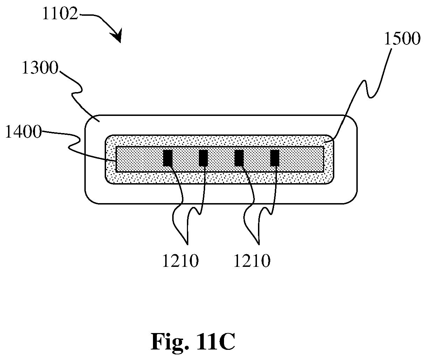

In an embodiment, if two or more adjacent polymeric substrate layers are provided, an interconnection layer may be provided using suitable techniques such as those from the semiconductor industry. The polymeric substrate layers may also be considered adjacent when one of more adhesion layers are used between them. Examples of suitable adhesion materials and adhesion layers are described below in relation to FIG. 11 to FIG. 12.

In an embodiment, lamination may also be used to provide a substrate 300 with the desired physical and chemical properties, and/or to provide a convenient method of manufacture. In a non-limiting example, a substrate 300 may comprise three laminated polymer layers: two high temperature thermoplastic layers with a low-temperature layer (bond-ply) in between, and high-temperature layers towards the first surface 310 and second surface 320.

In an alternative embodiment, two layers of silicone may be provided as polymeric substrate layers: one layer of silicone is provided, metal is patterned on one of its outer surfaces, and a second layer of silicone is added over the metal patterning by jetting, over-molding, or spin-coating.

In an embodiment, the electrical interconnections 250 may comprise one or more conductive materials, such as a metal, formed as required in one or more conductive elements: wire, strand, foil, lamina, plate, and/or sheet. They may be a substantially contiguous (one conductor). They may also comprise more than one conductor, configured and arranged to be, in use, electrically connected with each other - in other words, the one or more conductors are configured and arranged to be substantially electrically contiguous in use.

Alternatively, the one or more electrical interconnections 250 may be comprised in one or more conductive interconnection layers 250, the one or more conductive interconnection layers being comprised (or positioned) between two adjacent polymeric substrate layers. As depicted in FIG. 1A, a plurality of interconnections may be provided at different dispositions (or depths or positions) between the first surface 310 and the second surface 320.

In an embodiment, an interconnection 250 in the context of this disclosure is not configured or arranged to be, in use, in contact with human or animal tissue. The one or more interconnections 250 are embedded (or covered) in one or more layers of a low conductance or insulating polymer, such as LCP. Additionally or alternatively, one or more encapsulation layers may be used.

One or more interconnection layers 250 may also be provided by metallization using techniques from the PCB (Printed Circuit Board) industry, such as metallization with a bio-compatible metal such as gold or platinum. Electro-plating may be used. Layers comprising LCP films are particularly suitable for metallization. These electrical interconnections 250 and/or interconnect layers 250 are configured to transfer electrical energy as one or more electrical treatment stimulation pulses from the pulse generator 500 to the coupled first electrodes 200a, 200b and/or the second electrodes 400a, 400b.

Using suitable polymeric substrate materials, such as an LCP film, allows the conformable portions of the foil-like (or film-like) substrate 300 and electrode array 200, 300 to have a high width-to-height ratio, providing a bio-compatible electronic foil (or film), or bio-electronic foil (or film).

In an embodiment, when the substrate 300 conforms to a substantially planar surface, the ratio of maximum planar width to maximum thickness proximate the first 200a, 200b and second 400a, 400b electrodes may be 7:1 or higher, preferably 10:1 or higher, more preferably 15:1 or higher, yet more preferably 30:1 or higher, even more preferably 50:1 or higher.

Ratios of 100:1 or higher may also be advantageous, and may be provided using one or more mechanically strong substrate layers of an LCP film, with a width of approximately 20mm and a thickness of approximately 0.2 mm. This provides a high degree of flexibility, and therefore also a high degree of conformability. Additional measures may also be taken to increase the degree of conformability in the first transverse direction 700, such as varying the width of the substrate, adding one or more undulations and/or providing bending points.

In a non-limiting example, when using a single row of electrodes 200, 400 and/or electrodes 200, 400 with a smaller width, the width may be four mm with a thickness of approximately 0.2mm - this is a ratio of approximately 20:1.

In a non-limiting example, in a portion of the substrate proximate the pulse generator 500, greater extents may be required which further depend, to a high degree, on the dimensions of the electronic components used a width of twenty mm and a thickness of three mm. This is a ratio of approximately 6.67:1.

As depicted in FIG. 1A, the distal end (or distal portion) of the conformable foil-like substrate 300 comprises:
- two electrodes 200a, 200b of a first type, comprised in the first surface 310, and
- two electrodes 400a, 400b of a second type, also comprised in the first surface 310. From proximal to distal end, the order depicted is 200a, 400a, 200b, 400b - in other words, each electrode of the first type 200a, 200b is proximate an electrode of the second type 400a, 400b and comprised in the same surface 310.

The foil-like substrate 300 comprises an electrical interconnection 250 between each electrode 200a, 400a, 200b, 400b and the pulse generator. In this embodiment, each electrical interconnection 250 is configured and arranged such that each electrode 200a, 400a, 200b, 400b is electrically connected substantially independently - consequently, one of the operating modes available by suitably configuring the pulse generator 500 is substantially independent operation. The pulse generator 500 may be configured using one or more hardware, firmware and/or software parameters.

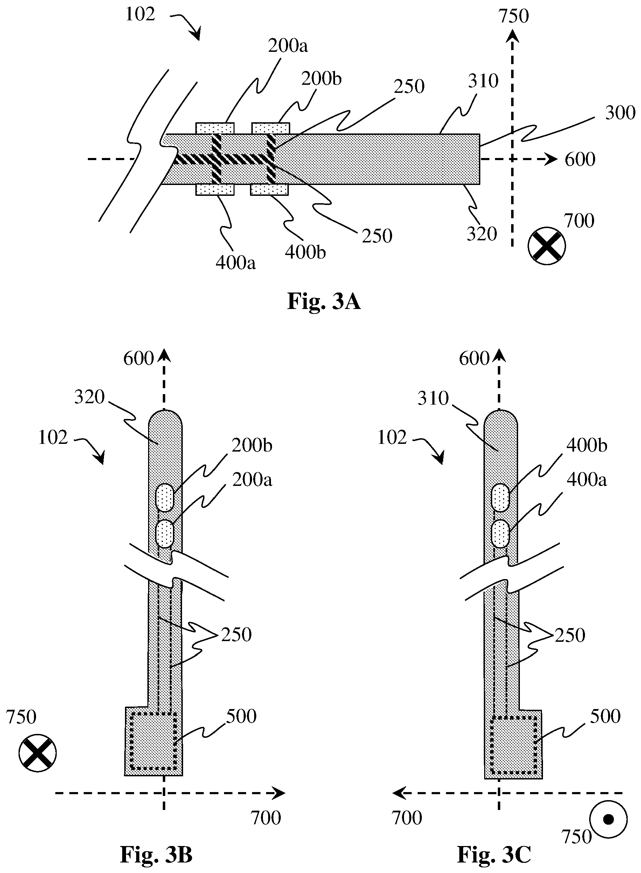

Although depicted in FIG. 1A as individual connections 250 at different distances (or positions) between the first 310 and second 320 surfaces, the skilled person will also realize that the same interconnections may be provided by a suitably configured interconnections 250 (or an interconnection layer 250) at approximately the same distance (or position) between the first 310 and second 320 surfaces, similar to the embodiment depicted in FIG. 3B, and described below.

"Comprised in" the first 310 or second 320 surface means that the electrodes 200a, 400a, 200b, 400b are relatively thin (such as when the substrate is arranged to conform to a substantially planar surface, it may have an extent along the second transverse axis of 20 to 50 microns or less. Thinner electrodes may also be used to further increase the degree of conformability, such as 1 micron or less), and attached to (or at least partially embedded in) the surface.

The electrodes 200, 400 may comprise a conductive material such as gold, platinum, platinum black, TiN, IrO₂, iridium, and/or platinum/iridium alloys and/or oxides. Conductive polymers, such as Pedot, may also be used. Preferably, bio-compatible conductive materials are used. PCB/metallization techniques may be used to manufacture them on or in the first 310 and/or second 330 surfaces of the one or more polymeric substrate layers.

Thicker metal layers are generally preferred over thinner metal layers for electrodes 200a, 200b, 400a, 400b because they can be subjected to bodily substances that may dissolve the metal. However, thicker metal layers typically increase rigidity (reduce conformability) proximate the thicker layer.

The stimulator 100 may be implanted by first creating a subcutaneous tunnel and/or using an implantation tool. However, the high degree of conformability may make successful implantation more difficult. Even when using a suitable insertion tool, the electrode positions may be found later to be incorrect due to misalignment, lead migration during implantation, or lead migration after transplantation.

At least the distal end comprising the electrode array 200, 400, is implanted. However, it may be advantageous to implant the stimulator 100.

In addition, during implantation, it may be difficult to precisely identify the desired position for the stimulation. When implanted, the stimulator electrodes should be positioned sufficiently close to the nerve to be stimulated. But nerve pathways may not always be clearly visible to the professional performing the implantation, and the disposition and path of the nerve pathways vary greatly from person-to-person.

As depicted in FIG. 1, there is no substantial hardware difference between the first-type 200a, 200b and second type 400a, 400b electrodes - any difference in functionality is determined in this implementation mainly by the configuration (one or more hardware, firmware and/or software parameters) of the pulse generator 500. There may be a smaller influence on the electrical properties due to the arrangement and routing of the interconnections 250.

One or more coupled electrodes of the same type 200a, 200b or 400a, 400b may be operated substantially the same by suitable configuration of the pulse generator 500 - in other words, the stimulation energy applied to the electrodes 200, 400 is substantially the same at substantially the same time instance (usually measured as a voltage, a current, a power, a charge, or any combination thereof). This may also be used to anticipate and/or correct for a misalignment and/or lead migration - this is advantageous as it allows the configuration to be performed at least partially using software.

Additionally or alternatively, two or more electrodes 200, 400 may be configured and arranged using one or more parameters of the pulse generator 500 as a stimulation electrode or a return electrode. This may provide a higher degree of configurability as it only becomes necessary to implant the substrate 300 such that at least two of the electrodes are proximate the desired stimulation location.

In this embodiment 100, the electrodes of the first type 200a, 200b are nominally configured and arranged to be operated as a stimulation electrode.

The electrodes of the second type 400a, 400b are nominally configured to be operated as a return electrode - each is configured to provide, in use, an electrical return for one or more stimulation electrode 200a, 200b. In other words, the electrical return 400a, 400b closes the electrical circuit. It may also be similarly configured to provide an electrical ground for a corresponding electrical energy source.

Three configurations are thus provided based on this nominal configuration: either:
- a stimulation / return electrode pair 200a / 400a proximate the first surface 310 at that stimulation / return location; or
- a stimulation / return electrode pair 200b / 400b proximate the first surface 310 at that stimulation / return location; or
. a combination thereof.

In an embodiment, one or more stimulation electrodes 200a, 200b may be provided in such a stimulator 100. The number, dimensions and/or spacings of the stimulating electrodes 200a, 200b may be selected and optimized depending on the treatment. In an embodiment, if more than one stimulation electrode 200a, 200b is provided, each stimulation electrode 200a, 200b may provide:
- a different stimulation effect, a similar stimulation effect or the same stimulation effect.

To avoid a misalignment, a selection may be made of one or two electrodes 200a, 200b proximate the tissues where the effect is to be created.

Two or more stimulation electrodes 200a, 200b may be made active at substantially the same time if stimulation over a larger area is required and/or at a location between the active stimulation electrodes 200a, 200b.

In an embodiment, a stimulation electrode 200a, 200b may have dimensions in the order of six to eight mm along the longitudinal axis 600, and three to five mm along the first transverse axis 700, so approximately 18 to 40 square mm (mm²).

In an embodiment, a foil-like substrate 300, suitable for an implantable stimulator, may comprise up to twelve stimulation 200a, 200b and return 400a, 400b electrodes over a length of 15cm to allow for a correction for misalignment, or to simply allow the specialist to select the most effective stimulation location.

In an embodiment, FIG. 1B depicts a view of the second surface 320 of the implantable distal end (or portion) of the foil-like substrate 300 depicted in FIG. 1A. In other words, the second surface 320 is depicted in the plane of the paper, lying along the longitudinal axis 600 (depicted from bottom to top) and in the first transverse axis 700 (depicted from left to right). The second transverse axis 750 extends into the page. The first surface 310 is not depicted in FIG. 1B, but lies at a higher position along the second transverse axis 750 (into the page), and is also substantially parallel to the plane of the drawing. The foil-like substrate 300 is arranged to conform to a substantially planar surface.

The pulse generator 500 may be disposed (or positioned) between the second 320 surface and the first 310 surface. In FIG. 1B and 1C, it is depicted with dotted lines. Alternatively, the pulse generator 500 may be at least partially disposed on the first surface 310 or on the second surface 320. Alternatively, the pulse generator 500 may be at least partially embedded in the first surface 310 or in the second surface 320.

Depending on the degree of embedding and the one or more electrical components used for the pulse generator 500, the maximum thickness may be optimized. Components may be thinned to minimize the thickness. If the substrate 300 is configured and arranged to be conformable and/or foil-like, the maximum thickness of the implantable stimulator 100 in a portion of the substrate proximate the pulse generator 500 may be five millimeters or less, preferably four millimeters or less, even more preferably three millimeters or less, the thickness being determined by a perpendicular distance between corresponding points on outer planar surfaces when the implantable stimulator 100 conforms to a substantially planar surface. Additional optional electrical components, such as an antenna, comprising a coil or dipole or fractal antenna, may also influence the thickness depending on the degree that they are embedded in the substrate.

The stimulator 100 and the foil-like substrate 300 extend along the first transverse axis 700 (considered the planar width of the stimulator 100 / foil-like substrate 300 when conforming to a substantially planar surface). As depicted, the planar width in a portion of the substrate proximate the pulse generator 500 may be greater than the planar width in another portion of the substrate proximate the electrodes 200a, 200b, 400a, 400b at the distal end (or portion) of the foil-like substrate 300. The planar width proximate the pulse generator 500 may depend on the hardware and components used for the pulse generator 500 - typically, it is at least the width of the integrated circuit used for the pulse generator 500. Additional optional electrical components, such as an antenna comprising a coil or dipole or fractal antenna, may also influence the planar width.

In an embodiment, the planar width proximate the electrodes 200a, 200b, 400a, 400b may depend on the conductors used for the electrodes 200a, 200b, 400a, 400b and the one or more interconnections 250. In an embodiment, the planar width is at least the width of the first electrode 200a, 200b or the second electrode 400a, 400b.

In an embodiment, FIG. 1C depicts a view of the first surface 310 of the implantable distal end (or portion) of the foil-like substrate 300 depicted in FIG. 1A and 1B. In other words, the first surface 310 is depicted in the plane of the paper, lying along the longitudinal axis 600 (depicted from bottom to top) and in the first transverse axis 700 (depicted from right to left). The second transverse axis 750 extends out of the page. This is the view facing the animal or human tissue which is stimulated (in use). The second surface 320 is not depicted in FIG. 1C, but lies at a lower position along the second transverse axis 750 (into the page), and is also substantially parallel to the plane of the drawing. The foil-like substrate 300 is arranged to conform to a substantially planar surface.

The one or more interconnections 250 are disposed (or positioned) between the first 310 surface and the second 320 surface, as depicted in FIG. 1A. In FIG. 1C, they are depicted as dotted lines, representing the interconnections 250 (or suitably configured one or more interconnection layers 250) that have been provided for each of the electrodes 200a, 200b, 400a, 400b in this embodiment. A single dotted line 250 is depicted between the pulse generator 500 and the electrodes 200, 400 to indicate, in embodiment 100, that the interconnections 250 are at approximately the same disposition along the first transverse axis 700.

As depicted in FIG. 1C, the electrodes 200a, 200b, 400a, 400b each have a longitudinal extent (length) along the longitudinal axis 600 and a transverse extent (width) along the first transverse axis 700.

Although depicted as similar, in practice, each electrode 200a, 200b, 400a, 400b may vary in shape, transverse cross-section, orientation and/or size (or extent), depending on the intended use and/or the desired degree of configurability.

After implantation of the stimulator 100, or at least of the distal end (or portion) comprising the electrode array 200, 400, the pulse generator 500 may be configured and arranged to provide, in use, electrical energy to the one or more coupled electrodes of the first type 200a, 200b with respect to the electrical return applied to the one or more coupled electrodes of the second type 400a, 400b.

The configurability of the stimulator 100 allows, before, during and/or after implantation of at least of the distal end (or portion) comprising the electrode array 200, 400, the operation of the one or more electrodes 200a, 200b, 400a, 400b to be determined and/or adapted. The operation may also be reconfigured one or more times during the period that the stimulator 100 is implanted to optimize and/or prolong treatment.

In an embodiment, the pulse generator 500 may be initially configured to nominally operate 200a and 400a as respectively a stimulation / return electrode pair. After implantation of at least the distal end 200, 400, insufficient stimulation may be observed and/or measured. If it is assumed to be due to a mainly longitudinal misalignment, the pulse generator 500 may be alternatively configured, using one or more parameters, to nominally operate 200b and 400b as respectively a stimulation / return electrode pair.

The stimulator 100 may be further configured and arranged to switch the pulse generator 500 under predetermined and/or controlled conditions between these configurations. It may be convenient to further consider these configurations as a first and second electrode modes, and allow a user to select a mode as a preference and/or switch mode. Alternatively, the pulse generator 500 may switch modes under predetermined and/or controlled conditions.

Additionally or alternatively, other modes may also be provided for configuring the pulse generator 500 to operate in:
- a first electrode mode, wherein electrical stimulation energy is provided to one or more coupled electrodes of the first type 200a, 200b as one or more electrical treatment stimulation pulses, the one or more coupled electrodes of the second type 400a, 400b being configured to provide, in use, a corresponding electrical return for the one or more first electrodes 200a, 200b; or
- a second electrode mode, wherein energy is provided to one or more coupled electrodes of the second type 400a, 400b as one or more electrical treatment stimulation pulses, the one or more coupled electrodes of the first type 200a, 200b being configured to provide, in use, a corresponding electrical return for the one or more second electrodes 400a, 400b.

Again, the stimulator 100 may be further configured and arranged to switch the pulse generator 500 under predetermined and/or controlled conditions between these configurations or modes. Additionally or alternatively, a user may be allowed to select a mode as a preference and/or switch mode.

The skilled person will realize that the electrodes 200a, 200b, 400a, 400b may be configured to operate in more complex configurations, such as:
- 400a and 200a may be operated as respectively a stimulation / return electrode pair (reversing the original intended operation);
- 400b and 200b may be operated as respectively a stimulation / return electrode pair;
- if an intermediate stimulation is preferred, two or more electrodes 200a, 200b, 400a, 400b may be operated substantially simultaneously as one or more stimulation electrodes;
- one or more electrodes 200a, 200b, 400a, 400b may be operated as one or more return electrodes;
- electrode 400a operated as a stimulation electrode, in combination with electrode 200a and electrode 200b as return electrodes;
- electrode 400a and 200b operated as a stimulation electrode, in combination with electrode 200a and electrode 400b as a return electrode.

Alternatively or additionally, the shape, orientation, transverse cross-section, and/or size (or length) of one or more stimulation electrodes may be differently configured compared to one or more return electrodes.

A number of parameters and properties may be considered when configuring and arranging a portion of the foil-like substrate 300 proximate the electrode array 200, 400 for conformability, such as:
- the transverse 700 and/or longitudinal extent 600 of the one or more electrodes 200a, 200b, 400a, 400b
- the thickness of the foil-like substrate 300, or the perpendicular distance between the first surface 310 and the second surface 320
- the materials comprised in the foil-like substrate 300, and their physical properties
- the number and extent of interconnections 250 and/or interconnection layers 250 between the first surface 310 and second surface 320.

There have been attempts to make traditional leads, such as cylindrical leads, much thinner to allow subcutaneous implantation and/or to increase comfort by flattening. But the surface area of the flattened electrodes may become disadvantageously small.

In a non-limiting example, a conventional 0.2mm round lead with 1 cm long electrodes is estimated to result in an electrode with approximately 6 mm² electrode surface.

However, using the conformable electrode arrays described herein, a thin substrate 300 with dimensions of 0.2 mm thick, and four mm wide may be configured and arranged to provide approximately 35 mm² electrode surface in the same length. It is estimated that this may reduce impedance by a factor of approximately 35/6, and reduce power consumption by approximately 35/6.

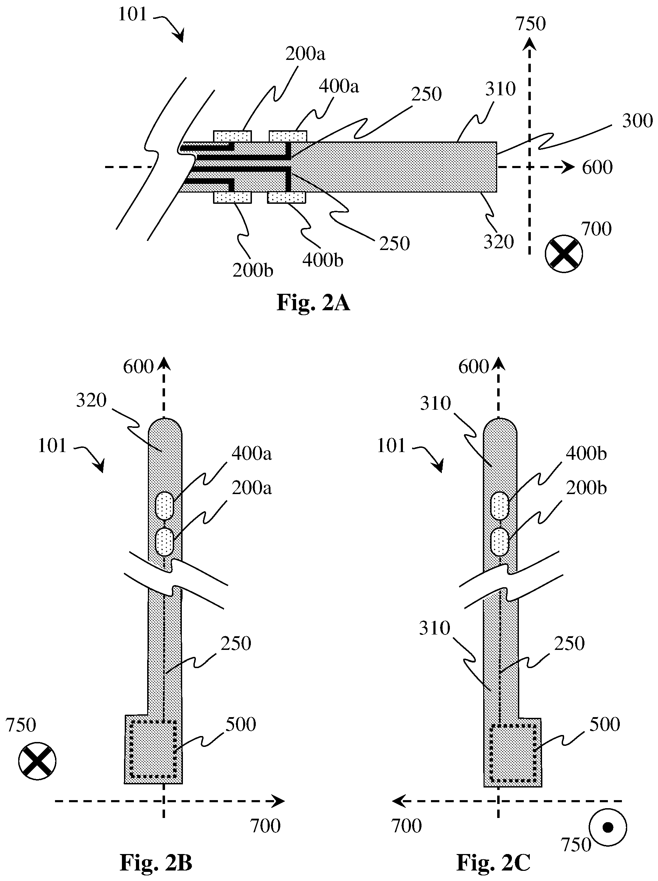

In an embodiment, FIG. 2A, 2B and 2C depict longitudinal cross-sections through a second embodiment 101 of an implantable stimulator. It is similar to the first embodiment 100, depicted in FIG. 1A, 1B and 1C except:
- instead of four electrodes comprised in the first surface 310, this embodiment comprises two electrodes in the first surface 310 - nominally an electrode of the first type 200a and nominally an electrode of the second type 400a. From proximal to distal end, the order depicted is 200a, 400a - in other words, an electrode of the first type 200a is proximate an electrode of the second type 400a in the first surface 310.
- the distal end of the stimulator 101 also comprises two electrodes in the second surface 320 - a further electrode nominally of the first type 200b and a further electrode nominally of the second type 400b. From proximal to distal end, the order depicted is 200b, 400b - in other words, an electrode of the first type 200b is proximate an electrode of the second type 400b in the second surface 320.

- In Fig. 2B, the view of the second surface 320 depicts the two electrodes 200a, 400a comprised in that surface, and one or more interconnections 250 are depicted using a dotted line;
- In Fig. 2C, the view of the second surface 320 depicts the two electrodes 200b, 400b comprised in that surface, and one or more interconnections 250 are depicted using a dotted line;

In this embodiment 101, the electrodes of the first type 200a, 200b are nominally configured and arranged to be operated as a stimulation electrode, and the electrodes of the second type 400a, 400b are nominally configured to be operated as a return electrode.

Three main configurations are thus provided:
- a stimulation / return electrode pair 200a / 400a proximate the first surface 310; or
- a stimulation / return electrode pair 200b / 400b proximate the second surface 320; or
- a combination thereof.

This may be advantageous if it is uncertain whether the implantable distal end of the foil-like substrate 300 may be "above" or "below" the targeted tissue such as "above" or "below" a nerve. This may be determined after implantation by attempting stimulation in each nominal configuration and observing and/or measuring the presence of neural stimulation.

As discussed above, in relation to FIG. 1A, 1B and 1C, each electrode 200a, 200b, 400a, 400b may be operated as one or more stimulation electrodes or operated as one or more return electrodes.

In an embodiment, FIG. 3A, 3B and 3C depict longitudinal cross-sections through a third embodiment 102 of an implantable stimulator. It is similar to the second embodiment 101, depicted in FIG. 2A, 2B and 2C except:
- interconnections 250 are disposed at approximately the same disposition along the second transverse axis 750, as depicted in FIG. 3A. The lines 250 are hatched to indicate that they are not depicted as being in the same longitudinal cross-section - there are interconnections 250 disposed at substantially different positions along the first transverse axis 700;
- interconnections 250 are disposed at substantially different dispositions along the first transverse axis 700, as depicted in FIG. 3B and 3C as two adjacent dashed lines between the electrode array 200, 400 and the pulse generator 500;
- instead of nominally comprising an electrode of the first 200 and second type 400 in the first surface 310, the first surface 310 comprises a first 200a and second 200b electrode nominally of the first type 200;
- instead of nominally comprising an electrode of the first 200 and second type 400 in the second surface 320, the second surface 320 comprises a first 400a and second 400b electrode nominally of the second type 400;

In this embodiment 102, the electrodes of the first type 200a, 200b are nominally configured and arranged to be operated as a stimulation electrode, and the electrodes of the second type 400a, 400b are nominally configured to be operated as a return electrode.

Three main configurations are thus provided:
- a stimulation / return electrode pair 200a / 400a for stimulating between the first surface 310 and second surface 320 proximate the location of this electrode pair; or
- a stimulation / return electrode pair 200b / 400b for stimulating between the first surface 310 and second surface 320 proximate the location of the electrode pair; or
- a combination thereof.

This may be advantageous to correct for a longitudinal misalignment, or to simply allow the healthcare professional to select the most effective stimulation location.

As discussed above, in relation to FIG. 2A, 2B and 2C, each electrode 200a, 200b, 400a, 400b may be operated as one or more stimulation electrodes or operated as one or more return electrodes.

Additionally or alternatively, one or more electrodes of the same type 200a, 200b or 400a, 400b may be electrically connected to each other by suitably configuring the one or more interconnections 250. They will then be operated substantially the same. This may be used to anticipate and/or correct for a misalignment and/or lead migration as longitudinal positioning is less sensitive (a stimulation is provided over a greater longitudinal and or transverse extent).

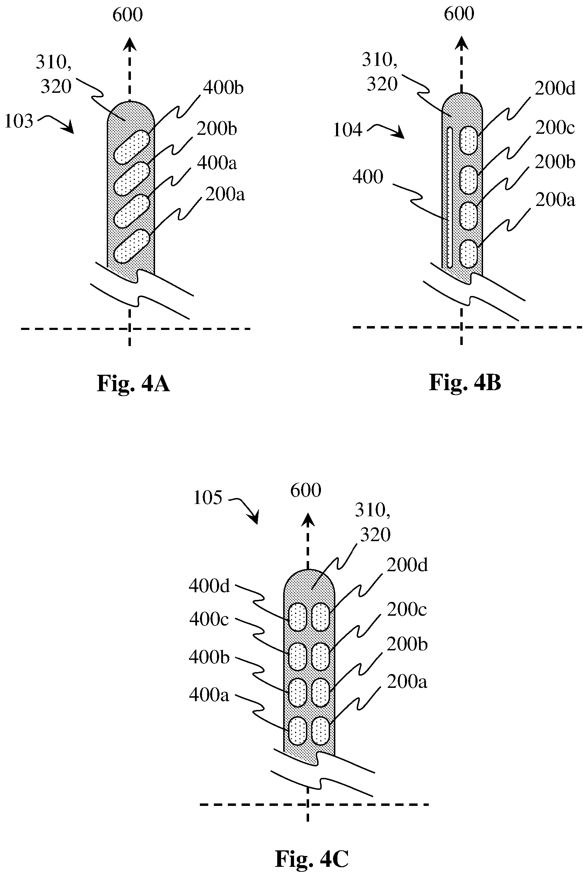

FIG. 4A, 4B and 4C depict alternative electrode array 200, 400 configurations suitable for being comprised in an implantable stimulator 100, 101, 102 as described herein.

FIG. 4A depicts an implantable distal end of a further embodiment 103 of a stimulator. Similar to the distal end depicted in FIG. 1C, the first surface 310 comprises:
- two electrodes 200a, 200b of a first type and two electrodes 400a, 400b of a second type. From proximal to distal end, the order depicted is 200a, 400a, 200b, 400b - in other words, each electrode of the first type 200a, 200b is proximate an electrode of the second type 400a, 400b and comprised in the same surface 310.

The distal end depicted in FIG. 4A is the same as that depicted in FIG. 1A, except:
- the electrodes 200, 400 are extended at angle to the longitudinal axis 600. This may reduce the sensitivity to longitudinal misalignment because the longitudinal locations over which tissue stimulation may be provided are increased.

Additionally or alternatively, the second surface 320 may similarly comprise two electrodes 200a, 200b of the first type and two electrodes 400a, 400b of the second type.

As discussed above, each electrode 200a, 200b, 400a, 400b may be operated as one or more stimulation electrodes or operated as one or more return electrodes.

FIG. 4B depicts an implantable distal end of a further embodiment 104 of a stimulator. Similar to the distal end depicted in FIG. 1C, the first surface 310 comprises four electrodes. However, in this embodiment 104, the first surface 310 comprises:
- four electrodes 200a, 200b, 200c, 200d of a first type and an electrode 400 of a second type. From proximal to distal end, the order depicted is 200a, 200b, 200c, 200d. Transversely adjacent to the four electrodes of the first type 200 is an electrode of the second type 400, extending longitudinally to be adjacent to each electrode of the first type 200.

Nominally, the electrodes of the first type 200 may be operated as one or more stimulation electrodes. The electrode of the second type 400 may be nominally operated as a return electrode for one or more of the stimulation electrodes.

This may reduce the sensitivity to longitudinal misalignment because the four different longitudinal locations are provided which may be selected for stimulation over which tissue stimulation may be provided are increased.

Additionally or alternatively, the second surface 320 may similarly comprise four electrodes 200a, 200b, 200c, 2003 of the first type and one adjacent and longitudinally extended electrode 400 of the second type.

As discussed above, each electrode 200a, 200b, 200c, 200d, 400 may be operated as one or more stimulation electrodes or operated as one or more return electrodes.

FIG. 4C depicts an implantable distal end of a further embodiment 105 of a stimulator. Similar to the distal end depicted in FIG. 4B, the first surface 310 comprises four electrodes 200a, 200b, 200c, 200d of a first type. However, in this embodiment 105, the first surface 310 further comprises four adjacent electrodes 400a, 400b, 400c, 400d of a second type. From proximal to distal end, the order depicted is 200a/400a, 200b/400b, 200c/400c, 200d/400d. Transversely adjacent to each of the four electrodes of the first type 200 is an electrode of the second type 400 at approximately the same disposition along the longitudinal axis 600.

Nominally, the electrodes of the first type 200 may be operated as one or more stimulation electrodes. The electrodes of the second type 400 may be nominally operated as a return electrode for one or more of the stimulation electrodes. Nominally, adjacent electrodes may be considered as a stimulation/return pair 200/400.

In other words, a 2x4 electrode array is provided - two along a transverse axis and four along the longitudinal axis.

This may reduce the sensitivity to longitudinal misalignment because the four different stimulation/return 200/400 pairs are provided at substantially different longitudinal locations are provided which may be selected for stimulation over which tissue stimulation may be provided are increased.

Additionally or alternatively, the second surface 320 may similarly comprise four electrodes 200a, 200b, 200c, 200d of the first type and four adjacent electrodes 400a, 400b, 400c, 400d of the second type.

As discussed above, each electrode 200a, 200b, 200c, 200d, 400a, 400b, 400c, 400d may be operated as one or more stimulation electrodes or operated as one or more return electrodes. This may also reduce the sensitivity to a transverse misalignment.

The stimulator 100, 101, 102, 103, 104, 105 may further comprise:
- an energy receiver, configured and arranged to wirelessly receive energy from an associated energy transmitter when the associated energy transmitter is proximate;
- the pulse generator 500 being further configured and arranged to receive electrical energy from the energy receiver for its operation.

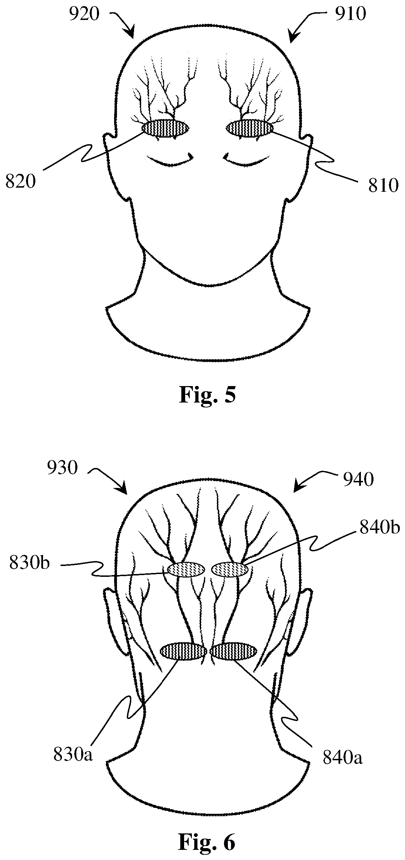

FIG. 5 and FIG. 6 depict configurations of nerves that may be stimulated using a suitably configured implantable distal end of stimulators 100, 101, 102, 103, 104, 105 to provide neurostimulation to treat conditions such as headaches or primary headaches.

FIG. 5 depicts the left supraorbital nerve 910 and right supraorbital nerve 920 which may be electrically stimulated using a suitably configured device. FIG. 6 depicts the left greater occipital nerve 930 and right greater occipital nerve 940 which may also be electrically stimulated using a suitably configured device.

Depending on the size of the region to be stimulated and the dimensions of the part of the device to be implanted, a suitable location is determined to provide the electrical stimulation required for the treatment. Approximate implant locations for the distal part of the stimulation device comprising stimulation devices 100, 101, 102, 103, 104, 105 are depicted as regions:
- location 810 for left supraorbital stimulation and location 820 for right supraorbital stimulation for treating chronic headache such as migraine and cluster.
- location 830a or 830b for left occipital stimulation and location 840a or 840b for right occipital stimulation for treating chronic headache such as migraine, cluster, and occipital neuralgia. Locations 830b, 840b for stimulation are located superior ("above") to the (external) occipital protuberance inion.

In many cases, these will be the approximate locations 810, 820, 830a/b, 840a/b for the implantable stimulator 100, 101, 102, 103, 104, 105.

For each implant location, 810, 820, 830a/b, 840a/b a separate stimulation system may be used. Where implant locations 810, 820, 830a/b, 840a/b are close together, or even overlapping, a single stimulation system may be configured to stimulate at more than one implant location 810, 820, 830a/b, 840a/b.

A plurality of stimulation devices 100, 101, 102, 103, 104, 105 may be operated separately, simultaneously, sequentially or any combination thereof to provide the required treatment.

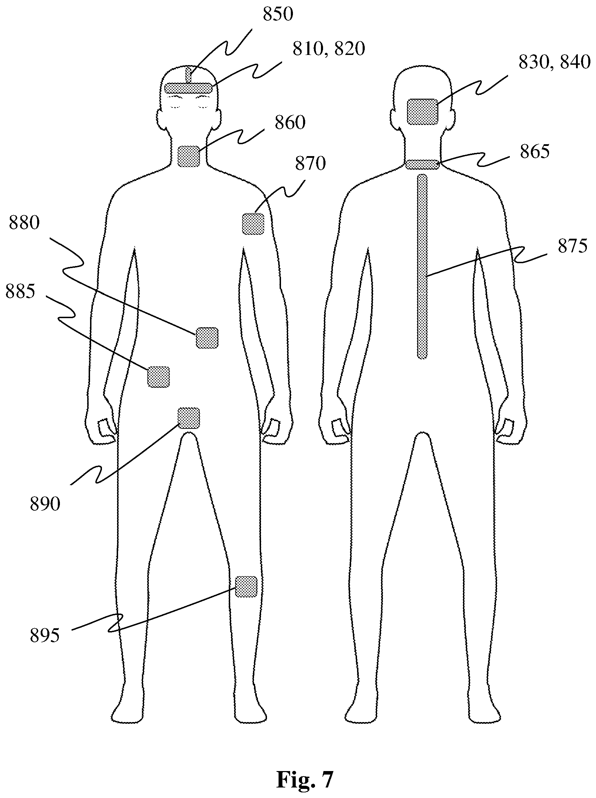

FIG 7 depict further configurations of nerves that may be stimulated using a suitably configured improved implantable stimulator 100, 101, 102, 103, 104, 105 to provide neurostimulation to treat other conditions. The locations depicted in FIG. 5 and FIG. 6 (810, 820, 830, 840) are also depicted in FIG. 7.

Depending on the size of the region to be stimulated and the dimensions of the part of the device to be implanted, a suitable location is determined to provide the electrical stimulation required for the treatment. Approximate implant locations for the part of the stimulation device comprising stimulation electrodes are depicted as regions:
- location 810 for cortical stimulation for treating epilepsy;
- location 850 for deep brain stimulation for tremor control treatment in Parkinson's disease patients; treating dystonia, obesity, essential tremor, depression, epilepsy, obsessive compulsive disorder, Alzheimer's, anxiety, bulimia, tinnitus, traumatic brain injury, Tourette's, sleep disorders, autism, bipolar; and stroke recovery
- location 860 for vagus nerve stimulation for treating epilepsy, depression, anxiety, bulimia, obesity, tinnitus, obsessive compulsive disorder, heart failure, Crohn's disease and rheumatoid arthritis;
- location 860 for carotid artery or carotid sinus stimulation for treating hypertension;
- location 860 for hypoglossal & phrenic nerve stimulation for treating sleep apnea;
- location 865 for cerebral spinal cord stimulation for treating chronic neck pain;
- location 870 for peripheral nerve stimulation for treating limb pain, migraines, extremity pain;
- location 875 for spinal cord stimulation for treating chronic lower back pain, angina, asthma, pain in general;
- location 880 for gastric stimulation for treatment of obesity, bulimia, interstitial cystitis;
- location 885 for sacral & pudendal nerve stimulation for treatment of interstitial cystitis;
- location 885 for sacral nerve stimulation for treatment of urinary incontinence, fecal incontinence;
- location 890 for sacral neuromodulation for bladder control treatment; and
- location 895 for fibular nerve stimulation for treating gait or footdrop.

Other conditions that may be treated include gastro-esophageal reflux disease, an autoimmune disorder, inflammatory bowel disease and inflammatory diseases.

The conformability and reduced thickness of the substrate 100 and electrode array 200, 400 makes one or more implantable stimulators 100, 101, 102, 103, 104, 105 highly advantageous for the stimulation of one or more nerves, one or more muscles, one or more organs, spinal cord tissue, brain tissue, one or more cortical surface regions, one or more sulci, and any combination thereof.

The implantable stimulators 100, 101, 102, 103, 104, 105 described above in relation to FIG. 1 to FIG.4 may be generally described as embodiments configured and arranged for improved conformance.

The stimulator 100, 101, 102, 103, 104, 105 may be further modified. In a non-limiting example:
- a portion of the foil-like substrate 300 and pulse generator 500 may be embedded in one or more flexible bio-compatible encapsulation layers, such as those described below. These layers may comprise: a Liquid Crystal Polymer (LCP), a Polydimethylsiloxane (PDMS), a silicone polyurethane, a Polyimide, a parylene, a biocompatible polymer, a biocompatible elastomer, and any combination thereof.

The implantable electrical devices 1100, 1101, 1102 described below in relation to FIG. 11 to FIG.12 may be generally described as embodiments configured and arranged for improved encapsulation. As described below, they may be comprised in an implantable medical device 1110, 1111 configured and arranged to provide a degree of stimulation.

FIG. 11A depicts a cross-section through an improved implantable electrical or electronic device 1100. It comprises:
- a substrate 1400 having a first surface 1410 and one or more electrical conductors 1210.

Optionally, the substrate 1400 may be substantially biocompatible - however, the use of one or more encapsulation layers 1310 may allow substrates 1400 and electrical conductors 1210 which are not biocompatible, partially biocompatible, or significantly biocompatible, to be used.

In general, the degree of biocompatibility of a material or layer may be determined by measuring the degree of tissue reaction and the length of period during which it is considered biostable. A low degree of tissue reaction and/or long period of biostability indicates a high degree of biocompatibility.

The substrate 1400 is further configured and arranged to be substantially flexible - in other words, the substrate is pliant or flexible or compliant (or conformable) to a substantial degree. The degree of flexibility may be adapted using parameters, such as:
- dimensioning of the device 1100 elements, and/or
- inclusion of materials and substances with desired properties, and/or
- combinations of materials and substances used, and/or
- percentages of materials and substances used, and/or
- inclusion of recesses, openings, apertures, reinforcement.

Additionally or alternatively, the skilled person will realize that the degree of flexibility may be adapted using parameters described above for the substrate 300 described in relation to FIG. 1 to FIG. 4.

The one or more electrical conductors 1210 are depicted very schematically - they may be conductors embedded in or deposited onto the substrate 1400 - for example, by having a single polymer layer and applying conductive material using suitable deposition techniques known from the semiconductor industry. The one or more conductors 1210, such as a metal, may be formed as required - for example, in one or more conductive elements: wire, strand, foil, lamina, plate, and/or sheet. Optionally, the one or more conductors may be positioned between the outer surfaces of the substrate 1400;

The device 1100 further comprises:
- a first biocompatible encapsulation layer 1310 comprising a polydimethylsiloxane (PDMS) rubber; and
- a first adhesion layer 1510,

Optionally, the first adhesion layer 1510 may be substantially biocompatible - however, the use of one or more encapsulation layers 1310 may allow one or more adhesion layers 1510 which are not biocompatible, partially biocompatible, or significantly biocompatible, to be used.

The first adhesion layer 1510 and the first encapsulation layer 1310 are configured and arranged to resist the ingress of fluids from a human or animal body into at least a portion of the first surface 1410. The configuration and arrangement are further described below.

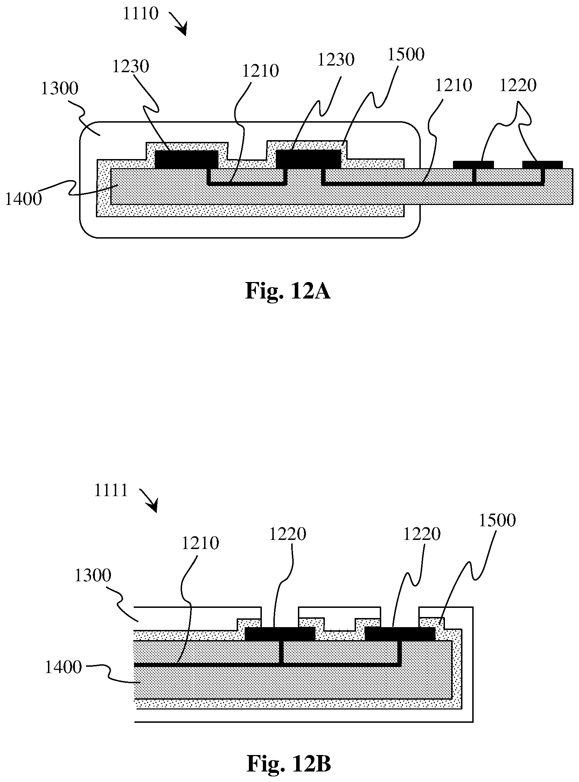

As depicted in FIG. 11A, the extent of the adhesion/encapsulation layer 1510/1310 in this cross-section may be less than the extent of the substrate 1400. In general, the extent of the adhesion/encapsulation layer 1510/1310 may be larger than, equal to or less than the extent of the substrate 1400. A "larger than" embodiment for the adhesion and encapsulation layers is depicted in FIG. 11C. Further "less than" embodiments for the adhesion and encapsulation layers are depicted in FIG. 11B and FIG. 12A.

In general, the portion of the first surface 1410 being protected against ingress of fluids is equal to or less than the extent of the adhesion/encapsulation layer 1510/1310.

As depicted in FIG. 11A, the extent of the adhesion layer 1510 in this cross-section may be less than the extent of the encapsulation layer 1310 - in some configurations, this may be advantageous as the edges of the adhesion layer 1510 are at least partially encapsulated 1310. In general, the extent of the adhesion layer 1510 may be larger than, equal to or less than the extent of the encapsulation layer 1310. Further "less than" embodiments are depicted in FIG. 11C and FIG. 12A. An "equal to" portion of a substrate is depicted in FIG. 12B.

In a preferred embodiment, the extent of the adhesion layer 1510 is equal to or larger than the extent of the encapsulation layer 1310 - this may be advantageous in certain configurations as the surface area of encapsulant 1310 in direct contact with the surface 1410 of the substrate 1400 is greatly reduced. In some cases, this surface area may be substantially zero, further reducing the possibility of fluid ingress. A "substantially zero" embodiment is depicted in FIG. 11C, and a portion of a substrate depicted in FIG. 12B.

FIG. 11B depicts another implantable electrical or electronic device 1101. It is the same as the implantable electrical device 1100 depicted in FIG. 11A, except for further comprising:
- a second surface 1420;
- a second biocompatible encapsulation layer 1320, comprising a polydimethylsiloxane (PDMS) rubber; and
- a second adhesion layer 1520, disposed between the second planar surface 1420 and the second encapsulation layer 1320. The second adhesion layer 1520 is further configured and arranged to conform to the second surface 1420 - in other words, it is a conformal layer.

The second adhesion layer 1520 and the second encapsulation layer 1320 are configured and arranged to resist the ingress of fluids from a human or animal body into at least a portion of the second surface 1420. The configuration and arrangement are further described below.

The second encapsulation layer 1320 may be substantially identical, similar to a high degree or substantially different to the first encapsulation layer 1310.

The second adhesion layer 1520 may be substantially identical, similar to a high degree or substantially different to the first adhesion layer 1510.

Although the first surface 1410 and second surface 1420 are depicted as opposite faces of a substrate in FIG. 11B, other combinations are possible, such as:
- applying the first adhesion/encapsulation layer 1310/1510 and the second adhesion/encapsulation layer 1320/1520 to different regions of the first surface 1410;
- applying the first adhesion/encapsulation layer 1310/1510 and the second adhesion/encapsulation layer 1320/1520 to different regions of the second surface 1420;
- the first surface 1410 and second surface 1420 being adjacent to each other;
- the first surface 1410 and second surface 1420 being opposite to each other;
- the first surface 1410 and second surface 1420 being at a predetermined angle to each other;
- the first surface 1410 and second surface 1420 being substantially perpendicular to each other.

FIG. 11C depicts a further implantable electrical or electronic device 1102. It is the same as the implantable electrical device 1100 depicted in FIG. 11A, except in this cross-section:
- the substrate 1400 comprises four protected surfaces, each surface being protected by a further adhesion layer 1500 and a further encapsulation layer 1300;
- the extent of the further adhesion layer 1500 is larger than the extent of substrate 1400 for each protected surface;
- the extent of the further encapsulation layer 1300 is larger than the extent of the substrate 1400 for each protected surface; and
- the extent of the further adhesion layer 1500 is less than the extent of the encapsulation layer 1300 for each protected surface.

Functionally, it may also be considered that the further encapsulation layer 1300 comprises the first 1310 and second 1320 encapsulation layers depicted in FIG. 11B.

Functionally, it may also be considered that the further adhesion layer 500 comprises the first 1510 and second 1520 adhesion layers depicted in FIG. 11B.

Functionally, it may also be considered that the substrate 1400 depicted in FIG. 11C comprises the protected portions of the first 1410 and second 1420 surfaces depicted in FIG. 11B. However, the substrate 1400 depicted in FIG. 11C, comprises two or more further protected surfaces, adjacent to such a protected first or second surface.

The further encapsulation layer 1300 of FIG. 11C may be substantially identical, similar to a high degree or substantially different to the first encapsulation layer 1310 depicted in FIG. 11A or 11B. The further encapsulation layer 1300 of FIG. 11C may be substantially identical, similar to a high degree or substantially different to the second encapsulation layer 1320 depicted in FIG. 11B.

The further adhesion layer 1500 of FIG. 11C may be substantially identical, similar to a high degree or substantially different to the first adhesion layer 1510 depicted in FIG. 11A or 11B. The further adhesion layer 1500 of FIG. 11C may be substantially identical, similar to a high degree or substantially different to the second adhesion layer 1520 depicted in FIG. 11B.

The further embodiment 1102 may be advantageous because:
- the portion of the surfaces of the substrate 1400 being protected against ingress of fluids is less than the extent of the further encapsulation layer 1300;
- the edges of the further adhesion layer 1500 are substantially encapsulated 1300; and
- the surface area of encapsulant 1300 in direct contact with a surface of the substrate 1400 is close to or substantially zero.

Experiments were performed to establish the suitability of a specific adhesion layer 1510, 1520 to provide a high degree of bonding to a PDMS.

### ALD Coating

Atomic layer deposition (ALD) is a coating process that may be used to create nm-thick conformal coatings. A suitable ALD process, for forming a monolayer comprising a first and second element, may comprise:
- loading a substrate as a sample into a reaction space;
- introducing a quantity of first molecules comprising the first element into the reaction space whereby at least a first portion of the first molecules adsorb to a surface of the substrate; and
- introducing a quantity of second molecules comprising the second element into the reaction space whereby at least a second portion of the second molecules reacts with the first portion on the surface of the substrate to form a monolayer of a compound comprising the first and second element.

PDMS encapsulation experiments:
Samples were encapsulated with a layer comprising a substantially biocompatible PDMS (MED2-6215, NuSil Carpinteria, USA) 1330.

From nusil.com/product/med-6215_optically-clear-low-consistency-silicone-elastomer:
MED-6215 is an optically clear, low consistency silicone elastomer. It is provided as two-parts which are solvent free and have a relatively low viscosity. It cures with heat via addition-cure chemistry. The mix ratio is 10:1 (Part A: Part B).

MED-6215 is considered substantially biocompatible - the manufacturer suggests that it may be used in human implantation for a period of greater than 29 days.

### Uncured:

| **Typical properties** | **Average Result** | **Standard** | **Nusil Test (NT-TM)** |
|---|---|---|---|
| Appearance | Translucent | ASTM D2090 | 002 |
| Viscosity, Part A | 5,500 cP (5,500 mPas) | ASTM D1084, D2196 | 001 |
| Viscosity, Part B | 95 cP (95 mPas) | ASTM D1084 | 001 |
| | | D2196 | |
| Work Time | 5 hours | ------ | 008 |

### Cured: 15 minutes at 150°C (302°F)

| **Typical properties** | **Average Result** | **Standard** | **Nusil Test (NT-TM)** |
|---|---|---|---|
| Specific Gravity | 1.03 | ASTM D792 | 003 |
| Durometer, Type A | 50 | ASTM D2240 | 006 |
| Tensile Strength | 1,250 psi (8.6 MPa) | ASTM D412 | 007 |
| Elongation | 100% | ASTM D412 | 007 |
| Tissue Culture (Cytotoxicity Testing) | Pass | USP ISO 10993-5 | 061 |
| Elemental Analysis of Trace Metals | Pass | ASTM E305 | 131 |

| **Property** | **Average Result** |
|---|---|
| Durometer | 50 Type A |
| Viscosity | 3,800 MPa*s (3800 cP) |
| Work Time | 5 hours |
| Tensile | 8.62 MPa (1250 psi) |
| Appearance | Transparent |
| Cure | 15 minutes / 150 °C |
| Cure System | Platinum |
| Elongation | 100 % |
| Mix Ratio | 10:1 |
| Specific Gravity | 1.03 |
| Tack Free Time | 16 hours |
| Comment | Clear, 1.41 R.I. |

The manufacturer suggests silicone primer Nu-Sil MED1-161 as a primer to further improve adhesion of MED-6215 to various substrates including: metals (such as stainless steel, steel, copper and aluminum), ceramic materials, rigid plastics, and other silicone materials.

MED-6215 is available in medical grade - in other words, substantially biocompatible and suitable for use in a medical implantable device. This is realized by ensuring all raw materials, intermediates, and finished products (for Medical Grade) are manufactured with applicable GMP and/or appropriate regulatory standards: cGMP 21 CFR § 820 (Device), cGMP 21 CFR § 210-211(Drug/API) and ISO 9001.

A dip-coating process was used for the encapsulation. The average relatively low viscosity, for example, 4000 to 7000 cP (mPas), appears to have allowed the PDMS to more easily flow over the sample. The thickness of the PDMS 1330 was estimated to be between 50 and 200 µm (micron).

The lifetime reliability of ALD coatings may depend on factors such as the conformality and adhesion of the layer, and its stability in ionic media.

PDMS-type materials are, in general, highly suitable for implantation due to their relatively high degree of biocompatibility. By appropriate selection and processing, many PDMS-type materials may be configured and arranged to be substantially biocompatible.

Polymeric materials comprised in the substrate 1400 are preferably selected for suitability to be flexible, and to comprise the one or more electrical conductors 1210. Preferably, the polymeric substrate materials have a high degree of biocompatibility and durability. Suitable polymer materials for being comprised in substrate 1400 include those mentioned above for conformable substrates in relation to FIG. 1 to FIG. 4. In particular, a polyimide, Parylene C, SU-8, an LCP, a polyurethane, or any combination thereof may be used.

Preferably, the first and/or second surface 1410, 1420 comprise a significant amount of one or more Liquid Crystal Polymers (LCP's). Optionally, the first and/or second surface 1410, 1420 may substantially consist of one or more LCP's. Optionally, the first and/or second surface 1410, 1420 may essentially consist of one or more LCP's.

The table below compares several physical and chemical properties of a typical polyimide and a typical LCP.

| | **Unit** | **LCP Flex** | **Polyimide Flex** |
|---|---|---|---|
| Thickness | Um (micron) | 25, 50, 100 | 12, 25, 50 |
| Dielectric constant (10 GHz) | -- | 2.9 | 3.2 |
| Dissipation factor (10 GHz) | -- | 0.002 | 0.002 |
| Surface resistivity | ohm | 1.0 E16 | 4.0 E13 |
| Volume resistivity | Ohm cm | 1.0 E18 | 2.6 E14 |
| Dielectric strength | kV / mil | 3.5 | 7 |
| Young's modulus | GPa | 2.3 | 7.1 |
| Tensile strength | MPa | 280 | 220 |
| CTE, x-y | ppm/K | 18 | 20 |
| CTE, z | ppm/K | 200 | 120 |
| Solder float | °C | > 288 | > 300 |
| temperature | | | |
| Melting temperature | °C | 330 | 343 |
| / glass transition | | | |
| Moisture absorption (23°C, 24h) | % | 0.04 | 1 |
| Flammability | -- | UL 94 VTM-0 | UL 94 V0 |

Advantageously, the substrate 1400, for example comprising an LCP, has a Young's modulus in the range 2500 to 3600 MPa (2.5 to 3.6 GPa).

Optionally, the substrate 1400 may further comprise one or more electrical or electronic components configured to receive energy when electrical energy is applied to the one or more electrical conductors 1210. For example, they may be inductively-coupled, capacitively-coupled or directly connected. This is particularly advantageous with substrates comprising significant amounts of one or more LCP's as PCB-techniques may be used. Preferably, a bio-compatible metal such as gold or platinum is used.

Preferably, one or more encapsulation layers 1310, 1320 and one or more adhesion layers 1510, 1520 are configured and arranged to resist the ingress of fluids to at least a portion of one or more surfaces 1410, 1420 proximate the one or more components.

For example, the one or more components may be an active component, a passive component, an electronic component, an integrated circuit (IC), an application-specific integrated circuit (ASIC), a field-programmable gate array (FPGA), an analog component, a digital component, a surface-mount device (SMD), a through-hole package, a chip carrier, a pin grid array, a fat package, a small outline package, a chip scale package, a ball grid array, a small-pin-count package, a flexible silicon device, a thin-film transistor (TFT), and any combination thereof.

The one or more electrical components may be configured and arranged to: resist, store charge, induct, sense, stimulate, amplify, process data, detect, measure, compare, switch, time, store data, count, oscillate, perform logic, add, generate stimulation pulses, and any combination thereof.

The substrates 1400 may be further configured and arranged to have a degree of conformance as described above. They may be foil-like (or film-like) and follow the contours of underlying anatomical features very closely by being flexible. Very thin foil-like substrates 1400 have the additional advantage that they have increased flexibility.

An implantable electrical device 1100, 1101 as described herein may be comprised in an implantable medical device 1110, 1111. For example, such a medical device 110, 1111 may be configured and arranged to provide a degree of sensing, stimulation, data processing, detection or measurement, data storage, oscillation, logic performance, stimulation pulses generation, or any combination thereof.

The embodiments described above in relation to FIG. 1 to FIG. 4, and in particular the implantable stimulators 101, 102, 103, 104, 105 may comprise an implantable electrical device 1100, 1101, 1102.

As depicted in FIG. 12A, an improved implantable medical device 1110 may be provided by modifying the implantable device 1100, depicted in FIG. 11A. It is the same as the implantable electrical device 1100 depicted in FIG. 11A, except in this cross-section:
- the substrate 1400 comprises three protected surfaces, each surface being protected by a further adhesion layer 1500 and a further encapsulation layer 1300. The three protected surfaces comprise two opposite protected surfaces and a further adjacent surface;
- the extent of the further adhesion layer 1500 is less than the extent of the substrate 1400 for the two opposite protected surfaces. The extent of the further adhesion layer 1500 is greater than the extent of the substrate 1400 for the third adjacent protected surface;
- the extent of the further encapsulation layer 1300 is less than the extent of the substrate 1400 for the two opposite protected surfaces. The extent of the further encapsulation layer 1300 is greater than the extent of the substrate 1400 for the third adjacent protected surface; and
- the extent of the further adhesion layer 1500 is less than the extent of the further encapsulation layer 1300 for each protected surface.

Functionally, it may also be considered that the further encapsulation layer 1300 comprises the first 1310 and second 1320 encapsulation layers depicted in FIG. 11B.

Functionally, it may also be considered that the further adhesion layer 1500 comprises the first 1510 and second 1520 adhesion layers depicted in FIG. 11B.

However, the substrate 1400 depicted in FIG. 12A, comprises a further protected surface, adjacent to such a protected first or second surface.

The further encapsulation layer 1300 of FIG. 12A may be substantially identical, similar to a high degree or substantially different to the first encapsulation layer 1310 depicted in FIG. 11A or 11B. The further encapsulation layer 1300 of FIG. 12A may be substantially identical, similar to a high degree or substantially different to the second encapsulation layer 1320 depicted in FIG. 11B.

The further adhesion layer 1500 of FIG. 12A may be substantially identical, similar to a high degree or substantially different to the first adhesion layer 1510 depicted in FIG. 11A or 11B. The further adhesion layer 1500 of FIG. 12A may be substantially identical, similar to a high degree or substantially different to the second adhesion layer 1520 depicted in FIG. 11B.

The medical device 1110 further comprises:
- one or more stimulation electrodes 1220, configured and arranged to transmit energy to human or animal tissue when electrical energy is applied to the one or more electrical conductors 1210. For example, they may be inductively-coupled, capacitively-coupled or directly connected. In the example depicted, the one or more stimulation electrodes 1220 are directly connected to the one or more electrical conductors 1210. In many neurostimulation applications, a plurality of electrodes 1220 may be required. These may be identical, similar or different to the electrodes 200, 400 described above in relation to FIG. 1 to FIG. 4.

Optionally or additionally, one or more sensors 1230 may similarly be provided - such sensors 1230 are configured to be provided electrical signals and/or data to the one or more electrical conductors 1210. For example, they may be inductively-coupled, capacitively-coupled or directly connected. If a multilayer substrate with electrical interconnections is provided, a high degree of customization is possible. For example, allowing direct measurements of parameters relevant for operation, such as humidity, temperature, electrical resistance and electrical activity.

Typically with neural-stimulation electrodes, one or more electrodes 1220 are configured and arranged to operate as a ground or return electrode - this may be one of the existing electrodes or one or more further electrodes as described above for the first 200a, 200b and second 400a, 400b electrodes described above in relation to FIG. 1 to FIG. 4.

The skilled person will realize that such a stimulation electrode 1220 and/or a tissue sensor is preferably not completely covered by an encapsulation layer 1300 and/or an adhesion layer 1500 as a sufficiently high degree of electrical connection or exposure to the implant environment are required for their function. For example, at least part of a stimulation electrode 1220 and/or tissue sensor is masked during the encapsulation process to provide a conductive surface towards tissue. Additionally or alternatively, portions of the device may not be encapsulated.

FIG. 12A depicts a device 1110 where substantially all of a stimulation electrode 1220 is substantially not covered. In addition, in this cross-section, a portion of the substrate 1400 is substantially not covered, providing a device 1110 with a substantially encapsulated portion and a substantially unencapsulated portion with one or more electrodes 1220. The extent of the further adhesion layer 1500 in this cross-section for the two opposite surfaces is less than the extent of the further encapsulation layer 1300 for these surfaces - this may be advantageous as the edges of the further adhesion layer 1500 are at least partially encapsulated 1300.

Applying this encapsulation to the implantable stimulators described above in relation to FIG. 1 and FIG. 4 generally provides a substantially unencapsulated portion with one or more electrodes 200, 400, and a substantially encapsulated portion comprising a pulse generator 500.

FIG. 12B depicts a further embodiment of a medical device 1111. More particularly, it depicts a cross-section through a portion of the substrate 1400 comprising one or more electrode 1220. The further medical device 1111 is the same as the device 1110 depicted in FIG. 12A except, in general, in this cross-section:
- the substrate 1400 comprises four protected surfaces, each surface being protected by a further adhesion layer 1500 and a further encapsulation layer 1300;
- the extent of the further adhesion layer 1500 is larger than the extent of substrate 1400 for each protected surface;
- the extent of the further encapsulation layer 1300 is larger than the extent of the substrate 1400 for each protected surface; and
- the extent of the further adhesion layer 1500 is less than the extent of the encapsulation layer 1300 for each protected surface.

In this cross-section, "a part" of the one or more stimulation electrodes 1220 is not completely covered to allow electrical connection or exposure to the implant environment after implantation. So, in the regions close to the stimulation electrodes 1220, the general statements made above do not all apply completely. In particular, in this cross-section:
- the further adhesion layer 1500 has been applied to the surface of the substrate 1400 adjacent to the stimulation electrodes 1220 and also applied to edge portions of the surface of the electrodes 1220. This may provide additional protection against ingress at any interface between the electrode 1220 and the substrate 1400; and
- the further encapsulation layer 1300 has been applied to the surface of the substrate 1400 adjacent to the stimulation electrodes 1220. However, is not significantly applied to edge portions of the surface of the electrodes 1220.

In other words, in this cross-section at the edge portions of the surface of the electrodes 1220, the extent of the further adhesion layer 1500 is approximately the same as the extent of the further encapsulation layer 1300.

This may be advantageous in certain configurations as the surface area of further encapsulation layer 1300 in direct contact with the surface of the electrodes 1220 is greatly reduced. In some cases, this surface area may be substantially zero.

Applying this encapsulation to the implantable stimulators described above in relation to FIG. 1 and FIG. 4 generally provides a substantially encapsulated portion in which "a part" of the one or more electrodes 200, 400, and a substantially encapsulated portion comprising a pulse generator 500.

Optionally, it may be advantageous if the extent of the further encapsulation layer 1300 in this cross-section at an edge portion of one or more electrodes 1220 is greater than the extent of the further adhesion layer 1500 - in some configurations, this may be advantageous as the edges of the further adhesion layer 1500 are at least partially encapsulated 1300.

So, the one or more stimulation electrodes 1220 and/or sensor are preferably comprised in a surface, configured and arranged to provide a tissue interface.

As described above, "comprised in a surface" means that the electrodes 1220 are relatively thin (for example, when the substrate conforms to a substantially planar surface, having an extent along a transverse axis. approximately perpendicular to a longitudinal axis of the substrate, of 20 to 50 microns or less. Thinner electrodes may also be used to further increase the degree of conformability, for example 1 micron or less), and attached to (or at least partially embedded in) the surface.

This is particularly advantageous with substrates comprising significant amounts of one or more LCP's as PCB/metallization-techniques may be used to provide conductive regions, which may be configured and arranged to be electrodes 1220 and/or sensors 1230. As described above, a conductive material is preferably used such as gold, platinum, platinum black, TiN, IrO₂, iridium, and/or platinum/iridium alloys and/or oxides. Conductive polymers, such as Pedot, may also be used. Preferably, bio-compatible conductive materials are used.

As described above, thicker metal layers are generally preferred over thinner metal layers for electrodes 1220 because they can be subjected to bodily substances that may dissolve the metal. However, thicker metal layers typically increase rigidity (reduce conformability) proximate the thicker layer.

In a further set of experiments, adhesion of PDMS MED2-4213 from NuSil to an LCP substrate was investigated using two different substrates and two different PDMS casting processes.

Different methods were used to evaluate the adhesion: adhesion evaluation by Peel-test dry, after PBS soaking at 60 degr.C, and a Peel-test based on ASTM D1876.

From nusil.com/product/med2-4213_fast-cure-silicone-adhesive:
MED2-4213 is a two-part, translucent, thixotropic, a relatively high extrusion rate, a relatively high tear strength, a relatively fast-cure silicone adhesive. It is also substantially free of tin (Sn), reducing the requirement for atmospheric moisture to cure. It also does not comprise significant amounts of curing byproducts, such as acetic acid or methyl alcohol.

MED2-4213 is considered substantially biocompatible - the manufacturer suggests that it may be used in human implantation for a period of greater than 29 days. Typical chemical and physical properties include:

### Uncured:

| **Typical properties** | **Result** | **Standard** | **Nusil Test Method** |
|---|---|---|---|
| Appearance | Translucent | ASTM D2090 | 002 |
| Viscosity, Part A | 80000 cP (80000 mPas) | ASTM D1084, D2196 | 001 |
| Work Time | 15 hours minimum | ------ | 008 |

### Cured: 15 minutes at 150°C

| **Typical properties** | **Result** | **Standard** | **Nusil Test Method** |
|---|---|---|---|
| Specific Gravity | 1.12 | ASTM D792 | 003 |
| Durometer, Type A | 15 | ASTM D2240 | 006 |
| Tensile Strength | 1,000 psi (6.9 MPa) | ASTM D412 | 007 |
| Elongation | 800% | ASTM D412 | 007 |
| Tear Strength | 130ppi (23.0 kN/m) | ASTM D624 | 009 |

| **Property** | **Average Result** |
|---|---|
| Durometer | 15 Type A |
| Viscosity | 80,000 MPa*s (80000 cP) |
| Work Time | 15 hours |
| Tensile | 6.9 MPa (1000 psi) |
| Appearance | Translucent |
| Cure | 15 minutes / 150 °C |
| Cure System | Platinum |
| Elongation | 800 % |
| Mix Ratio | 1:1 |
| Specific Gravity | 1.12 |
| Rheology | Thixotropic / non-slump |
| Tear | 22.93 kN/m (130 ppi) |

It may be advantageous if the first (1310) and/or second (1320) encapsulation layers have/has a tensile strength in the range 6 to 8 MPa.

NuSil suggests that in many bonding applications (for a substrate comprising Aluminum, Glass, PMMA, Silicone) the use of a silicone primer to improve suitable adhesion is not required.

Use of a primer is suggested by the manufacturer when adhering to substrates comprising Polyetherimide, PEEK, Plastic, Polycarbonate, Polyimide, Polysulphone, Polyurethane, and Stainless steel.

In order to study the adhesion properties of the PDMS on LCP with different processing methods and adhesion layers, two different test substrates were used:
- TYPE 1: an LCP 3-layered substrate with ALD coating on one side
- TYPE 2: an LCP 2-layered laminated substrate with ALD coating on one side.

In general, it is advantageous to perform as few steps as possible when manufacturing an implantable electrical device - this may reduce the risk of introducing contamination or transport related issues, and it may reduce one or more costs.

A process with relatively few steps may be based around overmoulding electronics that are directly mounted on a substrate (here LCP). Depending on the hardware configuration, the PDMS used may need to adhere sufficiently well to surfaces such as:
- an ASIC passivation layer in case of wire-bonding
- a Si-substrate in case of ASIC flip-chip or ACF mounting. Si-substrate is one of the relevant interfaces when using bare-die components. Bare die integrated circuits are often made from a wafer or substrate, that is a thin slice of crystalline silicon semiconductor. To make a bare-die component, this material undergoes many microfabrication processes to become an integrated circuit, but one side will always be the raw material that was used, most often crystalline silicon. Relevant interfaces include:

- an ASIC interconnect
- Gold from wire-bonds or stud bumps
- an ACF (Anisotropic conductive film), which is frequently epoxy based, with coated gold particles, for application of a bare-die component on bond-pads.
- the substrate - in this case, a substrate comprising significant amounts of LCP.

TYPE 1 LCP substrates were prepared using one or more of the following process steps:
a) Providing a substrate: these substrates were substantially planar sheets of LCP with an average thickness of approximately 0.150 mm. The substrate comprised three layers; two 0.050mm layers of ULTRALAM^{®} 3908, separated by one 0.050mm layer of ULTRALAM 3850.

ULTRALAM^{®} 3908 LCP is available from Rogers Corporation (www.rogerscorp.com) and may be used as a bonding medium (adhesive layer) between copper, other LCP materials and/or dielectric materials. It is characterized by low and stable dielectric constant. It has a relatively low modulus, allowing relatively easy bending for flex applications, and relatively low moisture absorption.

It may be used with one or more layers of ULTRALAM^{®} 3850 LCP to create substantially adhesive-less substantially all-LCP multi-layer substrates.

Typical values for physical and chemical properties of ULTRALAM^{®} 3908 LCP include:

### Mechanical Properties

| | **Typical value** | **Units** | **Test Methods** |
|---|---|---|---|
| Dimensional Stability | MD: <0.1 | % | IPC 2.2.4 method A |
| | CMD: <0.1 | | |
| Initiation Tear Strength, min | 1.4 (3.1) | Kg (lbs) | IPC 2.4.16 |
| Tensile Strength | 216 (31) | MPa (Kpsi) | IPC 2.4.19 |
| Tensile Modulus | 2450 (355) | MPa (Kpsi) | IPC 2.4.19 |
| Thickness Variation | < +/-10 | % | ASTM-D374 |

### Thermal Properties

| | **Typical value** | **Units** | **Test Methods** |
|---|---|---|---|
| Coefficient of Thermal | X:17 | ppm/°C | IPC 2.4.41.3 |
| Expansion, CTE | Y:17 | | |
| (30°C to 150°C) | Z:150 | | |
| Solder Float, Method B (288°C) | PASS | | IPC 2.4.13 |
| Thermal Conductivity @ 50°C | 0.20 | W/m/°K | ASTM D5470 |
| Melting Temperature | 280 | °C | DSC |
| Relative Thermal Index (RTI) mechanical | 190 | °C | |
| electrical | 240 | °C | |

### Electrical Properties

| | **Typical value** | **Units** | **Test Methods** |
|---|---|---|---|
| Dielectric Constant (10 GHz, 23°C) | 2.9 | | IPC 2.5.5.5.1 |
| Dissipation Factor (10 GHz, 23°C) | 0.0025 | | IPC 2.5.5.5.1 |
| Surface Resistivity | 1.2 X 10¹² | Mega Ohms | IPC 2.5.17 |
| Volume Resistivity | 2.6 X 10¹⁴ | Mega Ohms-cm | IPC 2.5.17 |
| Dielectric Breakdown Strength | 118 (3000) | KV/cm (V/mil) | ASTM-D-149 |

### Environmental Properties

| | **Typical value** | **Units** | **Test Methods** |
|---|---|---|---|
| Chemical Resistance | 98.7 | % | IPC 2.3.4.2 |
| Water Absorption (23°C, 24 hrs) | 0.04 | % | IPC 2.6.2 |
| Coefficient of | 4 | ppm/%RH | 60°C |
| Hydroscopic Expansion, CHE (60°C) | | | |
| Flammability | VTM-O | | UL-94 |

ULTRALAM^{®} 3850 is available from Rogers Corporation (www.rogerscorp.com) and is a relatively high-temperature resistant LCP. It may be provided as a double copper clad laminate for use as laminate circuit materials. The manufacturer suggests these products for use as a single layer or a multilayer substrate. ULTRALAM 3850 circuit materials are characterized by a relatively low and stable dielectric constant, and dielectric loss. It has a relatively low modulus, allowing relatively easy bending for flex applications, and relatively low moisture absorption.

It may be used with one or more layers of ULTRALAM^{®} 3908 LCP to create substantially adhesive-less substantially all-LCP multi-layer substrates.

Typical values for physical and chemical properties of ULTRALAM^{®} 3850 LCP include:

### Mechanical Properties

| | **Typical value** | **Units** | **Test Methods** |
|---|---|---|---|
| Dimensional Stability | MD: -0.06 | % | IPC 2.2.4 method B |
| | CMD: -0.03 | | |
| Peel Strength | 0.95 (8.52) | N/mm (lbs/in) | IPC 2.4.8 (1/2 oz. ED foil) |
| Initiation Tear Strength, min | 1.4 (3.1) | Kg (lbs) | IPC 2.4.16 |
| Tensile Strength | 200 (29) | MPa (Kpsi) | IPC 2.4.16 |
| Tensile Modulus | 2255 (327) | MPa (Kpsi) | IPC 2.4.19 |
| Density | 1.4 | gm/cm3, Typical | |

### Thermal Properties

| | **Typical value** | **Units** | **Test Methods** |
|---|---|---|---|
| Coefficient of Thermal | X:17 | ppm/°C | IPC 2.4.41.3 |
| Expansion, CTE | Y:17 | | |
| (30°C to 150°C) | Z:150 | | |
| Solder Float, Method B (288°C) | PASS | | IPC 2.4.13 |
| Melting Temperature | 315 | °C (Typical) | DSC |
| Relative Thermal Index (RTI) mechanical | 190 | °C | |
| electrical | 240 | °C | |
| Thermal Conductivity @ 50°C | 0.2 | W/m/°K | ASTM C518 |
| Thermal Coefficient of εr, -50°C to 150°C | (+)24 | ppm/°C | IPC 2.5.5.5, 8 GHz |

### Electrical Properties

| | **Typical value** | **Units** | **Test Methods** |
|---|---|---|---|
| Dielectric Constant, 10 GHz, 23°C (Process) | 2.9 | | IPC 2.5.5.5.1 |
| Dielectric Constant, 10 GHz, 23°C (Design) | 3.14 | | Differential Phase Length Method |
| Dissipation Factor (10 GHz, 23°C) | 0.0025 | | IPC 2.5.5.5.1 |
| Surface Resistivity | 1X10¹⁰ | Mega Ohms | IPC 2.5.17 |
| Volume Resistivity | 1X10¹² | Mega Ohms-cm | IPC 2.5.17 |
| Dielectric Breakdown Strength | 1378 (3500) | KV/cm (V/mil) | ASTM-D-149 |

### Environmental Properties

| | **Typical value** | **Units** | **Test Methods** |
|---|---|---|---|
| Chemical Resistance | 98.7 | % | IPC 2.3.4.2 |
| Water Absorption (23°C, 24 hrs) | 0.04 | % | IPC 2.6.2 |
| Coefficient of Hydroscopic Expansion, | 4 | ppm/%RH | 60°C |
| CHE (60°C) | | | |
| Flammability | VTM-0 | UL-94 | |

TYPE 1 LCP substrates were further prepared using one or more of the following process steps:
b1) an optional pre-cleaning of at least a portion of the substrate using IPA, followed by drying. Another suitable alcohol may also be used.
b2) Applying an adhesion coating: using ALD, a coating was applied to an outer surface of the substrate - in this case a surface comprising ULTRALAM^{®} 3908 LCP. The extent of the ALD coating was approximately the same as the extent of the substrate. It was applied at a temperature substantially lower than the melting temperature of the LCP. For these TYPE 1 LCP substrates, it was applied at approximately 125 degr C after an optional stabilization time of approximately 90 minutes.

For comparison, this step was omitted for some of the samples (in other words, the PDMS was applied directly to the LCP).

c) Cleaning at least a portion of the adhesion coating: as preparation for the PDMS coating, an optional ten-minute ozone (O3) plasma treatment was performed to clean the ALD surface. The PDMS was applied within fifteen minutes from the ozone cleaning. For comparison, some samples were not cleaned before the PDMS coating was applied.

UV O3 (ozone) plasma cleaning is suitable for dry, non-destructive atomic cleaning and removal of organic contaminants. It uses intense 185 nm and 254 nm ultraviolet light. In the presence of oxygen, the 185 line produces Ozone and while the 254 line excites organic molecules on the surface. This combination drives the rapid destruction and decimation of organic contaminants.

d) Applying an encapsulation coating: a PDMS coating of approximately 500 um to 1000 um of MED2-4213 was applied on top of the ALD coating. A syringe was filled with MED2-4213, and mixed & degassed at relatively high speed (2500 rpm) for three minutes. It was cured at 150 degr C for 10min and post-cured at 80 degr C for 24 hours. The extent of the PDMS coating was less than the extent of the ALD coating, whereby the ALD coating was exposed (not covered by encapsulant) close to the edge of the substrate. After applying the PDMS on the substrate, the substrate was placed on the PTFE (polytetrafluorethylene)-coated pre-heated plate, and a weight was pressed on top of it.

So, six samples of TYPE 1 LCP were prepared:

| | **Nr of samples** | **ALD coating** | **Cleaning** | **PDMS coating** |
|---|---|---|---|---|
| 1.1 | Two | None | None | MED2-4213 |
| 1.2 | Two | Ten x (5 nm A1203 + 5nm HfO2) | None | MED2-4213 |
| 1.3 | Two | Ten x (5 nm Al2O3 + 5nm HfO2) | O3-plasma (10 min) | MED2-4213 |

A Pass/ Fail test was defined for the TYPE 1 LCP substrates by hand:
- after curing the PDMS, a dry Peel-test was performed (no soaking) and the degree of delamination was noted
- after the dry Peel-test, the samples were soaked in a PBS solution at 60 degr C for 1 day, 1 week, and 4 weeks, and the Peel-test was repeated to determine a second degree of delamination.

Three degrees of delamination were defined:
- Delamination: the PDMS can be removed relatively easily from the substrate
- Partial delamination: the PDMS can be removed relatively easily in some areas, but sticks relatively well in other areas
- Adhesion: the PDMS does not substantially delaminate

Phosphate-buffered saline (abbreviated PBS) is a buffer solution commonly used in biological research. It is a water-based salt solution containing disodium hydrogen phosphate, sodium chloride and, in some formulations, potassium chloride and potassium dihydrogen phosphate. The buffer helps to maintain a constant pH. The osmolarity and ion concentrations of the solutions are selected to match those of the human body (isotonic).

| **Samples** | **Dry** | **Soak after 24h @60°C PBS** | **Soak after 1 week @60°C PBS** | **Soak after 4 weeks @60°C PBS** |
|---|---|---|---|---|
| 1.1 | Delamination | Not applicable | Not applicable | Not applicable |
| 1.2 | Adhesion | Partial Delamination | Partial Delamination | Partial Delamination |
| 1.3 | Adhesion | Adhesion | Adhesion | Adhesion |

| | | | | |
|---|---|---|---|---|
| Samples 1.1: in general, PDMS has a low degree of adhesion to LCP Samples 1.2: the PDMS could not be peeled from the surface in dry state. After twenty-four hours of soaking, part of the PDMS could be peeled from the substrate, although no moisture filled voids were observed. After peeling away some of the PDMS, the rest stuck so well to the substrate it could not be peeled off any further, not even after 1 or 2 weeks of additional soaking. It was suspected that the initial delamination was due to local contamination during PDMS processing or processing issues. Samples 1.3: these samples showed good adhesion. No delamination was achieved in dry and wet conditions until after two weeks of testing. | | | | |

### Conclusions:

- In general, PDMS has a low degree of adhesion to LCP without any adhesion layer (samples 1.1).
- In samples 1.2 (coated with ALD and then encapsulated with PDMS without 03 cleaning), the PDMS could not be peeled from the surface in dry state. After 24 hours of soaking, part of the PDMS could be peeled from the substrate, although no moisture filled voids were observed. After peeling away some of the PDMS, the rest stuck so well to the substrate it could not be peeled off any further, not even after two weeks of additional soaking. Samples with a more conformal ALD layer showed good adhesion even after two weeks of soaking.
- Samples 1.3 (coated with ALD and encapsulated with PDMS after an O3 cleaning step) showed the highest degree of adhesion. No substantial delamination was achieved in dry or wet conditions (after 2 weeks of soaking)

TYPE 2 LCP laminated substrates were prepared using one or more of the following process steps:
a) Providing a substrate: these substrates were laminated sheets of LCP, with an average thickness of approximately 0.110 mm. The substrate comprised four layers; one outer layer of copper connection pads, one 0.050 mm layer of ULTRALAM^{®} 3850, one inner layer of one or more copper conductors, and one 0.025mm layer of ULTRALAM 3908:
   a1) an approximately 50um-thick sheet of LCP ULTRALAM^{®} 3850, clad on a first surface with a first copper layer. This first copper layer was approximately 18um-thick. The first copper layer was configured and arranged to form copper connection pads, for example by masking and etching, which may be considered to be comprised in an outer surface of the laminated substrate;
   a2) the ULTRALAM^{®} 3850 was further clad with a further copper layer. This second layer was approximately 18um-thick. Optionally, it may be configured and arranged to form one or more conductors, for example by masking and etching, which may be considered to be comprised in an inner surface of the laminated substrate. If no inner conductors are required, the further copper layer may be omitted or completely removed;
   a3) an approximately 25um-thick sheet of LCP ULTRALAM^{®} 3908, bonded to the inner surface of the ULTRALAM^{®} 3850 layer, and further bonded to the one or more conductors.

Optionally, the laminated sheets may be substantially planar.
b) Applying an adhesion coating:
   b1) an optional pre-cleaning of at least a portion of the substrate using IPA, followed by drying. Another suitable alcohol may also be used.
   b2) using ALD, a coating was applied to an outer surface of the substrate - in this case a surface comprising ULTRALAM^{®} 3908 LCP. It was not the outer surface of the substrate comprising one or more connection pads. b3) Applying an adhesion improver: MED-166 from NuSil is a specially formulated primer which is suggested by the manufacturer to improve adhesion of PDMS to various substrates including: rigid plastics, and other silicone materials. The manufacturer suggests that it is suitable for use in human implantation for a period of greater than 29 days.
c) Cleaning at least a portion of the adhesion coating before encapsulation:
   c1) Option 1: cleaning using ethanol, followed by drying for 4 hours at 70°C. Another suitable alcohol may also be used.
   c2) Option 2: exposing the ALD surface to a plasma comprising O2.

O2 (oxygen) plasma refers to any plasma treatment performed while actively introducing oxygen gas to the plasma chamber. Oxygen plasma is created by utilizing an oxygen source on a plasma system.

Additionally or alternatively, ozone (O3) may be used.
d) Applying an encapsulation coating:
d1) Applying an encapsulation mask to simplify testing: a strip of Kapton tape (10mm wide) was applied to one edge to mask a small section to which the pull-tester is to be clamped during the Peel-test.
d2) Applying an encapsulation coating: a PDMS coating of approximately 500 um to 1000 um of MED2-4213 was applied on top of the ALD coating. Vacuum centrifugal casting at 100 degr. C used with PTFE-coated molds under a relatively low vacuum, for example 800 - 900 Pa (8 to 9 mbar) -vacuum centrifugal casting was used to reduce the risk of air inclusion in the PDMS. In general, applying a vacuum may be advantageous in improving the application to an adhesion coating of the encapsulation of a PDMS having an average viscosity in the range 55000 to 100000 cP (mPas) for a significant time period.

The extent of the PDMS coating was approximately the same as the extent of the ALD coating. After removing the Kapton tape, a strip of approximately 10mm wide was provided where the PDMS was not attached to the ALD coating.

e) Performing further processing: the coated substrate of approximately 100x75mm area was cut into 7 pieces of approximately 100x10mm for Peel-testing. Each piece had an area of approximately 10x10mm without the PDMS coating at is edge due to Kapton tape removal.

So, fifteen samples of type (2) were prepared:

| | **Nr of samples** | **ALD coating** | **Primer** | **Cleaning** | **PDMS coating** |
|---|---|---|---|---|---|
| 2.1 | Three | None | | Ethanol | MED2-4213 |
| 2.2 | Three | None | MED-166 | Ethanol | MED2-4213 |
| 2.3 | Three | Yes | None | Ethanol | MED2-4213 |
| 2.4 | Three | None | None | O2 plasma treatment | MED2-4213 |
| 2.5 | Three | Yes | None | O2 plasma treatment | MED2-4213 |

Peel-test according to ASTM D1876 was adapted for testing the TYPE 2 LCP or laminated substrates). A Peel-tester was used to measure the lamination force.

### Peel-test results

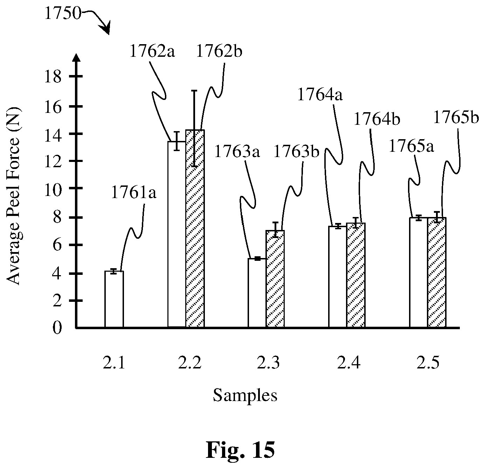

FIG. 15 depicts a graph 1750 comparing the average pull force under dry (not soaked) conditions with the average pull forces after 24 hours of soaking at 60 degr. C in PBS. The samples of LCP were coated with PDMS using different processes.

Average peel force is plotted along the vertical (Y) axis from 0 to 18 N, and the results are indicated for the different samples along the horizontal (X) axis. To simplify interpretation, the order of the samples chosen is numerical: from left to right, samples 2.2, 2.2, 2.3, 2.4 and 2.5.

For each sample, the vertical length of each bar indicates the average peel force in Newtons (N). For each bar, an "I" shaped line is also depicted to indicate the variation measured in the pull force values used to determine the average. For each sample, an unfilled bar is depicted on the left-hand side showing the average pull force under dry conditions, and a hatched bar on the right-hand side showing the average pull force after 24 hours of soaking at 60 degr. C in PBS.

For sample 2.1, an unfilled bar 1761a is depicted of approx. 4N, with a relatively small degree of variation. No value after soaking is depicted.

For sample 2.2, an unfilled bar 1762a is depicted of approx. 13N, with an average degree of variation. A hatched bar 1762b is depicted of approx. 14N, with a relatively high degree of variation.

For sample 2.3, an unfilled bar 1763a is depicted of approx. 5N, with a relatively small degree of variation. A hatched bar 1763b is depicted of approx. 7N, with an average degree of variation.

For sample 2.4, an unfilled bar 1764a is depicted of approx. 7N, with a relatively small degree of variation. A hatched bar 1764b is depicted of approx. 7.5N, with an average degree of variation.

For sample 2.5, an unfilled bar 1765a is depicted of approx. 8N, with a relatively small degree of variation. A hatched bar 1765b is depicted of approx. 8N, with an average degree of variation.

The average peel forces measured were:

| **Samples** | **Average Peel Force (N) - Dry** | **Average Peel Force (N) - After 24 hours soaking in PBS at 60 degr. C** |
|---|---|---|
| 2.1 | 4.05 (761a) | Not applicable |
| 2.2 | 13.38 (762a) | 14.22 (762b) |
| 2.3 | 5.23 (763a) | 7.29 (763b) |
| 2.4 | 7.31 (764a) | 7.56 (764b) |
| 2.5 | 8.31 (765a) | 8.32 (765b) |

It appears that a stable over molding encapsulation process was achieved, showing substantially none, or very few, air bubbles in the PDMS. Substantial delamination of the LCP/PDMS interface was observed on 3 out of 7 samples directly after over molding. For this reason, the Peel-test was applied to get a more qualitative measure of the adhesion strength.

Samples 2.1: without additional priming or cleaning, the PDMS had a very low degree (approx. 4N - 1761a) of adhesion to LCP.

Samples 2.2: substrates with a primer appeared to have a relatively high degree of adhesion (approx. 13N - 1762a - compared to approx. 4N - 1761a). During the test, some regions had a higher degree of adhesion, which resulted in the PDMS rupturing before peeling the samples completely. The average pull force after the soaking test appeared to be higher at approx. 14N - 1762b, but a relatively high degree of deviation was also observed.

Samples 2.3: by adding an ALD layer, the degree of dry adhesion appeared improved (from approx. 4N - 1761a - to approx. 5N - 1763a). The results under dry conditions - 1763a - appears to have a very low degree of deviation. The average pull force after the soaking test appeared to be higher at approx. 7N - 1763b.

Samples 2.4: O2-plasma activation also appeared to increase the adhesion (approx. 7N - 1764a - compared to approx. 4N - 1761a). . The average pull force after the soaking test appeared to be slightly higher at approx. 7.5N - 1764b.

Samples 2.5: plasma activation appeared to further improve the degree of adhesion (approx. 8N - 1765a - compared to approx. 4N - 1761a). The average pull force after the soaking test appeared to be approximately the same at 8N - 1765b. A small increase in deviation - 1765b - was observed after soaking.

### Conclusions:

A relatively high degree of adhesion was observed using a primer - for example, MED-166 from NuSil may be used. But it may be less-preferred in some uses. In particular, for implantable devices, it is advantageous to use materials that are significantly biocompatible and more preferably materials that are substantially biocompatible (have a high degree of biocompatibility).

In addition, for implantable devices, a high degree of quality control is often required to limit the risk of defects.

The skilled person will also realize that adhesion may be improved by optionally or additionally applying a conformal coating to such a substrate, for example with an ALD process, applying a layer of SiO2 (silicon dioxide).

PDMS is, in general, a silicone rubber, with siloxane as the basic repeating unit. Methyl groups are substituted by a variety of other groups, for example, phenyl, vinyl or trifluoropropyl groups, depending on the type of PDMS, enabling the linkage of organic groups to an inorganic backbone.

An adhesion layer may be a bilayer or multilayer, in which one or more layers may be configured and arranged for a relatively high degree of adhesion, and one or more layers may be configured and arranged for a relatively high degree of corrosion resistance (impermeability).

FIG. 5 and FIG. 6 also depict examples of nerves that may be stimulated using one or more suitably configured improved medical devices 1110, 1111, configured to provide neurostimulation to treat, for example, headaches, chronic headaches or primary headaches. In particular, if the substrate is substantially flexible (or conformable), it may conform better to the curved surfaces of the head and/or skull. This means that the comfort to the user of an implantable medical device 1110, 1111 may be increased by applying one or more of the features described above for improving conformance.

In many cases, these will be the approximate locations 810, 820, 830, 840 for the one or more implantable medical devices 110, 111.

For each implant location, 810, 820, 830a/b, 840a/b a separate stimulation device 110, 111 may be used. Where implant locations 810, 820, 830a/b, 840a/b are close together, or even overlapping a single stimulation device 110, 111 may be configured to stimulate at more than one implant location 810, 820, 830a/b, 840a/b.

A plurality of implantable medical devices 110, 111 may be operated separately, simultaneously, sequentially or any combination thereof to provide the required treatment.

FIG. 7 depicts further examples of nerves that may be stimulated using one or more suitably configured improved implantable medical devices 110, 111 to provide neurostimulation to treat other conditions.

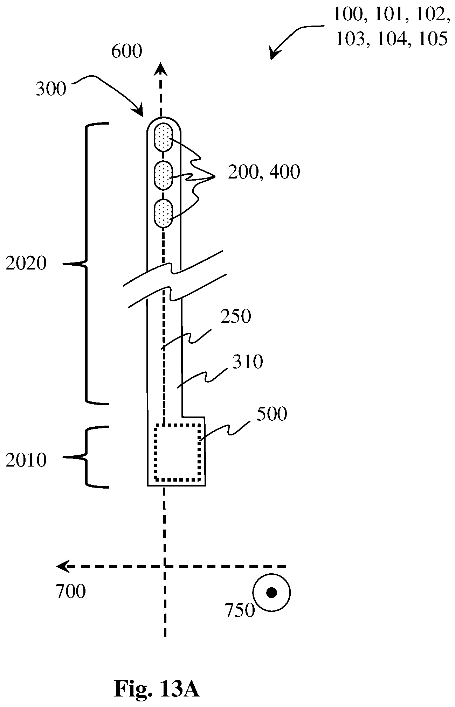

In an embodiment, FIG. 13A depicts a further bottom view of an implantable distal end (or portion) of the foil-like substrate 300 described elsewhere in this disclosure and comprised in a stimulator 100, 101, 102, 103, 104, 105. The first surface 310 of the substrate is depicted as lying in a plane through the longitudinal axis 600 and the first transverse axis 700.

The pulse generator is located along a first portion 2010 of the substrate, the pulse generator comprising one or more electrical or electronic components, configured and arranged to provide, in use, electrical energy to the one or more electrodes 200, 400 as one or more stimulation pulses.

A conformable second portion 2020 of the foil-like substrate 300 is depicted with a longitudinal axis 600 extending from a pulse generator 500 to a distal end 2020 of the substrate 300. The conformable second portion 2020 comprises at least two electrodes 200, 400 - in this example, three oval-shaped electrodes 200, 400 in a 1x3 array are depicted. However, any required electrode type and configuration may be used, including those described elsewhere in this disclosure.

In general, it is the conformability and reduced thickness of the substrate and electrodes 200, 400 that makes implantable stimulators according to this disclosure highly advantageous.

As depicted, the conformable second portion 2020 of the foil-like substrate 300 is preferably elongated along the longitudinal axis 600, having a tape-like shape, allowing the pulse generator 500 to be disposed (or located) further away from the position of the electrodes 200, 400.

The pulse generator 500 along the first portion 2010 is depicted with dotted lines because it may be at least partially embedded in the surfaces of the substrate 300.

One or more electrical interconnections 250 are depicted between the pulse generator 500 and the electrodes 200, for transferring electrical energy as one or more electrical treatment stimulation pulses. Interconnections 250 are indicated as a dotted line because they are not configured or arranged to be, in use, in contact with human or animal tissue - they are embedded (or covered) in one or more layers of a low conductance or insulating polymer, such as LCP.

Additionally or alternatively, one or more encapsulation layers may be used.

The degree of conformability of the substrate 300 may be determined by relevant parameters and properties of the different portions, for example: the transverse 700 and/or longitudinal extent 600 of the one or more electrodes 200, 400; the thickness of the foil-like substrate 300, and the perpendicular distance between the first and the second surfaces (not depicted in FIG. 13A); the materials comprised in the foil-like substrate 300, and their physical properties; the number and extent of interconnections 250 and/or interconnection layers 250.

In the embodiment depicted in FIG. 13A, the first portion 2010 is less conformable than the conformable second portion 2020 due to the presence of the pulse generator 500. Although the maximum thickness of the pulse generator 500 may be optimized, for example, by thinning any integrated circuits, the electrodes 200, 400 and the interconnections 250 are expected to be much thinner in practice. If the pulse generator 500 comprises additional electrical or electronic components, these may further influence the thickness and conformability of the first portion 2010, depending on the degree that they are embedded in the substrate.

The implantable stimulator 100, 101, 102, 103, 104, 105 further comprises a conformability changeover region at the meeting of the first portion 2010 and the conformable second portion 2020. The conformability changeover region typically encompasses at least part of the first portion 2010 and at least part of the second portion 2020. The position and extent of the conformability changeover region is affected by differences in relevant parameters and properties between the less conformable first portion 2010 and the conformable second portion 2020. For example, if the difference in conformability between the first portion and the second portion is greater, a longer conformability changeover region may be desirable to minimize the chances of separation. A part of the implantable stimulator 100, 101, 102, 103, 104, 105 within the conformability changeover region will preferably be less conformable than the part of the implantable stimulator which is in the area of the conformable second portion that is not within the conformability changeover region. Thus, the conformability changeover region provides a portion of the implantable stimulator with an intermediate conformability between that of the implantable stimulator in the first portion and that of the implantable stimulator in the area of the conformable second portion that is not within the conformability changeover region.

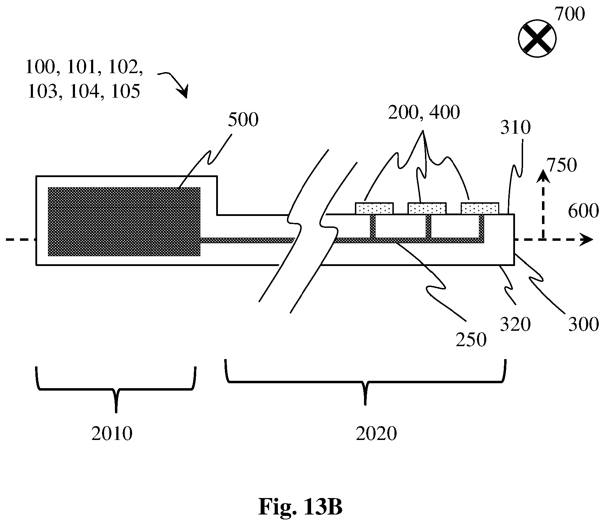

FIG. 13B depicts a transverse view of a longitudinal cross-section of the stimulator 100, 101, 102, 103, 104, 105 depicted in FIG. 13A. It depicts the pulse generator 500 along the first portion 2010. It further depicts the conformable second portion 2020 of the foil-like substrate 300 comprising the at least two electrodes 200, 400. As depicted, the substrate 300 has a first 310 and a second 320 planar (outer) surface. The longitudinal cross-section depicted passes through the one or more interconnections 250, and is depicted as lying in a plane through the longitudinal axis 600 and the second transverse axis 750.

As depicted, the pulse generator 500 is fully embedded or encapsulated. Optionally, it may be partially embedded or encapsulated.

The one or more interconnections 250 are depicted schematically as a single connection between the at least two electrodes 200, 400 and the pulse generator 500.

As depicted, the average thickness (or extent along the second transverse axis 750) of the pulse generator 500 is greater than the average thickness of the electrodes 200, 400 and/or the average thickness of the interconnections 250. The first portion 2010 is therefore less conformable than conformable second portion 2020. In some cases, the first portion 2010 may be considered rigid, inflexible or non-conformable when compared to the conformable second portion 2020. The degree of conformability of the first portion 2010 may be further reduced if, for example:
- one or more of the pulse generator 500 components are mounted to a circuit board, PCB and/or ceramic material;
- one or more of the pulse generator 500 components are mounted to a glass-reinforced epoxy laminate, such as G-10, G-11, FR-4, FR-5 and/or FR-6;
- one or more of the pulse generator components 500 are comprised in an enclosure, such as a metal can, titanium can, integrated circuit, or chip package;
or any combination thereof.

The third portion 2030 conformability changeover region is located at the meeting of the conformable second portion 2020 and the first portion 2010. As differences in conformability between the conformable second portion 2020 and the first portion 2010 increase, the risk of separation and/or damage to one or more surfaces may also increase. This may further increase the risk of moisture ingress, and may also reduce reliability.

As will be described below, at least one mechanical brace (or mechanical strain relief) may be provided and configured to resist separation of the conformable second portion 2020 from the first portion 2010. This is advantageous as it may reduce the risk of moisture ingress and also reduce the risk of damage to one or more interconnections 250 passing from the conformable second portion 2020 to the first portion 2010.

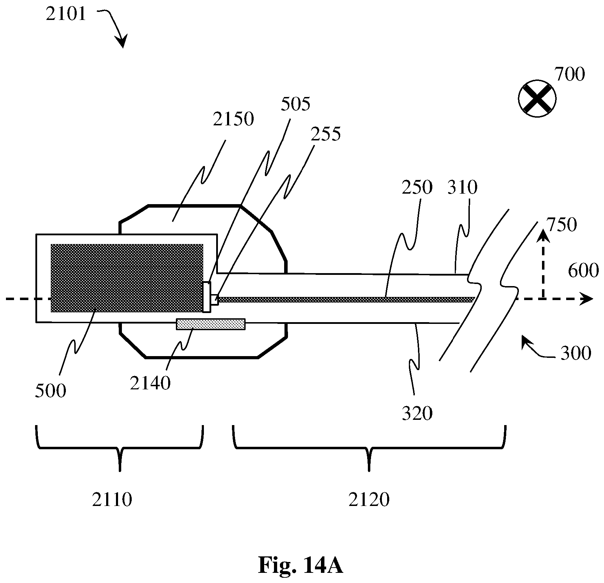

FIG. 14A depicts a depicts a transverse view of a longitudinal cross-section of a first embodiment 2101 of an implantable stimulator comprising a mechanical strain relief. For clarity, the electrode portion is not depicted. The longitudinal cross-section depicted is through the one or more interconnections 250, and is depicted as lying in a plane through the longitudinal axis 600 and the second transverse axis 750.

The implantable stimulator 2101 in FIG. 14A is the same as the implantable stimulator depicted in FIG. 13B, except for comprising:
- a first portion 2110 with a pulse generator 500 and one or more pulse generator electrical interfaces 505, configured and arranged to be electrically connected to one or more interconnection electrical interfaces 255. For example, the one or more pulse generator electrical interfaces 505 may comprise one or more solder pins, one or more connector pins, one or more connector sockets, one or more terminals, one or more wires, or any combination thereof;
- a conformable second portion 2120 with one or more interconnection electrical interfaces 255 to the one or more interconnections 250, configured and arranged to be electrically connected to the one or more pulse generator electrical interfaces 505. For example, the one or more interconnection electrical interfaces 255 may comprise one or more solder pins, one or more connector pins, one or more connector sockets, one or more terminals, one or more wires, or any connection thereof;
- at least one mechanical brace 2140 at the meeting of the conformable second portion 2120 and the first portion 2110, wherein the at least one mechanical brace 2140 is configured and arranged to resist separation of the conformable second portion 2120 from the first portion 2110. In the example depicted, the at least one mechanical brace 2140 is adjacent to and/or comprised in the second surface 320; and
- at least one encapsulation layer 2150, such as a PDMS, at least partially covering the first portion 2110 and at least partially covering the conformable second portion 2120. The at least one encapsulation layer 2150 is configured and arranged to provide a degree of protection against moisture ingress. Additionally or alternatively, the at least one encapsulation layer 2150 may be configured and arranged to provide a degree of separation resistance of the conformable second portion 2120 from the first portion 2110 due to, for example, its shape, extent, thickness and physical properties. For example, an encapsulation layer generally provides better separation protection the thicker it is, the greater adherence it has to the substrate, the stronger the material of the encapsulation layer is, the further it extends along the first and second portions of the substrate, etc. Additionally or alternatively, the at least one encapsulation layer 2150 may at least partially cover the conformable second portion 2120 and at least partially cover the first portion 2110. The region covered by the encapsulation may be coincident with the conformability changeover region, although in some such embodiments the conformability changeover region may extend beyond the encapsulation layer 2150, for example if another conformability-reducing feature extends beyond the encapsulation layer.

Optionally, the implantable stimulator 2101 depicted in longitudinal cross-section in FIG. 14A may be configured and arranged such that the first portion 2110 is fully covered by the at least one encapsulation layer 2150.

The electrical interfaces 255, 505 between the plurality of electrical interconnections 250 and the pulse generator 500 may be used to simplify manufacture and/or to allow a defective portion 2110, 2120, 2130 to be replaced. Additionally or alternatively, one or more pulse generator electrical interfaces 505 may be comprised in a wall of a hermetically-sealed enclosure of the pulse generator 500. This may further reduce the risk of moisture ingress to the pulse generator 500. Optionally, the one or more electrical interfaces 505 in the wall of an enclosure may be configured and arranged as an electrical feedthrough.

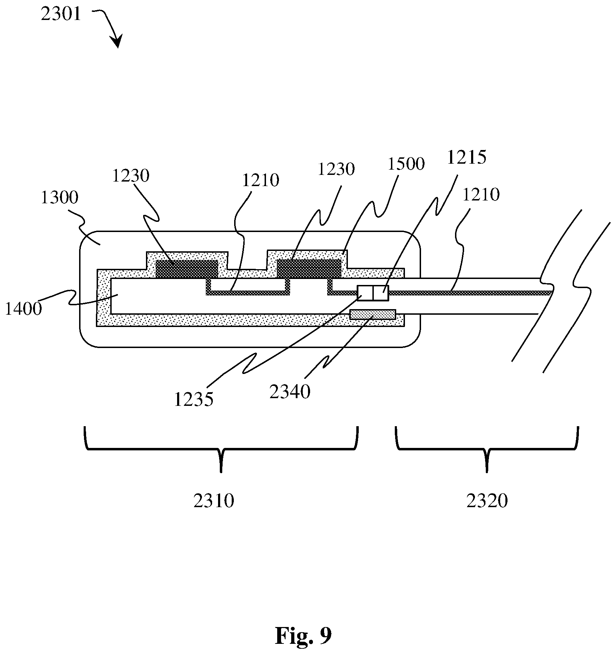

Optionally, an adhesion layer applied by vapor deposition, such as ALD, may be provided adjacent to at least part of the substrate 300 and covered by the at least one encapsulation layer 2150, for example in the conformability changeover region. This may improve adhesion of the at least one encapsulation layer 2150 to the substrate 300, particularly in combination with the use of at least one mechanical brace 2140 for strain relief. An example of such an adhesion layer is described below based on the exemplary embodiment depicted in FIG. 9.

Use of at least one mechanical brace 2140 for strain relief may further increase the usability of adhesion layers in devices where conformable portions are connected to less conformable (or non-conformable) portions. Such strain relief may allow even thinner adhesion layers to be used and/or reduce the risk that such a layer cracks and/or breaks.

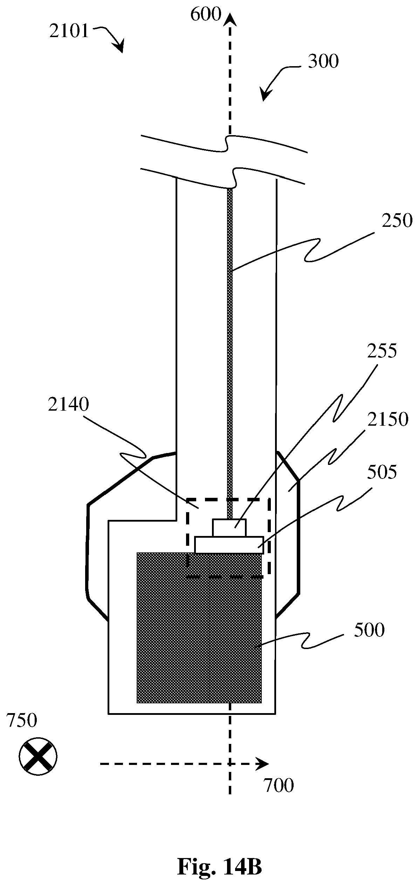

FIG. 14B depicts a depicts a transverse view of a longitudinal cross-section of the first embodiment 2101 - it is the same embodiment as depicted in FIG. 14A.

For clarity, the electrode portion is not depicted. The longitudinal cross-section depicted is through the one or more interconnections 250, and is depicted as lying in a plane through the longitudinal axis 600 and the first transverse axis 700.

The at least one mechanical brace 2140 is indicated in broken lines because, in this example, it is adjacent to and/or comprised in the second surface 320 as depicted in FIG. 14A.

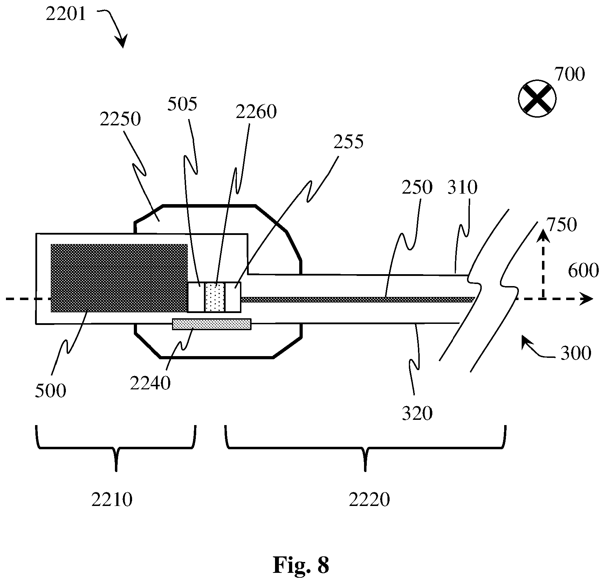

FIG. 8 depicts a transverse view of a longitudinal cross-section of a second embodiment 2201 of an implantable stimulator comprising a mechanical strain relief. For clarity, the electrode portion is not depicted. The longitudinal cross-section depicted is through the one or more interconnections 250, and is depicted as lying in a plane through the longitudinal axis 600 and the second transverse axis 750.

The implantable stimulator 2201 in FIG. 8 is the same as the implantable stimulator depicted in FIG. 14A, except that at least one conductive elastomer 2260, such an ACA (Anisotropic Conductive Adhesive), or an ACF (Anisotropic Conductive Film or Foam) or an ACP (Anisotropic Conductive Paste), is configured and arranged to electrically connect the one or more interconnection electrical interfaces 255 with the one or more pulse generator electrical interfaces 505. This elastomer may be separate from the electrical interfaces, or incorporated into one or more of the electrical interfaces. One or more conductors may extend through the conductive elastomer 2260, making electrical pathways through the at least one conductive polymer 2260. This may be further advantageous as it may allow simplified repair by replacement of a defective portion and/or simplified manufacturing.

Conductive elastomers, such as ACA elastomers, are most commonly used in dry environments, such as mobile phones or laptops, where many close electrical interconnections are to be made, and where soldering is not feasible due to the very small separation between interconnections and/or the materials used have a substantial degree of unsuitability for soldering. ACA elastomers are conventionally avoided in implantable devices because the separation between interconnection does not need to be small, the interconnection materials used are highly suitable for soldering, and because after implantation the implant may be subject to substantial ingress of moisture from the human or animal body. which may cause degradation and/or shorting. For example, conductive elastomers, such as ACA elastomers, have been shown to exhibit material degradation, resulting in reduced bonding strength, delamination and cracking when exposed to substantial degrees of moisture. See, for example, Table 1 in The detrimental effects of water on electronic devices (2021), Baylakoǧlu et al, doi: 10.1016/j.prime.2021.100016.

It is therefore surprising and unexpected to a skilled person that at least one conductive elastomer, such as an ACA elastomer, may be used in an implantable device. However, the use of a suitably configured encapsulant, such as a PDMS, may be used to substantially resist the ingress of moisture, an in particular to resist the ingress of damaging ions and/or salts from the human or animal body, into the region of the implantable device where the at least one conductive elastomer is located.

For example, the example depicted in FIG. 14A may be adapted such that one or more of the pulse generator 500 components are mounted to a less conformable (or more rigid) portion 2210 of a substrate, such as an FR-4 substrate, having an average transverse cross-sectional thickness of approximately 0.8mm to 2mm, such as 1mm. Additionally, a conformable portion 2220 (or less rigid) of a substrate 300, such as an LCP, may have an average transverse cross-sectional thickness of approximately 0.05mm to 0.5mm, such as 0.1mm. At least one encapsulation layer 2250 may be provided with an average transverse cross-sectional thickness of approximately 0.5mm to 2mm, such as 1.1 mm, on one or more sides of the substrate 300. The implantable device 2201 may have an average transverse cross-sectional thickness of approximately 1.8mm to 6mm, such as 3.3mm. The average transverse cross-sectional thickness may include two encapsulation regions on each side of the substrate 300, providing a high degree of protection of at least one conductive elastomer 2260. Conductive elastomers, such as an ACF, may typically provide a high degree of separation resistance.to a separation force directed approximately along the longitudinal axis 600. However, conductive elastomers may typically provide a low degree of separation resistance.to a separation force (or peel force) directed approximately along the second transverse axis 750 - for example, an ACF elastomer 2260 may provide a transverse separation resistance of approximately 5 N/cm with a substrate 300 having a transverse extent along the first transverse axis 700 of approximately 6mm to 10mm, such as 8mm. For an implantable stimulator 2201, a higher transverse separation resistance is preferred against a peel force of at least 15 Newton (N).By suitably configuring the one or more encapsulation layers 2250, a mechanical resistance may be provided against a peel force on the implantable device of up to 15 Newton which reduces the portion of the implantable device peel force which is applied to the conductive elastomer 2260, which may reduce the risk of mechanical separation along the second transverse axis 750. For example, it may be advantageous to reduce the portion of the implantable device peel force applied to the conductive elastomer 2260 by a factor of 3 or more.

Additionally or alternatively, the at least one mechanical brace 2240 may be configured and arranged to provide a degree of mechanical pre-tension which may be required to create one or more conducting paths through the conductive elastomer 2260. For example, by:
- assembling the implantable device while a degree of mechanical pressure is applied to force the first portion 2210 and the conformable second portion 2220 together. The force is then removed, whereby the at least one mechanical brace continues to force the first portion 2210 and the conformable second portion 2220 together; and/or
- configuring and arranging the at least one mechanical brace to 2240 to exert a degree of mechanical pressure to force the first portion 2210 and the conformable second portion 2220 together as the at least one mechanical brace 2240 is fixed in place during assembly.

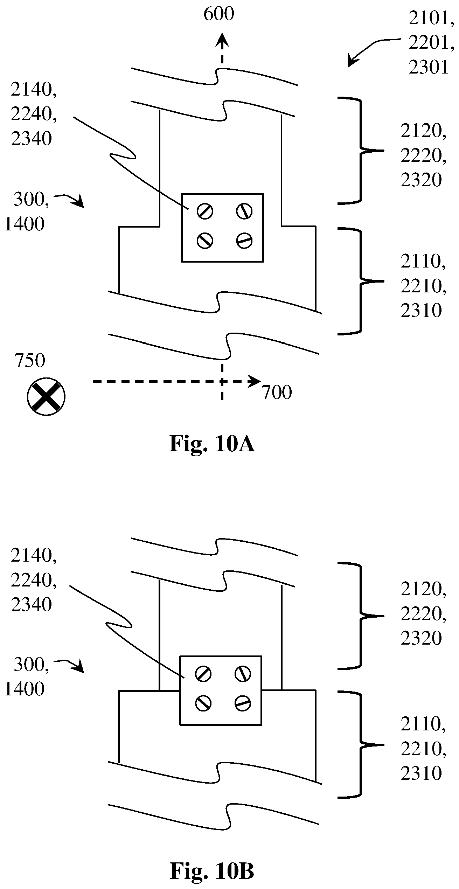

The conductive elastomer 2260 may advantageously configure the electrical interfaces 255, 505 to be releasable. This is further described below in relation to FIG. 10A to 10F.

The implantable stimulator 2201 in FIG. 8 further differs from the implantable stimulator depicted in FIG. 14A, by comprising:
- a first portion 2210 with a pulse generator 500 and one or more pulse generator electrical interfaces 505, configured and arranged to be electrically connected to the conductive elastomer 2260. In addition to the examples given for FIG. 14A, the one or more pulse generator electrical interfaces 505 may additionally or alternatively comprise one or more connector pins comprised in an electrical feedthrough;
- a conformable second portion 2220 with one or more interconnection electrical interfaces 255, configured and arranged to be electrically connected to the conductive elastomer 2260. In addition to the examples given for FIG. 14A, the one or more interconnection electrical interfaces 255 may additionally or alternatively comprise one or more connector pins comprised in an electrical feedthrough;

- at least one mechanical brace 2240 at the meeting of the conformable second portion 2220 and the first portion 2210, wherein the at least one mechanical brace 2240 is configured and arranged to resist separation of the conformable second portion 2220 from the first portion 2210. In the example depicted, the at least one mechanical brace 2240 is adjacent to and/or comprised in the second surface 320. Additionally or alternatively, the at least one mechanical brace 2240 may be configured and arranged to provide a degree of mechanical pre-tension; and
- at least one encapsulation layer 2250, at least partially covering the first portion 2210 and at least partially covering the conformable second portion 2220. Additionally or alternatively, the at least one encapsulation layer 2250 may be configured and arranged to provide a degree of separation resistance of the conformable second portion 2220 from the first portion 2210 due to, for example, its shape, extent, thickness and physical properties. Additionally or alternatively, the at least one encapsulation layer 2250 may fully cover the first portion 2210.

Additionally or alternatively, the at least one encapsulation layer 2250 may be configured and arranged to provide a degree of mechanical pre-tension which may be required to create one or more conducting paths through the conductive elastomer 2260. For example, in the same way as described above for FIG. 14A, the at least one encapsulation layer 2250 may be configured and arranged to provide a degree of separation resistance due to, for example, its shape, extent, thickness and physical properties. Additionally or alternatively, the at least one mechanical brace 2240 may comprise one or more opening, recess, cavity or similar, configured and arranged to receive an amount of encapsulant from the at least one encapsulation layer 2250.

The skilled person will also realize that the embodiments of implantable electrical or electronic devices described elsewhere in this disclosure may be similarly modified to comprise at least one mechanical brace at the meeting of a first portion comprising one or more electrical or electronic components, and a conformable second portion. For example, as depicted in the following figures and described in the relevant parts of the description:
- the implantable electrical or electronic device 1100 depicted in FIG. 11A;
- the implantable electrical or electronic device 1101 depicted in FIG. 11B; and
- the implantable electrical or electronic device 1102 depicted in FIG. 11C.

The skilled person will also realize that the embodiments of implantable devices described elsewhere in this disclosure may be similarly modified to comprise at least one mechanical brace at the meeting of a first portion comprising one or more electrical or electronic components, and a conformable second portion comprising two or more electrodes. For example, as depicted in the following figures and described in the relevant parts of the description:
- the implantable stimulator 100 depicted in FIG. 1A to 1C;
- the implantable stimulator 101 depicted in FIG. 2A to 2C;
- the implantable stimulator 102 depicted in FIG. 3A to 3C; and
- the implantable stimulators 103, 104, 105 depicted in FIG. 4A to 4C.

FIG. 9 depicts a transverse view of a longitudinal cross-section of a third embodiment 2301 of an implantable medical device, comprising an implantable electrical device, one or more electrodes, and a mechanical strain relief 2340. The implantable medical device 1110, described elsewhere in this disclosure, has been modified.

The third embodiment 2301 comprises:
- a substrate 1400 having one or more electrical conductors 1210. The substrate 1400 comprises three protected surfaces, each surface being protected by an optional adhesion layer 1500 and at least one encapsulation layer 1300. The three protected surfaces comprise two opposite protected surfaces and a further adjacent surface. In this example, the two opposite surfaces are depicted as the top and bottom surfaces, and the further adjacent surface is depicted on the left. In the example depicted, the optional adhesion layer 1500 is provided adjacent to at least part of the substrate 1400 and covered by the at least one encapsulation layer 1300. The adhesion layer 1500 is preferably applied by vapor deposition, such as ALD.

The extent of the adhesion layer 1500 is less than the extent of the substrate 1400 for the two opposite protected surfaces. The extent of the adhesion layer 1500 is greater than the extent of the substrate 1400 for the third adjacent protected surface. The extent of the at least one encapsulation layer 1300 is less than the extent of the substrate 1400 for the two opposite protected surfaces.

The extent of the at least one encapsulation layer 1300 is greater than the extent of the substrate 1400 for the third adjacent protected surface; and the extent of the adhesion layer 1500 is less than the extent of the at least one encapsulation layer 1300 for each protected surface.

The substrate comprises a first portion 2310 along which one or more electrical or electronic components 1230 are located, such as comprised in one or more sensors and/or comprised in a pulse generator.

One or more stimulation electrodes are located along a conformable second portion 2320 of the substrate 1400. For clarity, the one or more stimulation electrodes, are not depicted in FIG. 9.

The first portion 2310 is less conformable than the conformable second portion 2320 due to the presence of one or more electrical or electronic components 1230. The degree of conformability of the first portion 2310 may be reduced if the average thickness of the one or more electrical or electronic components 1230 is greater than the average thickness of the electrodes and/or the average thickness of the interconnections 1210. The degree of conformability of the first portion 2310 may be further reduced if, for example, one or more components 1230 are mounted to a board and/or material as described above.

The one or more interconnections 1210 are depicted schematically as a single connection between the at least two electrodes and the one or more electrical or electronic components 1230.

The implantable medical device 2301 further comprises:
- a conformability changeover region between the first portion 2310 and the conformable second portion 2320. The position and extent of the conformability changeover region is affected by differences in relevant parameters and properties between the less conformable first portion 2310 and the conformable second portion 2320.

As depicted, the one or more electrical or electronic components 1230 are embedded in or encapsulated by the at least one encapsulation layer 1300. In other words, the at least one encapsulation layer 1300 covers the one or more electrical or electronic components 1230. Optionally, the implantable medical device 2301 depicted in longitudinal cross-section in FIG. 9 may be configured and arranged such that the one or more electrical or electronic components 1230 are fully covered by the at least one encapsulation layer 1300.

In the example depicted, the optional adhesion layer 1500 is provided adjacent to at least part of the one or more electrical or electronic components 1230 and covered by the at least one encapsulation layer 1300. In other words, the optional adhesion layer 1500 covers the one or more electrical or electronic components 1230. Optionally, the implantable medical device 2301 depicted in longitudinal cross-section in FIG. 9 may be configured and arranged such that the one or more electrical or electronic components 1230 are fully covered by the optional adhesion layer 1500.

The implantable medical device 2301 further comprises:
- at least one mechanical brace 2340 provided for strain relief, configured to resist separation of the conformable second portion 2320 from the first portion 2310. In the example depicted, the at least one mechanical brace 2340 is adjacent to and/or comprised in the surface of the substrate 1400.

The first portion 2310 further comprises one or more component electrical interfaces 1235, configured and arranged to be electrically connected to one or more interconnection electrical interfaces 1215.

The conformable second portion 2320 comprises one or more interconnection electrical interfaces 1215, configured and arranged to be electrically connected to the one or more component electrical interfaces 1235.

In the example depicted, the at least one encapsulation layer 1300 covers the first portion 2310 of the substrate 1400, and at least partially covers the conformable second portion 2320. In the example depicted, the optional adhesion layer 1500 covers the first portion 2310 of the substrate 1400, and at least partially covers the conformable second portion 2320.

Optionally, the implantable medical device 2301 depicted in longitudinal cross-section in FIG. 9 may be configured and arranged such that the first portion 2310 is fully covered by the at least one encapsulation layer 1300.

Optionally, the implantable medical device 2301 depicted in longitudinal cross-section in FIG. 9 may be configured and arranged such that the first portion 2310 is fully covered by the optional adhesion layer 1500.

Use of at least one mechanical brace 2340 for strain relief may further increase the usability of adhesion layers 1500 in devices where conformable portions are connected to less conformable (or non-conformable) portions. Such strain relief may allow even thinner adhesion layers 1500 to be used and/or reduce the risk that such a layer cracks and/or breaks.

Other embodiments of implantable devices described elsewhere in this disclosure may also be modified - for example, those as depicted in the following figures and described in the relevant parts of the description:
- the implantable medical device 1110 depicted in FIG. 12A; and
- the implantable medical device 1111 depicted in FIG. 12B.

In general, the at least one mechanical brace 2140, 2240, 2340 may be configured and arranged to provide the most suitable degree of strain relief.

FIG. 10A to 10F are bottom views of exemplary implementations of at least one mechanical brace 2140, 2240, 2340 consistent with certain embodiments of the present invention. In these examples, the same view is used as described above in relation to FIG. 14B. For clarity, the at least one encapsulation layer and the optional adhesion layer are not depicted.

One or more aspects of these examples may be combined with each other and/or with any other equivalent mechanical brace. In these examples, as depicted, the extent of a first portion 2110, 2210, 2310 along the first transverse axis 700 is greater than the extent of a conformable second portion 2120, 2220, 2320 along the first transverse axis 700.

FIG. 10A depicts at least one mechanical brace 2140, 2240, 2340 comprising a rigid plate attached to the conformable second portion 2120, 2120, 2320 and the first portion 2110, 2210, 2310 of a substrate 300, 1400 using one or more fasteners. The one or more fasteners pass through the rigid plate.

As depicted in this example, four screws have been used with two screws attached to the conformable second portion 2120, 2120, 2320 and two screws attached to the first portion 2110, 2210, 2310. However, any other suitable fastener, such as one or more rivets, bolts, pins, pegs, spikes, hooks, protrusions, or any combination thereof may be used. Any suitable number of fasteners may be used in any suitable configuration.

Additionally or alternatively, a bonding agent, such as an adhesive, glue, epoxy, cement or any combination thereof, may be used to attach a rigid plate to a substrate 300, 1400.

Additionally or alternatively, a rigid plate may be provided with one or more mechanical elements, such as one or more openings, recesses, cavities, grooves or any combination thereof. Corresponding and co-operating mechanical elements are provided at suitable locations on a conformable second portion 2120, 2220, 2320 and/or a first portion 2110, 2210, 2310, such as one or more fasteners, such as one or more screws, rivets, bolts, pins, pegs, spikes, hooks, protrusions, projections, or any combination thereof.

One or more screws may be advantageous as they are releasable, allowing the at least one mechanical brace 2140, 2240, 234 to be fitted, removed and/or replaced.

FIG. 10B depicts a further example, which is the same as the example in FIG. 10A except for comprising a substrate 300, 1400 with a separate conformable second portion 2120, 2220, 2320 and a separate first portion 2110, 2210, 2310. This may be advantageous as it allows two different pieces of substrate 300, 1400 to be joined during a simplified manufacturing process. If required, at least one encapsulation layer may be applied to one or more portions. Optionally, an adhesion layer may also be used.

As depicted in this example, it may also be advantageous that the at least one mechanical brace 2140, 2240, 2340 is configured and arranged to be releasable, such as using a rigid plate and one or more screws. This may allow repair and/or upgrading of a previously manufactured implantable device by replacing one or more portions.

It may be further advantageous to make the one or more electrical interfaces mutually releasable. For example, the embodiment depicted in FIG. 14A may be modified to provide a separate conformable second portion 2120 and a separate first portion 2110. Each portion comprises one or more complementary releasable electrical interfaces 255, 505, such as one or more plugs co-operating with one or more sockets.

Similarly, the embodiment depicted in FIG. 9 may be modified to provide a separate conformable second portion 2320 and a separate first portion 2310. Each portion comprises one or more complementary releasable electrical interfaces 1215, 1235.

Similarly, the embodiment depicted in FIG. 8 may be advantageously modified to provide a separate conformable second portion 2220 and a separate first portion 2210. Each portion comprises one or more complementary electrical interfaces, configured to be releasable. For example, at least one conductive polymer 2260 is provided, such as an ACF, configured and arranged to electrically connect the one or more interconnection electrical interfaces 255 with the one or more pulse generator electrical interfaces 505.

Similarly, the embodiment depicted in FIG. 9 may be modified whereby a separable first portion 2310 comprises one or more releasable component electrical interfaces 1235, and a separable conformable portion 2320 comprises one or more releasable interconnection electrical interfaces 1215. At least one conductive polymer is provided, such as an ACF, configured and arranged to electrically connect the one or more interconnection electrical interfaces 1215 with the one or more component electrical interfaces 1235.

If required, at least one encapsulation layer may be applied to one or more portions after repair and/or upgrading. Optionally, an adhesion layer may also be used.

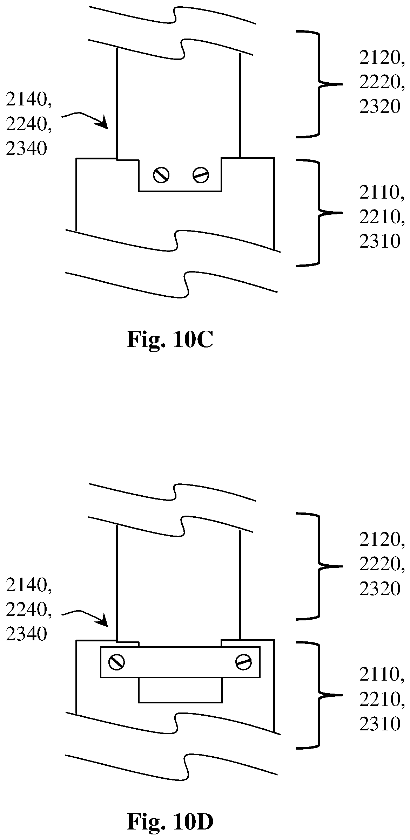

FIG. 10C depicts a further example, which is the same as the examples in FIG. 10A or FIG. 10B, except for an integral section of a conformable second portion 2120, 2220, 2320 proximate a first portion 2110, 2210, 2310 being configured and arranged as described above for a rigid plate. The at least one mechanical brace 2140, 2240, 2340 comprises the integral section of the conformable second portion 2120, 2220, 2320. Therefore, in this example, the one or more fasteners used are only provided for attachment to the first portion 2110, 2210, 2310, and pass through the integral section of the conformable second portion 2120, 2220, 2320.

Additionally or alternatively, an integral section of the first portion 2110, 2210, 2310 proximate the conformable second portion 2120, 2220, 2320 may be similarly configured and arranged as at least one mechanical brace 2140, 2240, 2340.

FIG. 10D depicts a further example, which is the same as the examples in FIG. 10A or FIG. 10B, except for an integral section of a conformable second portion 2120, 2220, 2320 proximate a first portion 2110, 2210, 2310 being longitudinally extended and passing through at least one clamp attached to the first portion 2110, 2210, 2310.

The at least one mechanical brace 2140, 2240, 2340 comprises the combination of the integral section of the conformable second portion 2120, 2220, 2320 and the clamp attached to the first portion 2110, 2210, 2310.

Additionally or alternatively, an integral section of the first portion 2110, 2210, 2310 proximate the conformable second portion 2120, 2220, 2320 may be similarly configured and arranged as at least one mechanical brace 2140, 2240, 2340 by cooperating with at least one clamp attached to the conformable second portion 2120, 2220, 2320.

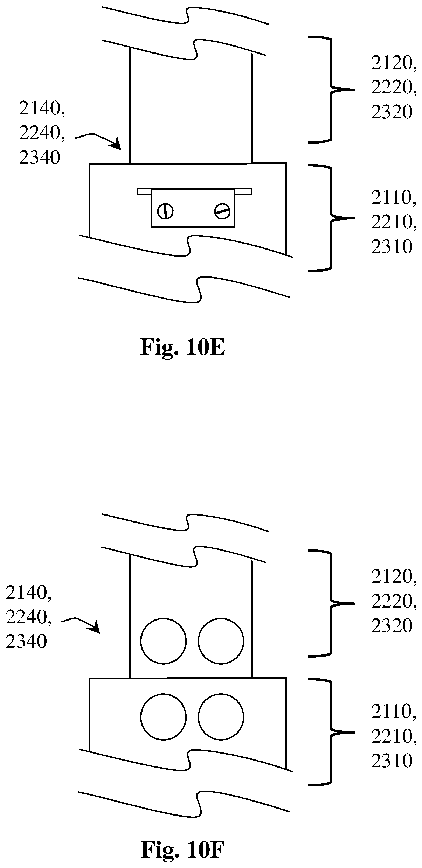

FIG. 10E depicts a further example, which is the same as the example in FIG. 10C, except for an integral section of a conformable second portion 2120, 2220, 2320 proximate a first portion 2110, 2210, 2310 being further longitudinally extended and passing through at least one slit-like openings in the first portion 2110, 2210, 2310.

The at least one mechanical brace 2140, 2240, 2340 in this example comprises the integral section of the conformable second portion 2120, 2220, 2320, the one or more slit-like openings, and the one or more fasteners pass through the integral section to attach to the first portion 2110, 2210, 2310.

Additionally or alternatively, an integral section of the first portion 2110, 2210, 2310 proximate the conformable second portion 2120, 2220, 2320 may be similarly configured and arranged as at least one mechanical brace 2140, 2240, 2340 by cooperating with at one or more slit-like openings in the conformable second portion 2120, 2220, 2320.

Additionally or alternatively, an integral section of a portion may be further longitudinally extended, resembling one or more belts, bands, straps, tapes, or any combination thereof. One or more suitable cooperating elements may be provided in the other portion, such as one or more slots, slits, grooves, buckles, channels, openings, or any combination thereof.

As described above, the at least one encapsulation layer 1300, 2150 may be configured and arranged to provide a degree of separation resistance due to, for example, its shape, extent, thickness and physical properties.

Additionally, the at least one mechanical brace 2140, 2240, 2340 may be an opening, recess, cavity or similar, configured and arranged to receive an amount of encapsulant from the at least one encapsulation layer 1300, 2150.

FIG. 10F depicts a further example, which is the same as the example in FIG. 10B, except for the rigid plate being replaced by one or more openings in a conformable second portion 2120, 2220, 2320, and one or more openings in a first portion 2110, 2210, 2310.

In this example, a mechanical brace 2140, 2240, 2340 is provided comprising one or more openings configured and arranged to receive significant amounts of encapsulant from the at least one encapsulation layer. After applying the at least one encapsulation layer, .the significant amounts of encapsulant may provide separation resistance of the conformable second portion 2120, 2220, 2320 from the first portion 2110, 2210, 2310. Optionally, an adhesion layer may also be used to improve adhesion to one or more surfaces of the openings.

The descriptions thereof herein should not be understood to prescribe a fixed order of performing the method steps described therein. Rather the method steps may be performed in any order that is practicable. Similarly, the examples used to explain the algorithm are presented as non-limiting examples, and are not intended to represent the only implementations of these algorithms. The person skilled in the art will be able to conceive many different ways to achieve the same functionality as provided by the embodiments described herein.

For example, one or more features that improve conformance may be applied to embodiments that are configured and arranged for improved encapsulation. In some embodiments, it may be advantageous to apply features that improve encapsulation but reduce conformance.

For example, one or more features that improve encapsulation may be applied to embodiments that are configured and arranged for improved conformance. In some embodiments, it may be advantageous to apply features that improve conformance but reduce encapsulation.

Many types of implantable distal ends of stimulation devices are depicted. But this does not exclude that the rest of the device is implanted. This should be interpreted as meaning that at least the electrode section of the distal end is preferably configured and arranged to be implanted.

Although the present invention has been described in connection with specific exemplary embodiments, it should be understood that various changes, substitutions, and alterations apparent to those skilled in the art can be made to the disclosed embodiments without departing from the scope of the invention as set forth in the appended claims.

In a non-limiting example,
- one or more electrodes of the first type 200a, 200b, 1220 are comprised in the first surface 310, 1410 and one or more electrodes of the second type 400a, 400b, 1220 are comprised in the second surface 320, 1420; or
- one or more electrodes of the first type 200a, 200b, 1220 are comprised in the first surface 310, 1410 and one or more electrodes of the second type 400a, 400b, 1220 are also comprised in the first surface 310, 1410; or
- one or more electrodes of the first type 200a, 200b, 1220 are comprised in the second surface 320, 1420 and one or more electrodes of the second type 400a, 400b, 1220 are comprised in the first surface 310, 1410; or
- one or more electrodes of the first type 200a, 200b, 1220 are comprised in the second surface 320, 1420 and one or more electrodes of the second type 400a, 400b, 1220 are also comprised in the second surface 320, 1420; or
- any combination thereof.

By providing relatively larger higher electrode 200, 400, 1220 surfaces, stimulators 100, 101, 102, 103, 104, 105, 1100, 1101, 1102 may be operated at a lower energy / lower power. This may be advantageous in applications where high frequency and/or burst stimulation is used.

High frequency operation may require more energy to be provided by the pulse generator 500. In applications where energy / power is critical, such as, in a non-limiting example, if an increased operating lifetime is desired from a power source for the pulse generator 500), any reduction in required power may be advantageous. High frequency operation may be considered as generating electrical stimulation pulses with a frequency of 1000 Hz or more, preferably 1500 Hz or more, more preferably 2000 Hz or more, yet more preferably 2500 Hz or more.

In an embodiment, experiments with burst stimulation have been performed such as Burst Occipital Nerve Stimulation for Chronic Migraine and Chronic Cluster Headache by Garcia-Ortega et al, Neuromodulation 2019; 22: 638-644, DOI: 10.1111/ner.12977.

For burst operation, the pulse generator 500 is further configured and arranged to generate electrical stimulation pulses in groups of stimulation pulses.

In a non-limiting example, groups (or bursts) of stimulation pulses may comprise 2 to 10 pulses, more preferably 2 to 5 stimulation pulses. Stimulation pulses in a group may have a repetition frequency of more than 500 Hz, typically 1000Hz or more. Groups may be repeated at more than 5 Hz, typically 40 Hz or more.

As with high frequency operations, burst operation may require more energy to be provided by the pulse generator 500, and any reduction in required power may be advantageous.

Additionally, the speed of charge-balance recovery may also increase with a lower impedance. By using a relatively thin-foil substrate 300, 1400, stimulation between an electrode of the first type 200, 1220 comprised in one surface 310, 1410, 320, 1420 and an electrode of the second type 400, 1220 comprised in the other surface 310, 1410, 320, 1420, the current path in tissue is relatively short, reducing impedance.

Similarly, using a substrate 300, 1400 and stimulation between an electrode of the first type 200, 1220 comprised in one surface 310, 1410, 320, 1420 and an adjacent electrode of the second type 400, 1220 comprised in the same surface 310, 1410, 320, 1420 provide a relatively short path through tissue.

While certain illustrative embodiments have been described, it is evident that many alternatives, modifications, permutations and variations will become apparent to those skilled in the art in light of the foregoing description without departing from the scope of the invention as set forth in the following claims.

The invention encompasses every possible combination of the various features of each embodiment disclosed. One or more of the elements described herein with respect to various embodiments can be implemented in a more separated or integrated manner than explicitly described, or even removed or rendered as inoperable in certain cases, as is useful in accordance with a particular application.

### REFERENCE NUMERALS:

100, 101, 102 implantable stimulators
103, 104, 105 further embodiments of implantable stimulators
200a, 200b, 200c, 200d one or more stimulation electrodes
250 one or more stimulation electrical interconnection layers
255 one or more interconnection electrical interfaces
300 a conformable foil-like substrate
310, 320 a first and second surface
400, 400a, 400b, 400c, 400d one or more return electrodes
500 a pulse generator
505 one or more pulse generator electrical interfaces
600 a longitudinal axis
700 a first transverse axis
750 a second transverse axis
810 location for left supraorbital nerve or cortical stimulation
820 location for right supraorbital stimulation
830, 830a, 830b location for left occipital nerve stimulation
840, 840a, 840b location for right occipital nerve stimulation
850 location for deep brain stimulation
860 location for vagus nerve, carotid artery, carotid sinus, phrenic nerve or hypoglossal stimulation
865 location for cerebral spinal cord stimulation
870 location for peripheral nerve stimulation
875 location for spinal cord stimulation
880 location for gastric stimulation
885 location for sacral & pudendal nerve stimulation
890 location for sacral neuromodulation
895 location for fibular nerve stimulation
910 left supraorbital nerve
920 right supraorbital nerve
930 left greater occipital nerve
940 right greater occipital nerve
1100, 1101, 1102 improved implantable electrical or electronic devices
1110, 1111 improved implantable medical devices
1210 one or more electrical conductor
1215 one or more interconnection electrical interfaces
1220 one or more stimulation electrodes
1230 one or more sensors
1235 one or more component electrical interfaces
1300 a further biocompatible encapsulation layer
1310 a first biocompatible encapsulation layer
1320 a second biocompatible encapsulation layer
1400 a substrate
1410 a first surface
1420 a second surface
1500 a further adhesion layer
1510 a first adhesion layer
1520 a second adhesion layer
1750 Graph comparing the average pull force under dry conditions and after soaking
1761a Average peel force for sample 2.1
1762a, 1762b Average peel force for sample 2.2
1763a, 1763b Average peel force for sample 2.3
1764a, 1764b Average peel force for sample 2.4
1765a, 1765b Average peel force for sample 2.5
2010 a first portion
2020 a conformable second portion
2101 a first embodiment comprising a mechanical strain relief
2110 a first portion with an electrical interface
2120 a conformable second portion with an electrical interface
2140 at least one mechanical brace
2150 at least one encapsulation layer
2201 a second embodiment comprising a mechanical strain relief
2210 a first portion with an electrical interface
2220 a conformable second portion with an electrical interface
2240 at least one mechanical brace
2250 at least one encapsulation layer
2260 a conductive elastomer
2301 a third embodiment comprising a mechanical strain relief
2310 a first portion with an electrical interface
2320 a conformable second portion with an electrical interface
2340 at least one mechanical brace

## Claims

1. An implantable stimulator, comprising: a substrate (300, 1400) comprising a first surface and a second surface, wherein a thickness of the substrate (300, 1400) is defined by the first and second surfaces;
the substrate (300, 1400) comprising a first portion (2010, 2110, 2210, 2310) along which a pulse generator (500) is located, the pulse generator (500) comprising one or more electrical or electronic components, configured to generate at least one stimulation pulse;
an electrode array (200, 400, 1220) comprising at least two electrodes located along a conformable second portion (2020, 2120, 2320) of the substrate (300, 1400);
a plurality of electrical interconnections (250, 1210) electrically coupling the pulse generator (500) to the at least two electrodes of the electrode array (200, 400, 1220), wherein the plurality of electrical interconnections (250, 1210) are positioned between the first and second surfaces of the substrate (300, 1400), the implantable stimulator (100, 101, 102, 103, 104, 105, 1110, 1111, 2101, 2201, 2301) comprising one or more electrical interfaces between the first portion (2010, 2110, 2210, 2310) and the conformable second portion (2020, 2120, 2320), the one or more electrical interfaces being between the plurality of electrical interconnections (250, 1210) and the pulse generator (500);
wherein the implantable stimulator (100, 101, 102, 103, 104, 105, 1110, 1111, 2101, 2201, 2301) comprises at least one conductive elastomer (2260), configured and arranged to electrically connect one or more electrical interfaces between the plurality of electrical interconnections (250, 1210) and the pulse generator (500);
wherein the thickness of the substrate (300, 1400) along the conformable second portion (2020, 2120, 2320) is equal to or less than 0.5 millimeters;
the implantable stimulator further comprising at least one encapsulation layer (1300, 1310, 1320, 2150, 2250) at least partially covering the first portion (2010, 2110, 2210, 2310) and at least partially covering the conformable second portion (2020, 2120, 2320) of the substrate (300, 1400), the at least one encapsulation layer (1300, 1310, 1320, 2150, 2250) being configured to resist separation of the conformable second portion (2020, 2120, 2320) from the first portion (2010, 2110, 2210, 2310) at the meeting of the first portion (2010, 2110, 2210, 2310) and the conformable second portion (2020, 2120, 2320); and
wherein the at least one encapsulation layer (1300, 1310, 1320, 2150, 2250) is configured and arranged to provide a degree of mechanical pre-tension such that one or more conducting paths through the at least one conductive elastomer is created.

2. The implantable stimulator of Claim 1, wherein the at least one conductive elastomer (2260) is at the meeting of the first portion (2010, 2110, 2210, 2310) and the conformable second portion (2020, 2120, 2220, 2320).

3. The implantable stimulator of Claim 1 or Claim 2, wherein the degree of separation resistance of the conformable second portion (2120) from the first portion (2110) provided by the at least one encapsulation layer (1300, 1310, 1320) is due to at least one property of the at least one encapsulation layer (1300, 1310, 1320), wherein the at least one property is a shape, an extent, a thickness, a physical property, an adherence, or any combination thereof.

4. The implantable stimulator of any preceding Claim, wherein the at least one encapsulation layer (1300, 1310, 1320, 2150, 2250) covers the first portion (2010, 2110, 2210, 2310) of the substrate (300, 1400).

5. The implantable stimulator of any preceding Claim, wherein the at least one conductive elastomer (2260), is an Anisotropic Conductive Adhesive, an Anisotropic Conductive Film, an Anisotropic Conductive Foam, an Anisotropic Conductive Paste, or any combination thereof.

6. The implantable stimulator of any preceding Claim, wherein the substrate (300, 1400) further comprises at least one mechanical brace (2140, 2240, 2340) at the meeting of the first portion (2010, 2110, 2210, 2310) and the conformable second portion (2020, 2120, 2320), the at least one mechanical brace (2140, 2240, 2340) being configured to resist separation of the conformable second portion (2020, 2120, 2320) from the first portion (2010, 2110, 2210, 2310) and/or the at least one mechanical brace (2140, 2240, 2340) being configured to provide a degree of mechanical pre-tension such that one or more conducting paths through the at least one conductive elastomer (2260) is created.

7. The implantable stimulator of Claim 6, wherein the at least one mechanical brace (2140, 2240, 2340) comprises: at least one protrusion, at least one projection, at least one opening, at least one groove, at least one pin, at least one hook, at least one rivet, or any combination thereof.

8. The implantable stimulator of any one of Claims 6 to 7, wherein the at least one mechanical brace (2140, 2240, 2340) comprises one or more openings and/or grooves, configured to receive significant amounts of encapsulant from the at least one encapsulation layer.

9. The implantable stimulator of any one of Claims 6 to 8, wherein the at least one mechanical brace (2140, 2240, 2340) is configured to be releasable.

10. The implantable stimulator of any preceding Claim, wherein the implantable stimulator (100, 101, 102, 103, 104, 105, 1110, 1111, 2101, 2201, 2301) comprises an adhesion layer (1500, 1510, 1520) adjacent to at least part of the first portion (2010, 2110, 2210, 2310) and at least part of the conformable second portion (2020, 2120, 2320) of the substrate (300, 1400).

11. The implantable stimulator of Claim 10, wherein the adhesion layer (2020, 2120, 2320) is applied by dip-coating adjacent to at least part of the encapsulation layer (1300, 1310, 1320, 2150, 2250).

12. The implantable stimulator of any preceding Claim, wherein the one or more electrical interfaces are between the plurality of electrical interconnections (250, 1210) and the pulse generator (500).

13. The implantable stimulator of any preceding Claim, wherein the thickness of the stimulator (100, 101, 102, 103, 104, 105, 1110, 1111, 2101, 2201, 2301) along the first portion (2010, 2110, 2210, 2310) is equal to or less than 5 millimeters, or equal to or less than 4 millimeters, or equal to or less than 3 millimeters.

14. The implantable stimulator of any preceding Claim, wherein the encapsulation layer (1300, 1310, 1320, 2150, 2250) comprises a polymer.

15. The implantable stimulator of Claim 14, wherein the encapsulation layer (1300, 1310, 1320, 2150, 2250) comprises Polydimethylsiloxane (PDMS).

16. The implantable stimulator of any preceding Claim, wherein the conformable second portion (2020, 2120, 2320) of the substrate (300, 1400) comprises a liquid crystal polymer (LCP).

17. The implantable stimulator of Claim 16, wherein the conformable second portion (2020, 2120, 2320) of the substrate (300, 1400) comprises one or more layers of the LCP.

18. The implantable stimulator of any preceding Claim, wherein the thickness of the substrate (300, 1400) along the conformable second portion (2020, 2120, 2320) is equal to or less than 0.3 millimeters, or equal to or less than 0.2 millimeters, or equal to or less than 0.1 millimeters.

19. The implantable stimulator of any preceding Claim, wherein the first portion (2010, 2110, 2210, 2310) of the substrate (300, 1400) comprises a rigid circuit board, a rigid PCB and/or a rigid ceramic substrate.

20. A method of manufacturing an implantable stimulator, comprising:
providing a substrate (300, 1400), the substrate (300, 1400) comprising a first surface and a second surface, wherein a thickness of the substrate (300, 1400) is defined by the first and second surfaces;
providing a pulse generator (500) along a first portion (2010, 2110, 2210, 2310) of the substrate (300, 1400), the pulse generator (500) comprising one or more electrical or electronic components configured to generate at least one stimulation pulse;
locating an electrode array (200, 400, 1220) comprising at least two electrodes along a conformable second portion (2020, 2120, 2320) of the substrate (300, 1400);
depositing or electro-plating onto the substrate (300, 1400) a plurality of electrical interconnections (250, 1210) electrically coupling the pulse generator (500) to the at least two electrodes of the electrode array (200, 400, 1220);
providing one or more electrical interfaces between the first portion (2010, 2110, 2210, 2310) and the conformable second portion (2020, 2120, 2320), wherein the one or more electrical interfaces are between the plurality of electrical interconnections (250, 1210) and the pulse generator (500);
providing one or more electrical interfaces between the first portion (2010, 2110, 2210, 2310) and the conformable second portion (2020, 2120, 2320), the one or more electrical interfaces being between the plurality of electrical interconnections (250, 1210) and the pulse generator (500);
providing at least one conductive elastomer (2260) to electrically connect one or more electrical interfaces between the plurality of electrical interconnections (250, 1210) and the pulse generator (500), wherein the thickness of the substrate (300, 1400) along the conformable second portion (2020, 2120, 2320) is equal to or less than 0.5 millimeters;
providing at least one encapsulation layer (1300, 1310, 1320, 2150, 2250) to at least partially cover the first portion (2010, 2110, 2210, 2310) and to at least partially cover the conformable second portion (2020, 2120, 2320) of the substrate (300, 1400), the at least one encapsulation layer (1300, 1310, 1320, 2150, 2250) being configured to resist separation of the conformable second portion (2020, 2120, 2320) from the first portion (2010, 2110, 2210, 2310) at the meeting of the first portion (2010, 2110, 2210, 2310) and the conformable second portion (2020, 2120, 2320); and
configuring and arranging the at least one encapsulation layer (1300, 1310, 1320, 2150, 2250) to provide a degree of mechanical pre-tension such that one or more conducting paths through the at least one conductive elastomer (2260) is created.

21. The method of Claim 20, wherein the method further comprises providing at least one mechanical brace (2140, 2240, 2340) at the meeting of the first portion (2010, 2110, 2210, 2310) and the conformable second portion (2020, 2120, 2320) of the substrate (300, 1400); and configuring the at least one mechanical brace (2140, 2240, 2340) to resist separation of the conformable second portion (2020, 2120, 2320) from the first portion (2010, 2110, 2210, 2310) and/or configuring the at least one mechanical brace (2140, 2240, 2340) to provide a degree of mechanical pre-tension such that one or more conducting paths through the lat least one conductive elastomer (2260) is created.

## Patentansprüche

1. Implantierbarer Stimulator, der Folgendes umfasst: ein Substrat (300, 1400), das eine erste Oberfläche und eine zweite Oberfläche umfasst, wobei eine Dicke des Substrats (300, 1400) durch die erste und zweite Oberfläche definiert ist;
wobei das Substrat (300, 1400) einen ersten Teil (2010, 2110, 2210, 2310) umfasst, entlang dessen sich ein Impulsgenerator (500) befindet, wobei der Impulsgenerator (500) eine oder mehrere elektrische oder elektronische Komponenten umfasst, die zur Erzeugung von mindestens einem Stimulationsimpuls konfiguriert sind;
eine Elektrodenanordnung (200, 400, 1220), die mindestens zwei Elektroden umfasst, die sich entlang eines anpassbaren zweiten Teils (2020, 2120, 2320) des Substrats (300, 1400) befinden;
eine Vielzahl von elektrischen Verbindungen (250, 1210), die den Impulsgenerator (500) mit den mindestens zwei Elektroden der Elektrodenanordnung (200, 400, 1220) elektrisch verbindet, wobei die Vielzahl von elektrischen Verbindungen (250, 1210) zwischen der ersten und zweiten Oberfläche des Substrats (300, 1400) positioniert ist, wobei der implantierbare Stimulator (100, 101, 102, 103, 104, 105, 1110, 1111, 2101, 2201, 2301) eine oder mehrere elektrische Schnittstellen zwischen dem ersten Teil (2010, 2110, 2210, 2310) und dem anpassbaren zweiten Teil (2020, 2120, 2320) umfasst, wobei die eine oder mehreren elektrischen Schnittstellen zwischen der Vielzahl von elektrischen Verbindungen (250, 1210) und dem Impulsgenerator (500) vorliegen;
wobei der implantierbare Stimulator (100, 101, 102, 103, 104, 105, 1110, 1111, 2101, 2201, 2301) mindestens ein leitfähiges Elastomer (2260) umfasst, das zur elektrischen Verbindung von einer oder mehreren elektrischen Schnittstellen zwischen der Vielzahl von elektrischen Verbindungen (250, 1210) und dem Impulsgenerator (500) konfiguriert und angeordnet ist;
wobei die Dicke des Substrats (300, 1400) entlang des anpassbaren zweiten Teils (2020, 2120, 2320) gleich oder kleiner als 0,5 Millimeter ist;
wobei der implantierbare Stimulator weiter mindestens eine Verkapselungsschicht (1300, 1310, 1320, 2150, 2250) umfasst, die zumindest teilweise den ersten Teil (2010, 2110, 2210, 2310) bedeckt und zumindest teilweise den anpassbaren zweiten Teil (2020, 2120, 2320) des Substrats (300, 1400) bedeckt, wobei die mindestens eine Verkapselungsschicht (1300, 1310, 1320, 2150, 2250) konfiguriert ist, um einer Abtrennung des anpassbaren zweiten Teils (2020, 2120, 2320) von dem ersten Teil (2010, 2110, 2210, 2310) beim Aufeinandertreffen des ersten Teils (2010, 2110, 2210, 2310) und des anpassbaren zweiten Teils (2020, 2120, 2320) zu widerstehen; und
wobei die mindestens eine Verkapselungsschicht (1300, 1310, 1320, 2150, 2250) zur Bereitstellung eines Grads an mechanischer Vorspannung konfiguriert und angeordnet ist, sodass ein oder mehrere leitende Pfade durch das mindestens eine leitfähige Elastomer erzeugt werden.

2. Implantierbarer Stimulator nach Anspruch 1, wobei das mindestens eine leitfähige Elastomer (2260) beim Aufeinandertreffen des ersten Teils (2010, 2110, 2210, 2310) und des anpassbaren zweiten Teils (2020, 2120, 2220, 2320) vorliegt.

3. Implantierbarer Stimulator nach Anspruch 1 oder Anspruch 2, wobei der Grad des Abtrennungswiderstands des anpassbaren zweiten Teils (2120) von dem ersten Teil (2110), der durch die mindestens eine Verkapselungsschicht (1300, 1310, 1320) bereitgestellt wird, aufgrund von mindestens einer Eigenschaft der mindestens einen Verkapselungsschicht (1300, 1310, 1320) vorliegt, wobei die mindestens eine Eigenschaft eine Form, eine Ausdehnung, eine Dicke, eine physikalische Eigenschaft, eine Haftung oder jedwede Kombination davon ist.

4. Implantierbarer Stimulator nach einem vorstehenden Anspruch, wobei die mindestens eine Verkapselungsschicht (1300, 1310, 1320, 2150, 2250) den ersten Teil (2010, 2110, 2210, 2310) des Substrats (300, 1400) bedeckt.

5. Implantierbarer Stimulator nach einem vorstehenden Anspruch, wobei das mindestens eine leitfähige Elastomer (2260) ein anisotropisch leitfähiges Haftmittel, ein anisotropisch leitfähiger Film, ein anisotropisch leitfähiger Schaum, eine anisotropisch leitfähige Paste oder jedwede Kombination davon ist.

6. Implantierbarer Stimulator nach einem vorstehenden Anspruch, wobei das Substrat (300, 1400) weiter mindestens eine mechanische Strebe (2140, 2240, 2340) beim Aufeinandertreffen des ersten Teils (2010, 2110, 2210, 2310) und des anpassbaren zweiten Teils (2020, 2120, 2320) umfasst, wobei die mindestens eine mechanische Strebe (2140, 2240, 2340) konfiguriert ist, um der Abtrennung des anpassbaren zweiten Teils (2020, 2120, 2320) von dem ersten Teil (2010, 2110, 2210, 2310) zu widerstehen, und/oder die mindestens eine mechanische Strebe (2140, 2240, 2340) zur Bereitstellung eines Grads an mechanischer Vorspannung konfiguriert ist, sodass ein oder mehrere leitende Pfade durch das mindestens eine leitfähige Elastomer (2260) erzeugt werden.

7. Implantierbarer Stimulator nach Anspruch 6, wobei die mindestens eine mechanische Strebe (2140, 2240, 2340) Folgendes umfasst: mindestens einen Überstand, mindestens einen Vorsprung, mindestens eine Öffnung, mindestens eine Rille, mindestens einen Stift, mindestens einen Haken, mindestens eine Niete oder jedwede Kombination davon.

8. Implantierbarer Stimulator nach einem der Ansprüche 6 bis 7, wobei die mindestens eine mechanische Strebe (2140, 2240, 2340) eine oder mehrere Öffnungen und/oder Rillen umfasst, die zur Aufnahme von erheblichen Mengen an Verkapselungsmittel von der mindestens einen Verkapselungsschicht konfiguriert sind.

9. Implantierbarer Stimulator nach einem der Ansprüche 6 bis 8, wobei die mindestens eine mechanische Strebe (2140, 2240, 2340) als lösbar konfiguriert ist.

10. Implantierbarer Stimulator nach einem vorstehenden Anspruch, wobei der implantierbare Stimulator (100, 101, 102, 103, 104, 105, 1110, 1111, 2101, 2201, 2301) eine Haftschicht (1500, 1510, 1520) angrenzend an zumindest einen Teil des ersten Teils (2010, 2110, 2210, 2310) und zumindest einen Teil des anpassbaren zweiten Teils (2020, 2120, 2320) des Substrats (300, 1400) umfasst.

11. Implantierbarer Stimulator nach Anspruch 10, wobei die Haftschicht (2020, 2120, 2320) durch Tauchbeschichten angrenzend an zumindest einen Teil der Verkapselungsschicht (1300, 1310, 1320, 2150, 2250) aufgetragen ist.

12. Implantierbarer Stimulator nach einem vorstehenden Anspruch, wobei die eine oder mehreren elektrischen Schnittstellen zwischen der Vielzahl von elektrischen Verbindungen (250, 1210) und dem Impulsgenerator (500) vorliegen.

13. Implantierbarer Stimulator nach einem vorstehenden Anspruch, wobei die Dicke des Stimulators (100, 101, 102, 103, 104, 105, 1110, 1111, 2101, 2201, 2301) entlang des ersten Teils (2010, 2110, 2210, 2310) gleich oder kleiner als 5 Millimeter oder gleich oder kleiner als 4 Millimeter oder gleich oder kleiner als 3 Millimeter ist.

14. Implantierbarer Stimulator nach einem vorstehenden Anspruch, wobei die Verkapselungsschicht (1300, 1310, 1320, 2150, 2250) ein Polymer umfasst.

15. Implantierbarer Stimulator nach Anspruch 14, wobei die Verkapselungsschicht (1300, 1310, 1320, 2150, 2250) Polydimethylsiloxan (PDMS) umfasst.

16. Implantierbarer Stimulator nach einem vorstehenden Anspruch, wobei der anpassbare zweite Teil (2020, 2120, 2320) des Substrats (300, 1400) ein Flüssigkristallpolymer (LCP) umfasst.

17. Implantierbarer Stimulator nach Anspruch 16, wobei der anpassbare zweite Teil (2020, 2120, 2320) des Substrats (300, 1400) eine oder mehrere Schichten des LCP umfasst.

18. Implantierbarer Stimulator nach einem vorstehenden Anspruch, wobei die Dicke des Substrats (300, 1400) entlang des anpassbaren zweiten Teils (2020, 2120, 2320) gleich oder kleiner als 0,3 Millimeter oder gleich oder kleiner als 0,2 Millimeter oder gleich oder kleiner als 0,1 Millimeter ist.

19. Implantierbarer Stimulator nach einem vorstehenden Anspruch, wobei der erste Teil (2010, 2110, 2210, 2310) des Substrats (300, 1400) eine starre Leiterplatte, eine starre PCB und/oder ein starres keramisches Substrat umfasst.

20. Verfahren zur Herstellung eines implantierbaren Stimulators, das Folgendes umfasst:
Bereitstellen eines Substrats (300, 1400), wobei das Substrat (300, 1400) eine erste Oberfläche und eine zweite Oberfläche umfasst, wobei eine Dicke des Substrats (300, 1400) durch die erste und zweite Oberfläche definiert ist;
Bereitstellen eines Impulsgenerators (500) entlang eines ersten Teils (2010, 2110, 2210, 2310) des Substrats (300, 1400), wobei der Impulsgenerator (500) eine oder mehrere elektrische oder elektronische Komponenten umfasst, die zur Erzeugung von mindestens einem Stimulationsimpuls konfiguriert sind;
Positionieren einer Elektrodenanordnung (200, 400, 1220), die mindestens zwei Elektroden entlang eines anpassbaren zweiten Teils (2020, 2120, 2320) des Substrats (300, 1400) umfasst;
Abscheiden oder Galvanisieren auf das Substrat (300, 1400) einer Vielzahl von elektrischen Verbindungen (250, 1210), die den Impulsgenerator (500) mit den mindestens zwei Elektroden der Elektrodenanordnung (200, 400, 1220) elektrisch verbinden;
Bereitstellen einer oder mehrerer elektrischer Schnittstellen zwischen dem ersten Teil (2010, 2110, 2210, 2310) und dem anpassbaren zweiten Teil (2020, 2120, 2320), wobei die eine oder mehreren elektrischen Schnittstellen zwischen der Vielzahl von elektrischen Verbindungen (250, 1210) und dem Impulsgenerator (500) vorliegen;
Bereitstellen einer oder mehrerer elektrischer Schnittstellen zwischen dem ersten Teil (2010, 2110, 2210, 2310) und dem anpassbaren zweiten Teil (2020, 2120, 2320), wobei die eine oder mehreren elektrischen Schnittstellen zwischen der Vielzahl von elektrischen Verbindungen (250, 1210) und dem Impulsgenerator (500) vorliegen;
Bereitstellen von mindestens einem leitfähigen Elastomer (2260) zur elektrischen Verbindung von einer oder mehreren elektrischen Schnittstellen zwischen der Vielzahl von elektrischen Verbindungen (250, 1210) und dem Impulsgenerator (500), wobei die Dicke des Substrats (300, 1400) entlang des anpassbaren zweiten Teils (2020, 2120, 2320) gleich oder kleiner als 0,5 Millimeter ist;
Bereitstellen von mindestens einer Verkapselungsschicht (1300, 1310, 1320, 2150, 2250) zur zumindest teilweisen Bedeckung des ersten Teils (2010, 2110, 2210, 2310) und zur zumindest teilweisen Bedeckung des anpassbaren zweiten Teils (2020, 2120, 2320) des Substrats (300, 1400), wobei die mindestens eine Verkapselungsschicht (1300, 1310, 1320, 2150, 2250) konfiguriert ist, um der Abtrennung des anpassbaren zweiten Teils (2020, 2120, 2320) von dem ersten Teil (2010, 2110, 2210, 2310) beim Aufeinandertreffen des ersten Teils (2010, 2110, 2210, 2310) und des anpassbaren zweiten Teils (2020, 2120, 2320) zu widerstehen; und
Konfigurieren und Anordnen der mindestens einen Verkapselungsschicht (1300, 1310, 1320, 2150, 2250) zur Bereitstellung eines Grads an mechanischer Vorspannung, sodass ein oder mehrere leitende Pfade durch das mindestens eine leitfähige Elastomer (2260) erzeugt werden.

21. Verfahren nach Anspruch 20, wobei das Verfahren weiter Folgendes umfasst: Bereitstellen von mindestens einer mechanischen Strebe (2140, 2240, 2340) beim Aufeinandertreffen des ersten Teils (2010, 2110, 2210, 2310) und des anpassbaren zweiten Teils (2020, 2120, 2320) des Substrats (300, 1400); und Konfigurieren der mindestens einen mechanischen Strebe (2140, 2240, 2340), um der Abtrennung des anpassbaren zweiten Teils (2020, 2120, 2320) von dem ersten Teil (2010, 2110, 2210, 2310) zu widerstehen, und/oder Konfigurieren der mindestens einen mechanischen Strebe (2140, 2240, 2340) zur Bereitstellung eines Grads an mechanischer Vorspannung, sodass ein oder mehrere leitende Pfade durch das mindestens eine leitfähige Elastomer (2260) erzeugt werden.

## Revendications

1. Stimulateur implantable comprenant : un substrat (300, 1400) comprenant une première surface et une seconde surface, dans lequel une épaisseur du substrat (300, 1400) est définie par les première et seconde surfaces ;
le substrat (300, 1400) comprenant une première partie (2010, 2110, 2210, 2310) le long de laquelle est placé un générateur d'impulsions (500), le générateur d'impulsions (500) comprenant un ou plusieurs composants électriques ou électroniques, configurés pour générer au moins une impulsion de stimulation ;
un réseau d'électrodes (200, 400, 1220) comprenant au moins deux électrodes situées le long d'une seconde partie conformable (2020, 2120, 2320) du substrat (300, 1400) ;
une pluralité d'interconnexions électriques (250, 1210) couplant électriquement le générateur d'impulsions (500) aux au moins deux électrodes du réseau d'électrodes (200, 400, 1220), dans lequel la pluralité d'interconnexions électriques (250, 1210) est positionnée entre les première et seconde surfaces du substrat (300, 1400), le stimulateur implantable (100, 101, 102, 103, 104, 105, 1110, 1111, 2101, 2201, 2301) comprenant une ou plusieurs interfaces électriques entre la première partie (2010, 2110, 2210, 2310) et la seconde partie conformable (2020, 2120, 2320), les une ou plusieurs interfaces électriques se trouvant entre la pluralité d'interconnexions électriques (250. 1210) et le générateur d'impulsions (500) ;
le stimulateur implantable (100, 101, 102, 103, 104, 105, 1110, 1111, 2101, 2201, 2301) comprenant au moins un élastomère conducteur (2260), configuré et agencé pour connecter électriquement une ou plusieurs interfaces électriques entre la pluralité d'interconnexions électriques (250, 1210) et le générateur d'impulsions (500) ;
dans lequel l'épaisseur du substrat (300, 1400) le long de la seconde partie conformable (2020, 2120, 2320) est égale ou inférieure à 0,5 millimètre ;
le stimulateur implantable comprenant en outre au moins une couche d'encapsulation conformable (1300, 1310, 1320, 2150, 2250) qui recouvre au moins partiellement la première partie (2010, 2110, 2210, 2310) et au moins partiellement la seconde partie conformable (2020, 2120, 2320) du substrat (300, 1400), l'au moins une couche d'encapsulation (1300, 1310, 1320, 2150, 2250) étant configurée pour résister à une séparation de la seconde partie conformable (2020, 2120, 2320) d'avec la première partie (2010, 2110, 2210, 2310) au point de rencontre de la première partie (2010, 2110, 2210, 2310) et de la seconde partie conforme (2020, 2120, 2320) : et
dans lequel l'au moins une couche d'encapsulation (1300, 1310, 1320, 2150, 2250) est configurée et agencée pour fournir un degré de prétension mécanique de manière à créer un ou plusieurs chemins conducteurs à travers l'au moins un élastomère conducteur.

2. Stimulateur implantable selon la revendication l, dans lequel l'au moins un élastomère conducteur (2260) se trouve au point de rencontre de la première partie (2010, 2110, 2210, 2310) et de la seconde partie conformable (2020, 2120, 2220, 2320).

3. Stimulateur implantable selon la revendication 1 ou la revendication 2, dans lequel le degré de résistance à la séparation de la seconde partie conformable (2120) d'avec la première partie (2110) fourni par l'au moins une couche d'encapsulation (1300, 1310, 1320) est dû à au moins une propriété de l'au moins une couche d'encapsulation (1300, 1310, 1320), dans lequel l'au moins une propriété est une forme, une étendue, une épaisseur, une propriété physique, une adhérence ou toute combinaison de celles-ci.

4. Stimulateur implantable selon l'une quelconque des revendications précédentes, dans lequel l'au moins une couche d'encapsulation (1300, 1310, 1320, 2150, 2250) recouvre la première partie (2010, 2110, 2210, 2310) du substrat (300, 1400).

5. Stimulateur implantable selon l'une quelconque des revendications précédentes, dans lequel l'au moins un élastomère conducteur (2260) est un élastomère conducteur anisotrope, un film conducteur anisotrope, une mousse conductrice anisotrope. une pâte conductrice anisotrope, ou toute combinaison de ceux-ci,

6. Stimulateur implantable selon l'une quelconque des revendications précédentes. dans lequel le substrat (300, 1400) comprend en outre au moins un renfort mécanique (2140, 2240, 2340) au point de rencontre de la première partie (2010, 2110, 2210, 2310) et de la seconde partie conformable (2020, 2120, 2320), l'au moins un renfort mécanique (2140, 2240, 2340) étant configuré pour résister à la séparation de la seconde partie conformable (2020, 2120, 2320) d'avec la première partie (2010, 2110, 2210, 2310) et/ou l'au moins un renfort mécanique (2140, 2240, 2340) étant configuré pour fournir un degré de prétension mécanique de manière à créer un ou plusieurs chemins conducteurs à travers l'élastomère conducteur (2260).

7. Stimulateur implantable selon la revendication 6, dans lequel l'au moins un renfort mécanique (2140, 2240, 2340) comprend : au moins une protubérance, au moins une saillie, au moins une ouverture, au moins une rainure, au moins une goupille, au moins un crochet, au moins un rivet, ou toute combinaison de ceux-ci.

8. Stimulateur implantable selon l'une quelconque des revendications 6 à 7, dans lequel l'au moins un renfort mécanique (2140, 2240, 2340) comprend une ou plusieurs ouvertures et/ou rainures, configurées pour recevoir des quantités significatives d'encapsulant provenant de l'au moins une couche d'encapsulation.

9. Stimulateur implantable selon l'une quelconque des revendications 6 à 8, dans lequel l'au moins un renfort mécanique (2140, 2240, 2340) est configuré pour pouvoir être détachable.

10. Stimulateur implantable selon l'une quelconque des revendications précédentes, le stimulateur implantable (100, 101, 102, 103, 104, 105, 1110, 1111, 2101, 2201, 2301) comprend une couche d'adhérence (1500, 1510, 1520) adjacente à au moins une zone de la première partie (2010, 2110, 2210, 2310) et à au moins une zone de la seconde partie conformable (2020, 2120, 2320) du substrat (300, 1400).

11. Stimulateur implantable selon la revendication 10, dans lequel la couche d'adhérence (2020, 2120, 2320) est appliquée par revêtement par immersion adjacent à au moins une zone de la couche d'encapsulation (1300, 1310, 1320, 2150, 2250).

12. Stimulateur implantable selon l'une quelconque des revendications précédentes, dans lequel les une ou plusieurs interfaces électriques se trouvent entre la pluralité d'interconnexions électriques (250, 1210) et le générateur d'impulsions (500).

13. Stimulateur implantable selon l'une quelconque des revendications précédentes, dans lequel l'épaisseur du stimulateur (100, 101, 102, 103, 104, 105, 1110, 1111, 2101, 2201, 2301) le long de la première partie (2010, 2110, 2210, 2310) est égale ou inférieure à 5 millimètres, ou égale ou inférieure à 4 millimètres, ou égale à ou inférieure à 3 millimètres.

14. Stimulateur implantable selon l'une quelconque des revendications précédentes, dans lequel la couche d'encapsulation (1300, 1310, 1320, 2150, 2250) comprend un polymère.

15. Stimulateur implantable selon la revendication 14, dans lequel la couche d'encapsulation (1300, 1310, 1320, 2150, 2250) comprend du polydiméthylsiloxane (PDMS).

16. Stimulateur implantable selon l'une quelconque des revendications précédentes, dans lequel la seconde partie conformable (2020, 2120, 2320) du substrat (300, 1400) comprend un polymère à cristaux liquides (LCP).

17. Stimulateur implantable selon la revendication 16, dans lequel la seconde partie conformable (2020, 2120, 2320) du substrat (300, 1400) comprend une ou plusieurs couches du LCP.

18. Stimulateur implantable selon l'une quelconque des revendications précédentes, dans lequel l'épaisseur du substrat (300, 1400) le long de la seconde partie conformable (2020, 2120, 2320) est égale ou inférieure à 0,3 millimètre, ou égale ou inférieure à 0,2 millimètre, ou égale ou inférieure à 0,1 millimètre.

19. Stimulateur implantable selon l'une quelconque des revendications précédentes, dans lequel la première partie (2010, 2110, 2210, 2310) du substrat (300, 1400) comprend une carte de circuit imprimé rigide, un circuit imprimé rigide et/ou un substrat céramique rigide.

20. Procédé de fabrication d'un stimulateur implantable, comprenant :
la fourniture d'un substrat (300, 1400), le substrat (300, 1400) comprenant une première surface et une seconde surface, dans lequel une épaisseur du substrat (300, 1400) est définie par les première et seconde surfaces ;
la fourniture d'un générateur d'impulsions (500) le long d'une première partie (2010, 2110, 2210, 2310) du substrat (300, 1400), le générateur d'impulsions (500) comprenant un ou plusieurs composants électriques ou électroniques configurés pour générer au moins une impulsion de stimulation ;
le placement d'un réseau d'électrodes (200, 400, 1220) comprenant au moins deux électrodes le long d'une seconde partie conformable (2020, 2120, 2320) du substrat (300, 1400) ;
le dépôt ou la galvanoplastie sur le substrat (300, 1400) d'une pluralité d'interconnexions électriques (250. 1210) couplant électriquement le générateur d'impulsions (500) aux au moins deux électrodes du réseau d'électrodes (200, 400, 1220) :
la fourniture d'une ou plusieurs interfaces électriques entre la première partie (2010, 2110, 2210, 2310) et la seconde partie conforme (2020, 2120, 2320), dans lequel les une ou plusieurs interfaces électriques se trouvent entre la pluralité d'interconnexions électriques (250, 1210) et le générateur d'impulsions (500) ;
la fourniture d'une ou plusieurs interfaces électriques entre la première partie (2010, 2110, 2210, 2310) et la seconde partie conformable (2020, 2120, 2320), les une ou plusieurs interfaces électriques se trouvant entre la pluralité d'interconnexions électriques (250, 1210) et le générateur d'impulsions (500) ;
la fourniture d'au moins un élastomère conducteur (2260) pour connecter électriquement une ou plusieurs interfaces électriques entre la pluralité d'interconnexions électriques (250, 1210) et le générateur d'impulsions (500), dans lequel l'épaisseur du substrat (300, 1400) le long de la seconde partie conformable (2020, 2120, 2320) est égale ou inférieure à 0,5 millimètre ;
la fourniture d'au moins une couche d'encapsulation (1300, 1310, 1320, 2150, 2250) pour recouvrir au moins partiellement la première partie (2010, 2110, 2210, 2310) et recouvrir au moins partiellement la seconde partie conformable (2020. 2120. 2320) du substrat (300, 1400), l'au moins une couche d'encapsulation (1300, 1310, 1320, 2150, 2250) étant configurée pour résister à une séparation de la seconde partie conformable (2020, 2120, 2320) d'avec la première partie (2010, 2110, 2210, 2310) au point de rencontre de la première partie (2010, 2110, 2210, 2310) et de la seconde partie conformable (2020, 2120, 2320) ; et
la configuration et l'agencement de l'au moins une couche d'encapsulation (1300, 1310, 1320, 2150, 2250) pour fournir un degré de prétension mécanique de manière à créer un ou plusieurs chemins conducteurs à travers l'au moins un élastomère conducteur (2260).

21. Procédé selon la revendication 20, le procédé comprenant en outre la fourniture d'au moins un renfort mécanique (2140, 2240, 2340) au point de rencontre de la première partie (2010, 2110, 2210, 2310) et de la seconde partie conformable (2020, 2120, 2320) du substrat (300, 1400) ; et la configuration de l'au moins un renfort mécanique (2140, 2240, 2340) pour résister à une séparation de la seconde partie conformable (2020, 2120, 2320) d'avec la première partie (2010, 2110, 2210, 2310) et/ou la configuration de l'au moins un renfort mécanique (2140, 2240, 2340) pour fournir un degré de prétension mécanique de manière à créer un ou plusieurs chemins conducteurs à travers l'au moins un élastomère conducteur (2260).
